# EUROPEAN PATENT SPECIFICATION

(11) **EP 1 356 064 B1**
(45) Date of publication and mention of the grant of the patent: **16.09.2009**
(21) Application number: 01995339.7
(22) Date of filing: 04.12.2001
(51) Int. Cl.: C12N 15/82, C12N 15/29, C07K 14/47, C12N 5/10, A01H 5/00

(54) **TRANSCRIPTIONAL REGULATOR NUCLEIC ACIDS, POLYPEPTIDES AND METHODS OF USE THEREOF**
TRANSKRIPTIONALE REGULATOR-NUKLEINSÄUREN, POLYPEPTIDE UND VERFAHREN IHRER ANWENDUNG
ACIDES NUCLEIQUES DE REGULATION TRANSCRIPTIONNELLE, POLYPEPTIDES ET PROCEDES D'UTILISATION CORRESPONDANTS

(30) Priority: 06.12.2000 US 251555 P
(43) Date of publication of application: 29.10.2003
(73) Proprietor: PIONEER HI-BRED INTERNATIONAL, INC., Des Moines, Iowa 50309 (US); E.I. DU PONT DE NEMOURS AND COMPANY, Wilmington, DE 19898 (US)
(72) Inventor: TAO, Yumin, Urbandale, IA 50322 (US); GORDON-KAMM, William, J., Urbandale, IA 50322 (US); SHEN, Bo, Johnston, IA 50131 (US); LOWE, Keith, S., Johnston, IA 50131 (US); DANILEVSKAYA, Olga, N., Johnston, IA 50131 (US); MAHAJAN, Pramod, B., Urbandale, IA 50322 (US); RAFALSKI, Jan, Antoni, Wilmington, DE 19810 (US); SAKAI, Hajime, Neward, DE 19711 (US); KLEIN, Theodore, M., Wilmington, DE 19810 (US)
(74) Representative: Bentham, Andrew
(86) International application number: PCT/US2001/046326
(87) International publication number: WO 2002/046443

(56) References cited:
- WO-A-01/14519
- WO-A-96/39505
- OGAS J ET AL: "PICKLE IS A CHD3 CHROMATIN-REMODELING FACTOR THAT REGULATES THE TRANSITION FROM EMBRYONIC TO VEGETATIVE DEVELOPMENT IN ARABIDOPSIS" PROCEEDINGS OF THE NATIONAL ACADEMY OF SCIENCES OF USA, NATIONAL ACADEMY OF SCIENCE. WASHINGTON, US, vol. 96, no. 24, 23 November 1999 (1999-11-23), pages 13839-13844, XP002938317 ISSN: 0027-8424 cited in the application
- ESHED Y ET AL: "Distinct mechanisms promote polarity establishment in carpels of Arabidopsis." CELL. UNITED STATES 15 OCT 1999, vol. 99, no. 2, 15 October 1999 (1999-10-15), pages 199-209, XP002223446 ISSN: 0092-8674 -& DATABASE EMBL [Online] 1 May 2002 (2002-05-01) ESHED ET AL.: "Chromain remoddeling factor CHD3" retrieved from EBI Database accession no. Q9S775 XP002223447
- RIDER ET AL: 'Metabolic profiling of the Arabidopsis pkl mutant reveals selective derepression of embryonic traits' PLANTA vol. 219, no. 3, July 2004, BERLIN, pages 489 - 499
- HENDERSON ET AL: 'PICKLE acts throughout the plant to repress expression of embryonic traits and may play a role in gibberellin-dependent responses' PLANT PHYSIOLOGY vol. 134, no. 3, March 2004, ROCKVILLE, pages 995 - 1005
- LI ET AL: 'PICKLE acts during germination to repress expression of embryonic traits' THE PALNT JOURNAL vol. 44, no. 6, December 2005, pages 1010 - 1022

### SEQUENCE LISTING

<110> Pioneer Hi-Bred, Intl
   E.I. du Pont de Nemours and Company
<120> Transcriptional Regulator Nucleic Acids,
   Polypeptides and Methods of Use Thereof
<130> 1288-PCT
<150> 60/251,555
   <151> 2000-12-06
<160> 41
<170> FastSEQ for Windows Version 3.0
<210> 1
   <211> 1874
   <212> DNA
   <213> Zea mays
<220>
   <221> CDS
   <222> (3)...(1656)
<400> 1
   ac gag aat gat gaa tct cgc caa att cat tat gac gaa gct gca att         47
      Glu Asn Asp Glu Ser Arg Gln Ile His Tyr Asp Glu Ala Ala Ile
       1               5                  10                  15
   gag agg ttg tta gac cgt gat caa gtt gac ggt gat gaa tct gtg gaa        95
   Glu Arg Leu Leu Asp Arg Asp Gln Val Asp Gly Asp Glu Ser Val Glu
                   20                  25                  30
   gat gaa gaa gaa gat gga ttc tta aaa gga ttc aag gtt gca aac ttt       143
   Asp Glu Glu Glu Asp Gly Phe Leu Lys Gly Phe Lys Val Ala Asn Phe
               35                  40                  45
   gaa tat atc gat gag gca aag gct cag gca gaa aaa gag gag gca cgg       191
   Glu Tyr Ile Asp Glu Ala Lys Ala Gln Ala Glu Lys Glu Glu Ala Arg
           50                  55                  60
   aga aag gct gca gct gag gct gaa aat tct gaa aga aac tac tgg gat       239
   Arg Lys Ala Ala Ala Glu Ala Glu Asn Ser Glu Arg Asn Tyr Trp Asp
       65                  70                  75
   gaa cta ttg aag gat aga tat gat gta cag aaa gtt gaa gaa cat act       287
   Glu Leu Leu Lys Asp Arg Tyr Asp Val Gln Lys Val Glu Glu His Thr
   80                  85                  90                  95
   gct atg gga aaa ggg aaa aga agc cgc aaa cag atg gct gcc gct gat       335
   Ala Met Gly Lys Gly Lys Arg Ser Arg Lys Gln Met Ala Ala Ala Asp
                   100                 105                 110
   gaa gat gac att cat gat tta agt tcc gaa gat gag gat tac tca ttg      383
   Glu Asp Asp Ile His Asp Leu Ser Ser Glu Asp Glu Asp Tyr Ser Leu
               115                 120                 125
   gag gat gac att tca gat aat gac aca agt ttg caa gga aat att tct      431
   Glu Asp Asp Ile Ser Asp Asn Asp Thr Ser Leu Gln Gly Asn Ile Ser
           130                 135                 140
   ggg aag agg gga caa tat tct aag aga aaa tca cgt aat gtt gat tct      479
   Gly Lys Arg Gly Gln Tyr Ser Lys Arg Lys Ser Arg Asn Val Asp Ser
       145                 150                 155
   att cca ttg atg gag ggc gaa gga cgt acc ttg aga gtt ctt gga ttc      527
   Ile Pro Leu Met Glu Gly Glu Gly Arg Thr Leu Arg Val Leu Gly Phe
   160                 165                 170                 175
   aac cat gct caa cga gca atg ttc cta cag aca ctc aat aga ttc ggt      575
   Asn His Ala Gln Arg Ala Met Phe Leu Gln Thr Leu Asn Arg Phe Gly
                   180                 185                 190
   ttt cag aat tat gac tgg aaa gag tat ctt cct cgt ctt aaa gga aaa      623
   Phe Gln Asn Tyr Asp Trp Lys Glu Tyr Leu Pro Arg Leu Lys Gly Lys
               195                 200                 205
   agt gtc gag gaa atc cag aga tat gct gaa ctt gtc atg gca cat ctt      671
   Ser Val Glu Glu Ile Gln Arg Tyr Ala Glu Leu Val Met Ala His Leu
           210                 215                 220
   gtt gaa gaa att aat gat tct gac tat ttt tca gat ggc gtt cca aag      719
   Val Glu Glu Ile Asn Asp Ser Asp Tyr Phe Ser Asp Gly Val Pro Lys
       225                 230                 235
   gaa atg atg cgt gtt gat gat gta cta gtc agg ata gca aac ata tcc      767
   Glu Met Met Arg Val Asp Asp Val Leu Val Arg Ile Ala Asn Ile Ser
   240                 245                 250                 255
   ctt atc gag gag aag atg gct gcc aca gga cca gga aaa att aca aac      815
   Leu Ile Glu Glu Lys Met Ala Ala Thr Gly Pro Gly Lys Ile Thr Asn
                   260                 265                 270
   att ttt cct aat tac ttg ctc tat gag ttc caa ggc tta tct ggt gga      863
   Ile Phe Pro Asn Tyr Leu Leu Tyr Glu Phe Gln Gly Leu Ser Gly Gly
               275                 280                 285
   aga ata tgg aaa gcg gag cat gat cta ctg tta ctg aga ggc ata ctg      911
   Arg Ile Trp Lys Ala Glu His Asp Leu Leu Leu Leu Arg Gly Ile Leu
           290                 295                 300
   aag cat gga tat gca agg tgg cag tat ata tca gat gac aga gag aat      959
   Lys His Gly Tyr Ala Arg Trp Gln Tyr Ile Ser Asp Asp Arg Glu Asn
       305                 310                 315
   ggg ctt ttt gag gct gca cga cga gag ctg cat ctc cct tcg gtt aat     1007
   Gly Leu Phe Glu Ala Ala Arg Arg Glu Leu His Leu Pro Ser Val Asn
   320                 325                 330                 335
   gaa ata att ggt gct cag ttg aac gag gca aat ggg aat ttg gaa ggt     1055
   Glu Ile Ile Gly Ala Gln Leu Asn Glu Ala Asn Gly Asn Leu Glu Gly
                   340                 345                 350
   gca cag gaa ggc caa gcg aac aca aca agc atg tcg cat tac aag gag      1103
   Ala Gln Glu Gly Gln Ala Asn Thr Thr Ser Met Ser His Tyr Lys Glu
               355                 360                 365
   atc cag aga aag ata gtt gag ttc ttg aga aag aga tat cat ctt atg      1151
   Ile Gln Arg Lys Ile Val Glu Phe Leu Arg Lys Arg Tyr His Leu Met
           370                 375                 380
   gag aga gcc ttg aat ctg gaa tat gct gtg ata aag aaa aaa att cct      1199
   Glu Arg Ala Leu Asn Leu Glu Tyr Ala Val Ile Lys Lys Lys Ile Pro
       385                 390                 395
   gtt cct gat gat att act gaa caa ggt gtt cca gca gga cat gct ccg      1247
   Val Pro Asp Asp Ile Thr Glu Gln Gly Val Pro Ala Gly His Ala Pro
   400                 405                 410                 415
   ctt att cca gat atc agt gaa ctg ttg cgg gaa ttg ccc aat ctt gag      1295
   Leu Ile Pro Asp Ile Ser Glu Leu Leu Arg Glu Leu Pro Asn Leu Glu
                   420                 425                 430
   cca att tct acc aat gaa ttg att tct gag ggc aca gct ggt cag tta      1343
   Pro Ile Ser Thr Asn Glu Leu Ile Ser Glu Gly Thr Ala Gly Gln Leu
               435                 440                 445
   caa gtt ccc cat ctc tac aat aag atg tgt gga gtg ctt gaa gag agt      1391
   Gln Val Pro His Leu Tyr Asn Lys Met Cys Gly Val Leu Glu Glu Ser
           450                 455                 460
   ggt gct tat gcg ctc agt tcc ttc ttt gga gac aag tcc gca tct tct      1439
   Gly Ala Tyr Ala Leu Ser Ser Phe Phe Gly Asp Lys Ser Ala Ser Ser
       465                 470                 475
   act ttg gcc aat agc ctt cga cag ttt gaa act gtg tgt gag aat gtc      1487
   Thr Leu Ala Asn Ser Leu Arg Gln Phe Glu Thr Val Cys Glu Asn Val
   480                 485                 490                 495
   gtc gag gcc tta cga cca cac caa aat ggt act gcc agt gcc atc aaa      1535
   Val Glu Ala Leu Arg Pro His Gln Asn Gly Thr Ala Ser Ala Ile Lys
                   500                 505                 510
   gag gaa ttg gta gat gca gcc acc aaa gca gca gca gca gca gct cct      1583
   Glu Glu Leu Val Asp Ala Ala Thr Lys Ala Ala Ala Ala Ala Ala Pro
               515                 520                 525
   caa caa gat tca ggc cat gat gca ccg cat ggg cag tct tcg aca gcc      1631
   Gln Gln Asp Ser Gly His Asp Ala Pro His Gly Gln Ser Ser Thr Ala
           530                 535                 540
   aag gcg gac atg gaa atc gat ggt t gatttgtagg ttccagagtg              1676
   Lys Ala Asp Met Glu Ile Asp Gly
       545                 550
   gcaagaaagg gaatcccctc taatcattat gtatactgtg gtcagaatgt ccgctatata    1736
   ttgtaacatc aaagaaagca cctccaggcc tgagggtgtt actgctaatg cgtttggttt    1796
   acttgtcctt gtaatatgca tacacattta gaactcatgc agccattgtg tgaaaaaaaa 1856
   aaaaaaaaaa aaaaaaaa 1874
<210> 2
   <211> 551
   <212> PRT
   <213> Zea mays
<400> 2
   Glu Asn Asp Glu Ser Arg Gln Ile His Tyr Asp Glu Ala Ala Ile Glu
    1               5                  10                  15
   Arg Leu Leu Asp Arg Asp Gln Val Asp Gly Asp Glu Ser Val Glu Asp
               20                  25                  30
   Glu Glu Glu Asp Gly Phe Leu Lys Gly Phe Lys Val Ala Asn Phe Glu
           35                  40                  45
   Tyr Ile Asp Glu Ala Lys Ala Gln Ala Glu Lys Glu Glu Ala Arg Arg
       50                  55                  60
   Lys Ala Ala Ala Glu Ala Glu Asn Ser Glu Arg Asn Tyr Trp Asp Glu
   65                  70                  75                  80
   Leu Leu Lys Asp Arg Tyr Asp Val Gln Lys Val Glu Glu His Thr Ala
                   85                  90                  95
   Met Gly Lys Gly Lys Arg Ser Arg Lys Gln Met Ala Ala Ala Asp Glu
               100                 105                 110
   Asp Asp Ile His Asp Leu Ser Ser Glu Asp Glu Asp Tyr Ser Leu Glu
           115                 120                 125
   Asp Asp Ile Ser Asp Asn Asp Thr Ser Leu Gln Gly Asn Ile Ser Gly
       130                 135                 140
   Lys Arg Gly Gln Tyr Ser Lys Arg Lys Ser Arg Asn Val Asp Ser Ile
   145                 150                 155                 160
   Pro Leu Met Glu Gly Glu Gly Arg Thr Leu Arg Val Leu Gly Phe Asn
                   165                 170                 175
   His Ala Gln Arg Ala Met Phe Leu Gln Thr Leu Asn Arg Phe Gly Phe
               180                 185                 190
   Gln Asn Tyr Asp Trp Lys Glu Tyr Leu Pro Arg Leu Lys Gly Lys Ser
           195                 200                 205
   Val Glu Glu Ile Gln Arg Tyr Ala Glu Leu Val Met Ala His Leu Val
       210                 215                 220
   Glu Glu Ile Asn Asp Ser Asp Tyr Phe Ser Asp Gly Val Pro Lys Glu
   225                 230                 235                 240
   Met Met Arg Val Asp Asp Val Leu Val Arg Ile Ala Asn Ile Ser Leu
                   245                 250                 255
   Ile Glu Glu Lys Met Ala Ala Thr Gly Pro Gly Lys Ile Thr Asn Ile
               260                 265                 270
   Phe Pro Asn Tyr Leu Leu Tyr Glu Phe Gln Gly Leu Ser Gly Gly Arg
           275                 280                 285
   Ile Trp Lys Ala Glu His Asp Leu Leu Leu Leu Arg Gly Ile Leu Lys
       290                 295                 300
   His Gly Tyr Ala Arg Trp Gln Tyr Ile Ser Asp Asp Arg Glu Asn Gly
   305                 310                 315                 320
   Leu Phe Glu Ala Ala Arg Arg Glu Leu His Leu Pro Ser Val Asn Glu
                   325                 330                 335
   Ile Ile Gly Ala Gln Leu Asn Glu Ala Asn Gly Asn Leu Glu Gly Ala
               340                 345                 350
   Gln Glu Gly Gln Ala Asn Thr Thr Ser Met Ser His Tyr Lys Glu Ile
           355                 360                 365
   Gln Arg Lys Ile Val Glu Phe Leu Arg Lys Arg Tyr His Leu Met Glu
       370                 375                 380
   Arg Ala Leu Asn Leu Glu Tyr Ala Val Ile Lys Lys Lys Ile Pro Val
   385 390 395 400
   Pro Asp Asp Ile Thr Glu Gln Gly Val Pro Ala Gly His Ala Pro Leu
   405 410 415
   Ile Pro Asp Ile Ser Glu Leu Leu Arg Glu Leu Pro Asn Leu Glu Pro
   420 425 430
   Ile Ser Thr Asn Glu Leu Ile Ser Glu Gly Thr Ala Gly Gln Leu Gln
   435 440 445
   Val Pro His Leu Tyr Asn Lys Met Cys Gly Val Leu Glu Glu Ser Gly
   450 455 460
   Ala Tyr Ala Leu Ser Ser Phe Phe Gly Asp Lys Ser Ala Ser Ser Thr
   465 470 475 480
   Leu Ala Asn Ser Leu Arg Gln Phe Glu Thr Val Cys Glu Asn Val Val
   485 490 495
   Glu Ala Leu Arg Pro His Gln Asn Gly Thr Ala Ser Ala Ile Lys Glu
   500 505 510
   Glu Leu Val Asp Ala Ala Thr Lys Ala Ala Ala Ala Ala Ala Pro Gln
   515 520 525
   Gln Asp Ser Gly His Asp Ala Pro His Gly Gln Ser Ser Thr Ala Lys
   530 535 540
   Ala Asp Met Glu Ile Asp Gly
   545 550
<210> 3
   <211> 21
   <212> DNA
   <213> Artificial Sequence
<220>
   <221> primer_bind
   <222> (1) ... (21)
<400> 3
   acgagaatga tgaatctcgc c 21
<210> 4
   <211> 23
   <212> DNA
   <213> Artificial Sequence
<220>
   <221> primer_bind
   <222> (1) ... (23)
<400> 4
   tcaaccatcg atttccatgt ccg 23
<210> 5
   <211> 941
   <212> DNA
   <213> Zea mays
<220>
   <221> CDS
   <222> (48)...(941)
<400> 5
   gtcgacccac gcgtccgcag gattctggga agctccacac cttggat atg cta cta      56
                                                       Met Leu Leu
                                                       1
   cga cgc ctt cga gct gaa ggt cat cgt gtg ctt ctt ttt gct cag atg     104
   Arg Arg Leu Arg Ala Glu Gly His Arg Val Leu Leu Phe Ala Gln Met
       5                   10                  15
   act aaa atg ttg gac att ctt gag gat tac atg aat ttc aga aaa ttc     152
   Thr Lys Met Leu Asp Ile Leu Glu Asp Tyr Met Asn Phe Arg Lys Phe
   20                  25                  30                  35
   aag tat ttc aga ctt gat ggg tct tca gcc atc tca gac cgc cgt gac     200
   Lys Tyr Phe Arg Leu Asp Gly Ser Ser Ala Ile Ser Asp Arg Arg Asp
                   40                  45                  50
   atg gtc cga gat ttt cag aac agg aat gac ata ttt gtt ttc ttg tta     248
   Met Val Arg Asp Phe Gln Asn Arg Asn Asp Ile Phe Val Phe Leu Leu
               55                  60                  65
   agc aca aga gct ggg ggg ctt ggt att aat ttg act gct gct gat act     296
   Ser Thr Arg Ala Gly Gly Leu Gly Ile Asn Leu Thr Ala Ala Asp Thr
           70                  75                  80
   gtt att ttt tat gaa att gac tgg aat cca aca caa gac cag cag gca     344
   Val Ile Phe Tyr Glu Ile Asp Trp Asn Pro Thr Gln Asp Gln Gln Ala
       85                  90                  95
   atg gat aga aca cac aga ctt ggt caa aca aag gag gta act gtg tac     392
   Met Asp Arg Thr His Arg Leu Gly Gln Thr Lys Glu Val Thr Val Tyr
   100                 105                 110                 115
   agg ctt ata tgc aaa gat acc att gag gag aaa ata ttg caa aga gca     440
   Arg Leu Ile Cys Lys Asp Thr Ile Glu Glu Lys Ile Leu Gln Arg Ala
                   120                 125                 130
   aag cag aaa aat gca gtg caa gag tta gtt atg aag ggg aaa cat gtc     488
   Lys Gln Lys Asn Ala Val Gln Glu Leu Val Met Lys Gly Lys His Val
               135                 140                 145
   caa gac gat cat ttg atg aga caa gag gat gtt gtt tca tta ctt att     536
   Gln Asp Asp His Leu Met Arg Gln Glu Asp Val Val Ser Leu Leu Ile
           150                 155                 160
   gat gac aca cag att gca cac aag ttg aaa gaa ata tcc atg cag gcg     584
   Asp Asp Thr Gln Ile Ala His Lys Leu Lys Glu Ile Ser Met Gln Ala
       165                 170                 175
   aag gat cga caa aag agg aga cga gcg aag ggc atc aag gtt gac aaa     632
   Lys Asp Arg Gln Lys Arg Arg Arg Ala Lys Gly Ile Lys Val Asp Lys
   180                 185                 190                 195
   gaa gga gat ttg acg ctc gaa gac ttg gat gat gct act gca gaa gct     680
   Glu Gly Asp Leu Thr Leu Glu Asp Leu Asp Asp Ala Thr Ala Glu Ala
                   200                 205                 210
   gta gat caa gac aaa acg acc agc aaa aag aaa aag agc tcc cac aag     728
   Val Asp Gln Asp Lys Thr Thr Ser Lys Lys Lys Lys Ser Ser His Lys
               215                 220                 225
   aaa cat acg aat act cat gat aat gac aat ata gac aag aat gga gag      776
   Lys His Thr Asn Thr His Asp Asn Asp Asn Ile Asp Lys Asn Gly Glu
           230                 235                 240
   gcc gat gtg gga gat cat ccg ggg agt agt aac aca gaa aac gaa cag      824
   Ala Asp Val Gly Asp His Pro Gly Ser Ser Asn Thr Glu Asn Glu Gln
       245                 250                 255
   atg ccc gaa tca aga cct aaa aga tca aaa agg ctg atg aag agc att      872
   Met Pro Glu Ser Arg Pro Lys Arg Ser Lys Arg Leu Met Lys Ser Ile
   260                 265                 270                 275
   act gat gac aag gaa cta gct gct gct gcg gat cat gag aaa ccg gta      920
   Thr Asp Asp Lys Glu Leu Ala Ala Ala Ala Asp His Glu Lys Pro Val
                   280                 285                 290
   aat gaa gcg gaa aat cac tga                                          941
   Asn Glu Ala Glu Asn His  *
               295
<210> 6
   <211> 297
   <212> PRT
   <213> Zea mays
<220>
   <221> VARIANT
   <222> (1) ... (425)
   <223> Xaa = Any Amino Acid
<400> 6
   Met Leu Leu Arg Arg Leu Arg Ala Glu Gly His Arg Val Leu Leu Phe
    1               5                  10                  15
   Ala Gln Met Thr Lys Met Leu Asp Ile Leu Glu Asp Tyr Met Asn Phe
               20                  25                  30
   Arg Lys Phe Lys Tyr Phe Arg Leu Asp Gly Ser Ser Ala Ile Ser Asp
           35                  40                  45
   Arg Arg Asp Met Val Arg Asp Phe Gln Asn Arg Asn Asp Ile Phe Val
       50                  55                  60
   Phe Leu Leu Ser Thr Arg Ala Gly Gly Leu Gly Ile Asn Leu Thr Ala
   65                  70                  75                  80
   Ala Asp Thr Val Ile Phe Tyr Glu Ile Asp Trp Asn Pro Thr Gln Asp
                   85                  90                  95
   Gln Gln Ala Met Asp Arg Thr His Arg Leu Gly Gln Thr Lys Glu Val
               100                 105                 110
   Thr Val Tyr Arg Leu Ile Cys Lys Asp Thr Ile Glu Glu Lys Ile Leu
           115                 120                 125
   Gln Arg Ala Lys Gln Lys Asn Ala Val Gln Glu Leu Val Met Lys Gly
       130                 135                 140
   Lys His Val Gln Asp Asp His Leu Met Arg Gln Glu Asp Val Val Ser
   145                 150                 155                 160
   Leu Leu Ile Asp Asp Thr Gln Ile Ala His Lys Leu Lys Glu Ile Ser
                   165                 170                 175
   Met Gln Ala Lys Asp Arg Gln Lys Arg Arg Arg Ala Lys Gly Ile Lys
               180                 185                 190
   Val Asp Lys Glu Gly Asp Leu Thr Leu Glu Asp Leu Asp Asp Ala Thr
           195                 200                 205
   Ala Glu Ala Val Asp Gln Asp Lys Thr Thr Ser Lys Lys Lys Lys Ser
       210                 215                 220
   Ser His Lys Lys His Thr Asn Thr His Asp Asn Asp Asn Ile Asp Lys
   225                 230                 235                 240
   Asn Gly Glu Ala Asp Val Gly Asp His Pro Gly Ser Ser Asn Thr Glu
                   245                 250                 255
   Asn Glu Gln Met Pro Glu Ser Arg Pro Lys Arg Ser Lys Arg Leu Met
               260                 265                 270
   Lys Ser Ile Thr Asp Asp Lys Glu Leu Ala Ala Ala Ala Asp His Glu
           275                 280                 285
   Lys Pro Val Asn Glu Ala Glu Asn His
       290                 295
<210> 7
   <211> 22
   <212> DNA
   <213> Artificial Sequence
<220>
   <221> primer_bind
   <222> (1)...(22)
<400> 7
   aagctccaca ccttggatat gc 22
<210> 8
   <211> 23
   <212> DNA
   <213> Artificial Sequence
<220>
   <221> primer_bind
   <222> (1) ... (23)
<400> 8
   tcatgggctc agtgattttc cgc 23
<210> 9
   <211> 1913
   <212> DNA
   <213> Zea mays
<220>
   <221> CDS
   <222> (3)...(1913)
<400> 9
   ca cac tta ata att gct cca aaa gca gta tta cca aat tgg tct aac      47
      His Leu Ile Ile Ala Pro Lys Ala Val Leu Pro Asn Trp Ser Asn
       1               5                  10                  15
   gaa ttc aaa acc tgg gct ccc agt att ggg aca att ctg tat gat ggt     95
   Glu Phe Lys Thr Trp Ala Pro Ser Ile Gly Thr Ile Leu Tyr Asp Gly
                   20                  25                  30
   cgt cca gaa gag agg aag ctt tta agg gaa aag aat ttt gat gga ttg        143
   Arg Pro Glu Glu Arg Lys Leu Leu Arg Glu Lys Asn Phe Asp Gly Leu
                   35                  40                  45
   caa ttt aat gtt ttg ctc acg cat tat gac ttg ata ctg aaa gat aag        191
   Gln Phe Asn Val Leu Leu Thr His Tyr Asp Leu Ile Leu Lys Asp Lys
               50                  55                  60
   aag ttc cta aag aaa gtt cac tgg cat tat ttg att gtt gat gaa gga        239
   Lys Phe Leu Lys Lys Val His Trp His Tyr Leu Ile Val Asp Glu Gly
           65                  70                  75
   cat cgt ctg aaa aat cat gaa tgt gct ctt gct cgc aca cta gtt tca        287
   His Arg Leu Lys Asn His Glu Cys Ala Leu Ala Arg Thr Leu Val Ser
       80                  85                  90                  95
   gga tat cag atc cgc cgc aga cta ctt tta act ggc act cca atc caa        335
   Gly Tyr Gln Ile Arg Arg Arg Leu Leu Leu Thr Gly Thr Pro Ile Gln
                   100                 105                 110
   aat agc cta caa gaa ctg tgg tct ttg ctt aac ttt att ctg ccc aat        383
   Asn Ser Leu Gln Glu Leu Trp Ser Leu Leu Asn Phe Ile Leu Pro Asn
               115                 120                 125
   att ttt aat tca tct cag aat ttt gag gaa tgg ttt aat gca cca ttt        431
   Ile Phe Asn Ser Ser Gln Asn Phe Glu Glu Trp Phe Asn Ala Pro Phe
           130                 135                 140
   gca tgt gat gtt agt ctt aat gat gag gaa cag cta tta atc ata cat        479
   Ala Cys Asp Val Ser Leu Asn Asp Glu Glu Gln Leu Leu Ile Ile His
       145                 150                 155
   cgt ctg cat caa gtt ttg cgt cca ttt ttg ctg agg agg aaa aaa gat        527
   Arg Leu His Gln Val Leu Arg Pro Phe Leu Leu Arg Arg Lys Lys Asp
   160                 165                 170                 175
   gaa gtg gaa aaa tat ctc cct gtc aaa aca caa gta att ctc aag tgt        575
   Glu Val Glu Lys Tyr Leu Pro Val Lys Thr Gln Val Ile Leu Lys Cys
                   180                 185                 190
   gat atg tct gct tgg caa aaa gca tac tat gaa caa gtc aca agc agg        623
   Asp Met Ser Ala Trp Gln Lys Ala Tyr Tyr Glu Gln Val Thr Ser Arg
               195                 200                 205
   gaa aag gtt gca cta gga ttt ggg ctc aga tca aag gct ctg cag aat        671
   Glu Lys Val Ala Leu Gly Phe Gly Leu Arg Ser Lys Ala Leu Gln Asn
           210                 215                 220
   ctg tca atg caa ctt agg aaa tgt tgc aac cac ccc tat cta ttt gta        719
   Leu Ser Met Gln Leu Arg Lys Cys Cys Asn His Pro Tyr Leu Phe Val
       225                 230                 235
   gag cac tac aac atg tac cag cgg gag gaa att gtt aga gca tca ggg        767
   Glu His Tyr Asn Met Tyr Gln Arg Glu Glu Ile Val Arg Ala Ser Gly
   240                 245                 250                 255
   aag ttt gaa ttg ctt gat cgt cta ctt cca aaa ctg cag aga gct ggt      815
   Lys Phe Glu Leu Leu Asp Arg Leu Leu Pro Lys Leu Gln Arg Ala Gly
                   260                 265                 270
   cac agg gtt ctg ctt ttc tct cag atg acg aaa ctg ctt gat gtt tta      863
   His Arg Val Leu Leu Phe Ser Gln Met Thr Lys Leu Leu Asp Val Leu
               275                 280                 285
   gaa ata tat ttg caa atg tac aat ttc aag tac atg agg ctt gat gga      911
   Glu Ile Tyr Leu Gln Met Tyr Asn Phe Lys Tyr Met Arg Leu Asp Gly
           290                 295                 300
   tcc acg aag act gaa gaa cga ggg agg tta ctg gca gat ttt aat aag      959
   Ser Thr Lys Thr Glu Glu Arg Gly Arg Leu Leu Ala Asp Phe Asn Lys
       305                 310                 315
   aag gat tcg gaa tat ttc atg ttt ctc ctc agc aca cgt gct gga gga     1007
   Lys Asp Ser Glu Tyr Phe Met Phe Leu Leu Ser Thr Arg Ala Gly Gly
   320                 325                 330                 335
   ctt ggg ttg aac ttg cag acg gcg gac act gtc att ata ttt gat agt     1055
   Leu Gly Leu Asn Leu Gln Thr Ala Asp Thr Val Ile Ile Phe Asp Ser
                   340                 345                 350
   gac tgg aac cct caa atg gac caa caa gct gaa gac cgt gcc cat cgt     1103
   Asp Trp Asn Pro Gln Met Asp Gln Gln Ala Glu Asp Arg Ala His Arg
               355                 360                 365
   ata ggc aga aga atg aag tgc gtg tgt ttg ttc ttg tta gtg tcg gct     1151
   Ile Gly Arg Arg Met Lys Cys Val Cys Leu Phe Leu Leu Val Ser Ala
           370                 375                 380
   cca ttg aag aag aga tcc tgg acc gtg caa aac aaa aga tgg gta tcg     1199
   Pro Leu Lys Lys Arg Ser Trp Thr Val Gln Asn Lys Arg Trp Val Ser
       385                 390                 395
   atg caa aag tta ctc cag gct ggg ttg ttt aac aca act tcc aca gca     1247
   Met Gln Lys Leu Leu Gln Ala Gly Leu Phe Asn Thr Thr Ser Thr Ala
   400                 405                 410                 415
   cag gac aga cga gca ttg ctg cag gag atc ctt agg agg ggg aca agc     1295
   Gln Asp Arg Arg Ala Leu Leu Gln Glu Ile Leu Arg Arg Gly Thr Ser
                   420                 425                 430
   tcg ctg gga aca gat atc ccc agt gag cgc gag ata aat cgt ttg gct     1343
   Ser Leu Gly Thr Asp Ile Pro Ser Glu Arg Glu Ile Asn Arg Leu Ala
               435                 440                 445
   gca cga act gat gaa gaa ttc tgg ttg ttt gag aag atg gat gaa gaa     1391
   Ala Arg Thr Asp Glu Glu Phe Trp Leu Phe Glu Lys Met Asp Glu Glu
           450                 455                 460
   agg agg ctt aga gaa aac tac aaa tct aga ctt atg gat ggg aat gag     1439
   Arg Arg Leu Arg Glu Asn Tyr Lys Ser Arg Leu Met Asp Gly Asn Glu
       465                 470                 475
   gtt cca gat tgg gta ttc gcc aac aat gat tta ccc aag aga acc gtg      1487
   Val Pro Asp Trp Val Phe Ala Asn Asn Asp Leu Pro Lys Arg Thr Val
   480                 485                 490                 495
   gca gat gag ttc cag aat ata atg gtc ggt gcg aag cga cgt aga aag      1535
   Ala Asp Glu Phe Gln Asn Ile Met Val Gly Ala Lys Arg Arg Arg Lys
                   500                 505                 510
   gag gtt gtc tat tca gac tct ttc ggt gat cag tgg atg aaa tcc gat      1583
   Glu Val Val Tyr Ser Asp Ser Phe Gly Asp Gln Trp Met Lys Ser Asp
               515                 520                 525
   gag gga ttt gaa gac att cca aag gcg act cag agg tcg aag aag act      1631
   Glu Gly Phe Glu Asp Ile Pro Lys Ala Thr Gln Arg Ser Lys Lys Thr
           530                 535                 540
   gct tac tca tct gac atc caa gtt gag ttt agt gaa agg agg aaa aga      1679
   Ala Tyr Ser Ser Asp Ile Gln Val Glu Phe Ser Glu Arg Arg Lys Arg
       545                 550                 555
   cct agg tct gta gaa aac agc gca gac ggt gtg agc aac ccg acg tgg      1727
   Pro Arg Ser Val Glu Asn Ser Ala Asp Gly Val Ser Asn Pro Thr Trp
   560                 565                 570                 575
   acg cct gac aaa gga agg gct gga gtt tca tca tac agc aag gac gag      1775
   Thr Pro Asp Lys Gly Arg Ala Gly Val Ser Ser Tyr Ser Lys Asp Glu
                   580                 585                 590
   act gaa gat gat ggc gaa gac gaa gtc att act agc ggc tta caa aag      1823
   Thr Glu Asp Asp Gly Glu Asp Glu Val Ile Thr Ser Gly Leu Gln Lys
               595                 600                 605
   gga aac agt ttc aca tgg aat acc cta gga aga aga agg tca agc cac      1871
   Gly Asn Ser Phe Thr Trp Asn Thr Leu Gly Arg Arg Arg Ser Ser His
           610                 615                 620
   ttc agt tcg tca tcg gac tcg aga ggg cgc cca aca ttc taa              1913
   Phe Ser Ser Ser Ser Asp Ser Arg Gly Arg Pro Thr Phe  *
       625                 630                 635
<210> 10
   <211> 636
   <212> PRT
   <213> Zea mays
<400> 10
   His Leu Ile Ile Ala Pro Lys Ala Val Leu Pro Asn Trp Ser Asn Glu
    1               5                  10                  15
   Phe Lys Thr Trp Ala Pro Ser Ile Gly Thr Ile Leu Tyr Asp Gly Arg
               20                  25                  30
   Pro Glu Glu Arg Lys Leu Leu Arg Glu Lys Asn Phe Asp Gly Leu Gln
           35                  40                  45
   Phe Asn Val Leu Leu Thr His Tyr Asp Leu Ile Leu Lys Asp Lys Lys
       50                  55                  60
   Phe Leu Lys Lys Val His Trp His Tyr Leu Ile Val Asp Glu Gly His
   65                  70                  75                  80
   Arg Leu Lys Asn His Glu Cys Ala Leu Ala Arg Thr Leu Val Ser Gly
   85 90 95
   Tyr Gln Ile Arg Arg Arg Leu Leu Leu Thr Gly Thr Pro Ile Gln Asn
   100 105 110
   Ser Leu Gln Glu Leu Trp Ser Leu Leu Asn Phe Ile Leu Pro Asn Ile
   115 120 125
   Phe Asn Ser Ser Gln Asn Phe Glu Glu Trp Phe Asn Ala Pro Phe Ala
   130 135 140
   Cys Asp Val Ser Leu Asn Asp Glu Glu Gln Leu Leu Ile Ile His Arg
   145 150 155 160
   Leu His Gln Val Leu Arg Pro Phe Leu Leu Arg Arg Lys Lys Asp Glu
   165 170 175
   Val Glu Lys Tyr Leu Pro Val Lys Thr Gln Val Ile Leu Lys Cys Asp
   180 185 190
   Met Ser Ala Trp Gln Lys Ala Tyr Tyr Glu Gln Val Thr Ser Arg Glu
   195 200 205
   Lys Val Ala Leu Gly Phe Gly Leu Arg Ser Lys Ala Leu Gln Asn Leu
   210 215 220
   Ser Met Gln Leu Arg Lys Cys Cys Asn His Pro Tyr Leu Phe Val Glu
   225 230 235 240
   His Tyr Asn Met Tyr Gln Arg Glu Glu Ile Val Arg Ala Ser Gly Lys
   245 250 255
   Phe Glu Leu Leu Asp Arg Leu Leu Pro Lys Leu Gln Arg Ala Gly His
   260 265 270
   Arg Val Leu Leu Phe Ser Gln Met Thr Lys Leu Leu Asp Val Leu Glu
   275 280 285
   Ile Tyr Leu Gln Met Tyr Asn Phe Lys Tyr Met Arg Leu Asp Gly Ser
   290 295 300
   Thr Lys Thr Glu Glu Arg Gly Arg Leu Leu Ala Asp Phe Asn Lys Lys
   305 310 315 320
   Asp Ser Glu Tyr Phe Met Phe Leu Leu Ser Thr Arg Ala Gly Gly Leu
   325 330 335
   Gly Leu Asn Leu Gln Thr Ala Asp Thr Val Ile Ile Phe Asp Ser Asp
   340 345 350
   Trp Asn Pro Gln Met Asp Gln Gln Ala Glu Asp Arg Ala His Arg Ile
   355 360 365
   Gly Arg Arg Met Lys Cys Val Cys Leu Phe Leu Leu Val Ser Ala Pro
   370 375 380
   Leu Lys Lys Arg Ser Trp Thr Val Gln Asn Lys Arg Trp Val Ser Met
   385 390 395 400
   Gln Lys Leu Leu Gln Ala Gly Leu Phe Asn Thr Thr Ser Thr Ala Gln
   405 410 415
   Asp Arg Arg Ala Leu Leu Gln Glu Ile Leu Arg Arg Gly Thr Ser Ser
   420 425 430
   Leu Gly Thr Asp Ile Pro Ser Glu Arg Glu Ile Asn Arg Leu Ala Ala
   435 440 445
   Arg Thr Asp Glu Glu Phe Trp Leu Phe Glu Lys Met Asp Glu Glu Arg
   450 455 460
   Arg Leu Arg Glu Asn Tyr Lys Ser Arg Leu Met Asp Gly Asn Glu Val
   465 470 475 480
   Pro Asp Trp Val Phe Ala Asn Asn Asp Leu Pro Lys Arg Thr Val Ala
   485 490 495
   Asp Glu Phe Gln Asn Ile Met Val Gly Ala Lys Arg Arg Arg Lys Glu
   500 505 510
   Val Val Tyr Ser Asp Ser Phe Gly Asp Gln Trp Met Lys Ser Asp Glu
   515 520 525
   Gly Phe Glu Asp Ile Pro Lys Ala Thr Gln Arg Ser Lys Lys Thr Ala
       530                 535                 540
   Tyr Ser Ser Asp Ile Gln Val Glu Phe Ser Glu Arg Arg Lys Arg Pro
   545                 550                 555                 560
   Arg Ser Val Glu Asn Ser Ala Asp Gly Val Ser Asn Pro Thr Trp Thr
                   565                 570                 575
   Pro Asp Lys Gly Arg Ala Gly Val Ser Ser Tyr Ser Lys Asp Glu Thr
               580                 585                 590
   Glu Asp Asp Gly Glu Asp Glu Val Ile Thr Ser Gly Leu Gln Lys Gly
           595                 600                 605
   Asn Ser Phe Thr Trp Asn Thr Leu Gly Arg Arg Arg Ser Ser His Phe
       610                 615                 620
   Ser Ser Ser Ser Asp Ser Arg Gly Arg Pro Thr Phe
   625                 630                 635
<210> 11
   <211> 23
   <212> DNA
   <213> Artificial Sequence
<220>
   <221> primer bind
   <222> (1) ... (23)
<400> 11
   cgaattcaaa acctgggctc cca 23
<210> 12
   <211> 21
   <212> DNA
   <213> Artificial Sequence
<220>
   <221> primer bind
   <222> (1) ... (21)
<400> 12
   ttagaatgtt gggcgccctc t 21
<210> 13
   <211> 1463
   <212> DNA
   <213> Zea mays
<220>
   <221> CDS
   <222> (3)...(1463)
   <221> misc_feature
   <222> (1) ... (1463)
   <223> n= A,T,C or G
<400> 13
   gt cga ccc acg cgt ccg cca gaa gag cgg aac cat ata agg gac aat        47
      Arg Pro Thr Arg Pro Pro Glu Glu Arg Asn His Ile Arg Asp Asn
       1               5                  10                  15
   ttg ctg caa cct ggg aaa ttt gat gtg tgt gtg act agt ttt gaa atg       95
   Leu Leu Gln Pro Gly Lys Phe Asp Val Cys Val Thr Ser Phe Glu Met
                   20                  25                  30
   gca atc aaa gaa aaa tct gcg ttg agg cgc ttc agc tgg cgc tac ata      143
   Ala Ile Lys Glu Lys Ser Ala Leu Arg Arg Phe Ser Trp Arg Tyr Ile
               35                  40                  45
   atc att gat gaa gct cac cgg ata aaa aat gaa aat tct ctt cta tca      191
   Ile Ile Asp Glu Ala His Arg Ile Lys Asn Glu Asn Ser Leu Leu Ser
           50                  55                  60
   aag act atg agg att tac aac act aat tat cgt ctc ctc atc aca ggc      239
   Lys Thr Met Arg Ile Tyr Asn Thr Asn Tyr Arg Leu Leu Ile Thr Gly
       65                  70                  75
   act cca ctc cag aat aat ctc cat gag ctc tgg gct ctc ctc aat ttc      287
   Thr Pro Leu Gln Asn Asn Leu His Glu Leu Trp Ala Leu Leu Asn Phe
   80                  85                  90                  95
   ttg cta cct gaa ata ttt agc tct gcg gag acc ttt gat gaa tgg ttt      335
   Leu Leu Pro Glu Ile Phe Ser Ser Ala Glu Thr Phe Asp Glu Trp Phe
                   100                 105                 110
   caa ata tct ggg gaa aat gat caa cag gag gtg gtg cag cag ctt cat      383
   Gln Ile Ser Gly Glu Asn Asp Gln Gln Glu Val Val Gln Gln Leu His
               115                 120                 125
   aag gtt ctt cgc cca ttc ctt ctt agg agg ctc aag tct gat gta naa      431
   Lys Val Leu Arg Pro Phe Leu Leu Arg Arg Leu Lys Ser Asp Val Xaa
           130                 135                 140
   aag ggc cta cct cca aag aaa gaa aca att ctt aaa gtt gga atg tct      479
   Lys Gly Leu Pro Pro Lys Lys Glu Thr Ile Leu Lys Val Gly Met Ser
       145                 150                 155
   cag atg caa aag cag tac tat cgt gct ctg ctt cag aag gat ttg gag      527
   Gln Met Gln Lys Gln Tyr Tyr Arg Ala Leu Leu Gln Lys Asp Leu Glu
   160                 165                 170                 175
   gtt att aat gct ggt ggt gaa cgc aag cga ttg ctt aac att gcc atg      575
   Val Ile Asn Ala Gly Gly Glu Arg Lys Arg Leu Leu Asn Ile Ala Met
                   180                 185                 190
   cag ttg cgc aag tgc tgc aac cat cca tat tta ttc caa gga gct gaa      623
   Gln Leu Arg Lys Cys Cys Asn His Pro Tyr Leu Phe Gln Gly Ala Glu
               195                 200                 205
   cct ggg cca ccc tac aca act ggt gaa cat cta att gag aat gca gga      671
   Pro Gly Pro Pro Tyr Thr Thr Gly Glu His Leu Ile Glu Asn Ala Gly
           210                 215                 220
   aaa atg gtt cta ctt gat aaa ttg ctg ccc aag cta aag gag cgt gat      719
   Lys Met Val Leu Leu Asp Lys Leu Leu Pro Lys Leu Lys Glu Arg Asp
       225                 230                 235
   tcc aga gtc ctt att ttt tca cag atg acc agg ctt ttg gat atc ttg      767
   Ser Arg Val Leu Ile Phe Ser Gln Met Thr Arg Leu Leu Asp Ile Leu
   240                 245                 250                 255
   gaa gat tat ctt atg tat agg gga tat cag tat tgt cga att gat gga      815
   Glu Asp Tyr Leu Met Tyr Arg Gly Tyr Gln Tyr Cys Arg Ile Asp Gly
                   260                 265                 270
   aat aca ggt gga gaa gat cgt gat gca tcc att gaa gcc ttc aat agt      863
   Asn Thr Gly Gly Glu Asp Arg Asp Ala Ser Ile Glu Ala Phe Asn Ser
               275                 280                 285
   cca gga agt gag aag ttt gtt ttc tta ctt tca act agg gca ggt ggc      911
   Pro Gly Ser Glu Lys Phe Val Phe Leu Leu Ser Thr Arg Ala Gly Gly
           290                 295                 300
   ctt ggt atc aac ttg gcc act gct gat gtt gtg gtt ctc tat gac agc      959
   Leu Gly Ile Asn Leu Ala Thr Ala Asp Val Val Val Leu Tyr Asp Ser
       305                 310                 315
   gat tgg aat ccc caa gct gat ctg caa gct cag gac cgt gca cat aga     1007
   Asp Trp Asn Pro Gln Ala Asp Leu Gln Ala Gln Asp Arg Ala His Arg
   320                 325                 330                 335
   ata ggt caa aaa gaa aga agt tca agt gtt ccg ctt ttg cac ttg agt     1055
   Ile Gly Gln Lys Glu Arg Ser Ser Ser Val Pro Leu Leu His Leu Ser
                   340                 345                 350
   tca act att gag gaa aag gtg att gag aga gca tat aag aag cta gca     1103
   Ser Thr Ile Glu Glu Lys Val Ile Glu Arg Ala Tyr Lys Lys Leu Ala
               355                 360                 365
   ttg gat gct ttg gtt att cag caa gga cga ttg gca gag cag aaa act     1151
   Leu Asp Ala Leu Val Ile Gln Gln Gly Arg Leu Ala Glu Gln Lys Thr
           370                 375                 380
   gtc aat aag gat gat ctt ctg caa atg gtg cgg ttt ggt gct gaa atg     1199
   Val Asn Lys Asp Asp Leu Leu Gln Met Val Arg Phe Gly Ala Glu Met
       385                 390                 395
   gtt ttc agt tct aag gac agc aca ata act gat gag gac att gac cgt     1247
   Val Phe Ser Ser Lys Asp Ser Thr Ile Thr Asp Glu Asp Ile Asp Arg
   400                 405                 410                 415
   att ata gct aaa gga gag gag aca aca gca gaa ctt gat gcg aaa atg     1295
   Ile Ile Ala Lys Gly Glu Glu Thr Thr Ala Glu Leu Asp Ala Lys Met
                   420                 425                 430
   aaa aag ttc act gag gat gcc atc aaa ttt aag atg gat gat aat gct     1343
   Lys Lys Phe Thr Glu Asp Ala Ile Lys Phe Lys Met Asp Asp Asn Ala
               435                 440                 445
   gaa ttg tat gac ttc gat gat gag aag gat gaa aac aag gtt gat ttc     1391
   Glu Leu Tyr Asp Phe Asp Asp Glu Lys Asp Glu Asn Lys Val Asp Phe
           450                 455                 460
   aag aaa ctt gtt agt gat aac tgg att gag cca cct aga aga gaa agg     1439
   Lys Lys Leu Val Ser Asp Asn Trp Ile Glu Pro Pro Arg Arg Glu Arg
       465                 470                 475
   aag nga aac tac tct gag tct tga                              1463
   Lys Xaa Asn Tyr Ser Glu Ser  *
   480                 485
<210> 14
   <211> 486
   <212> PRT
   <213> Zea mays
<220>
   <221> VARIANT
   <222> (1)...(486)
   <223> Xaa = Any Amino Acid
<400> 14
   Arg Pro Thr Arg Pro Pro Glu Glu Arg Asn His Ile Arg Asp Asn Leu
    1               5                  10                  15
   Leu Gln Pro Gly Lys Phe Asp Val Cys Val Thr Ser Phe Glu Met Ala
               20                  25                  30
   Ile Lys Glu Lys Ser Ala Leu Arg Arg Phe Ser Trp Arg Tyr Ile Ile
           35                  40                  45
   Ile Asp Glu Ala His Arg Ile Lys Asn Glu Asn Ser Leu Leu Ser Lys
       50                  55                  60
   Thr Met Arg Ile Tyr Asn Thr Asn Tyr Arg Leu Leu Ile Thr Gly Thr
   65                  70                  75                  80
   Pro Leu Gln Asn Asn Leu His Glu Leu Trp Ala Leu Leu Asn Phe Leu
                   85                  90                  95
   Leu Pro Glu Ile Phe Ser Ser Ala Glu Thr Phe Asp Glu Trp Phe Gln
               100                 105                 110
   Ile Ser Gly Glu Asn Asp Gln Gln Glu Val Val Gln Gln Leu His Lys
           115                 120                 125
   Val Leu Arg Pro Phe Leu Leu Arg Arg Leu Lys Ser Asp Val Xaa Lys
       130                 135                 140
   Gly Leu Pro Pro Lys Lys Glu Thr Ile Leu Lys Val Gly Met Ser Gln
   145                 150                 155                 160
   Met Gln Lys Gln Tyr Tyr Arg Ala Leu Leu Gln Lys Asp Leu Glu Val
                   165                 170                 175
   Ile Asn Ala Gly Gly Glu Arg Lys Arg Leu Leu Asn Ile Ala Met Gln
               180                 185                 190
   Leu Arg Lys Cys Cys Asn His Pro Tyr Leu Phe Gln Gly Ala Glu Pro
           195                 200                 205
   Gly Pro Pro Tyr Thr Thr Gly Glu His Leu Ile Glu Asn Ala Gly Lys
       210                 215                 220
   Met Val Leu Leu Asp Lys Leu Leu Pro Lys Leu Lys Glu Arg Asp Ser
   225                 230                 235                 240
   Arg Val Leu Ile Phe Ser Gln Met Thr Arg Leu Leu Asp Ile Leu Glu
                   245                 250                 255
   Asp Tyr Leu Met Tyr Arg Gly Tyr Gln Tyr Cys Arg Ile Asp Gly Asn
               260                 265                 270
   Thr Gly Gly Glu Asp Arg Asp Ala Ser Ile Glu Ala Phe Asn Ser Pro
           275                 280                 285
   Gly Ser Glu Lys Phe Val Phe Leu Leu Ser Thr Arg Ala Gly Gly Leu
       290                 295                 300
   Gly Ile Asn Leu Ala Thr Ala Asp Val Val Val Leu Tyr Asp Ser Asp
   305                 310                 315                 320
   Trp Asn Pro Gln Ala Asp Leu Gln Ala Gln Asp Arg Ala His Arg Ile
   325 330 335
   Gly Gln Lys Glu Arg Ser Ser Ser Val Pro Leu Leu His Leu Ser Ser
   340 345 350
   Thr Ile Glu Glu Lys Val Ile Glu Arg Ala Tyr Lys Lys Leu Ala Leu
   355 360 365
   Asp Ala Leu Val Ile Gln Gln Gly Arg Leu Ala Glu Gln Lys Thr Val
   370 375 380
   Asn Lys Asp Asp Leu Leu Gln Met Val Arg Phe Gly Ala Glu Met Val
   385 390 395 400
   Phe Ser Ser Lys Asp Ser Thr Ile Thr Asp Glu Asp Ile Asp Arg Ile
   405 410 415
   Ile Ala Lys Gly Glu Glu Thr Thr Ala Glu Leu Asp Ala Lys Met Lys
   420 425 430
   Lys Phe Thr Glu Asp Ala Ile Lys Phe Lys Met Asp Asp Asn Ala Glu
   435 440 445
   Leu Tyr Asp Phe Asp Asp Glu Lys Asp Glu Asn Lys Val Asp Phe Lys
   450 455 460
   Lys Leu Val Ser Asp Asn Trp Ile Glu Pro Pro Arg Arg Glu Arg Lys
   465 470 475 480
   Xaa Asn Tyr Ser Glu Ser
   485
<210> 15
   <211> 23
   <212> DNA
   <213> Artificial Sequence
<220>
   <221> primer_bind
   <222> (1) ... (23)
<400> 15
   ccagaagagc ggaaccatat aag 23
<210> 16
   <211> 23
   <212> DNA
   <213> Artificial Sequence
<220>
   <221> primer_bind
   <222> (1)...(23)
<400> 16
   ctcttctagg tggctcaatc cag 23
<210> 17
   <211> 1645
   <212> DNA
   <213> Zea mays
<220>
   <221> CDS
   <222> (2)...(1645)
   <221> misc_feature
   <222> (1)...(1645)
   <223> n = A,T,C or G
<400> 17
   a gca gat ggg aga aga tac atg atc cgc cgg aga cta ctt tta aca ggc      49
     Ala Asp Gly Arg Arg Tyr Met Ile Arg Arg Arg Leu Leu Leu Thr Gly
      1               5                  10                  15
   act cct atc caa aac agc ctg caa gag ctc tgg tct ttg ctt aac ttc        97
   Thr Pro Ile Gln Asn Ser Leu Gln Glu Leu Trp Ser Leu Leu Asn Phe
               20                  25                  30
   atc ctg ccc aat att ttt aat tca tcc cag aat ttt gag gaa tgg ttt       145
   Ile Leu Pro Asn Ile Phe Asn Ser Ser Gln Asn Phe Glu Glu Trp Phe
           35                  40                  45
   aat gca cca ttt gca tgt gat gtc agt ctt aat gat gag gaa caa cta       193
   Asn Ala Pro Phe Ala Cys Asp Val Ser Leu Asn Asp Glu Glu Gln Leu
       50                  55                  60
   cta atc ata cat cgt ttg cat caa gtt ttg cgt cca ttc ttg ctg agg       241
   Leu Ile Ile His Arg Leu His Gln Val Leu Arg Pro Phe Leu Leu Arg
   65                  70                  75                  80
   agg aag aaa gat gaa gta nag aaa tat ctc cct gtg aaa aca caa gta       289
   Arg Lys Lys Asp Glu Val Xaa Lys Tyr Leu Pro Val Lys Thr Gln Val
                   85                  90                  95
   att ctc aag tgt gac atg tct gct tgg caa aaa gca tac tac gaa caa       337
   Ile Leu Lys Cys Asp Met Ser Ala Trp Gln Lys Ala Tyr Tyr Glu Gln
               100                 105                 110
   gtc aca agc agg gaa aag gtt gcg cta gga tat ggg atc aga aag aag       385
   Val Thr Ser Arg Glu Lys Val Ala Leu Gly Tyr Gly Ile Arg Lys Lys
           115                 120                 125
   gct ctg caa aat ctg tca atg caa ctt agg aag tgt tgc aat cat ccc       433
   Ala Leu Gln Asn Leu Ser Met Gln Leu Arg Lys Cys Cys Asn His Pro
       130                 135                 140
   tac cta ttc gta gag cat tat aac atg tac caa cgg gag gaa ata gtt       481
   Tyr Leu Phe Val Glu His Tyr Asn Met Tyr Gln Arg Glu Glu Ile Val
   145                 150                 155                 160
   aga gca tcc gga aag ttt gaa ttg ctt gat cgt cta ctt ccg aaa ttg       529
   Arg Ala Ser Gly Lys Phe Glu Leu Leu Asp Arg Leu Leu Pro Lys Leu
                   165                 170                 175
   cag aga gct ggt cac agg gtt tta ctt ttc tct cag atg aca aaa ttg       577
   Gln Arg Ala Gly His Arg Val Leu Leu Phe Ser Gln Met Thr Lys Leu
               180                 185                 190
   ctt gac gtt tta gaa ata tat ttg cag atg tac aat ttc aag tac atg       625
   Leu Asp Val Leu Glu Ile Tyr Leu Gln Met Tyr Asn Phe Lys Tyr Met
           195                 200                 205
   agg ctt gat gga tcc aca aag act gaa gaa cgt ggg agg tta ctg gca       673
   Arg Leu Asp Gly Ser Thr Lys Thr Glu Glu Arg Gly Arg Leu Leu Ala
       210                 215                 220
   gat ttt aat aag aag aat tca gaa tat ttc atg ttt ctt ctc agc aca      721
   Asp Phe Asn Lys Lys Asn Ser Glu Tyr Phe Met Phe Leu Leu Ser Thr
   225                 230                 235                 240
   cga gcc gga ggt ctt gga ttg aac ttg cag act gca gac acc gtc att      769
   Arg Ala Gly Gly Leu Gly Leu Asn Leu Gln Thr Ala Asp Thr Val Ile
                   245                 250                 255
   atc ttt gat agt gac tgg aac cct cag atg gac caa caa gct gag gac      817
   Ile Phe Asp Ser Asp Trp Asn Pro Gln Met Asp Gln Gln Ala Glu Asp
               260                 265                 270
   cgt gcc cat cgt ata ggg caa aag aac gaa gta cgt gtg ttt gtt ctt      865
   Arg Ala His Arg Ile Gly Gln Lys Asn Glu Val Arg Val Phe Val Leu
           275                 280                 285
   gtt agc gtt ggt tca att gaa gaa gag ata ttg gat cgt gcg aaa cag      913
   Val Ser Val Gly Ser Ile Glu Glu Glu Ile Leu Asp Arg Ala Lys Gln
       290                 295                 300
   aag atg ggt att gat gca aaa gta atc cag gct ggg ttg ttt aac acg      961
   Lys Met Gly Ile Asp Ala Lys Val Ile Gln Ala Gly Leu Phe Asn Thr
   305                 310                 315                 320
   acc tcc aca gca cag gac agg cga gca ttg ctg cag gag ata ctc agg      1009
   Thr Ser Thr Ala Gln Asp Arg Arg Ala Leu Leu Gln Glu Ile Leu Arg
                   325                 330                 335
   aga gga aca agc tca ctg gga acg gat atc ccc agt gaa cgt gag ata      1057
   Arg Gly Thr Ser Ser Leu Gly Thr Asp Ile Pro Ser Glu Arg Glu Ile
               340                 345                 350
   aac cgc ttg gct gct cga aac gat gaa gaa ttc cgg ttg ttt gag aag      1105
   Asn Arg Leu Ala Ala Arg Asn Asp Glu Glu Phe Arg Leu Phe Glu Lys
           355                 360                 365
   atg gat gaa gaa agg agg cta aag gag aac tac aaa tct aga ctt atg      1153
   Met Asp Glu Glu Arg Arg Leu Lys Glu Asn Tyr Lys Ser Arg Leu Met
       370                 375                 380
   gat gga aat gag gtc cca gat tgg gtg ttt gcc aat gat aat gaa acc      1201
   Asp Gly Asn Glu Val Pro Asp Trp Val Phe Ala Asn Asp Asn Glu Thr
   385                 390                 395                 400
   tta cgc aag aaa acc gtg gca gat gaa ttc cgg aat ata att gtt ggt      1249
   Leu Arg Lys Lys Thr Val Ala Asp Glu Phe Arg Asn Ile Ile Val Gly
                   405                 410                 415
   tca aag aga cgt aga aag gag gtt gtc tat tcg gac tct ttt ggt gat      1297
   Ser Lys Arg Arg Arg Lys Glu Val Val Tyr Ser Asp Ser Phe Gly Asp
               420                 425                 430
   cag tgg atg aaa tcc gac gag gga ttt gaa gag att gca aag atg act      1345
   Gln Trp Met Lys Ser Asp Glu Gly Phe Glu Glu Ile Ala Lys Met Thr
               435                     440                     445
   cca agg gtg aag cga act gct tat tcg cct gac att caa gtt gag tac      1393
   Pro Arg Val Lys Arg Thr Ala Tyr Ser Pro Asp Ile Gln Val Glu Tyr
       450                     455                     460
   aat gaa agg agg aaa agg ccc aag tct gtg gaa aac agc gca gat ggc      1441
   Asn Glu Arg Arg Lys Arg Pro Lys Ser Val Glu Asn Ser Ala Asp Gly
   465                     470                     475                     480
   gca agc aac cca aca cgg aca ccc gac aaa gga agg gct gga gtt tca      1489
   Ala Ser Asn Pro Thr Arg Thr Pro Asp Lys Gly Arg Ala Gly Val Ser
                   485                     490                     495
   tca tac agc aag gat gag acc gaa gat gat ggt gaa gac gaa gtc atc      1537
   Ser Tyr Ser Lys Asp Glu Thr Glu Asp Asp Gly Glu Asp Glu Val Ile
               500                     505                     510
   acc agt ggc tta cag aag ggt aac agt ttc aca tgg aag acc ctt gga      1585
   Thr Ser Gly Leu Gln Lys Gly Asn Ser Phe Thr Trp Lys Thr Leu Gly
           515                     520                     525
   aga aaa agg tca agc cac tta agt tcg tcg tcg gac tca aaa ggg cga      1633
   Arg Lys Arg Ser Ser His Leu Ser Ser Ser Ser Asp Ser Lys Gly Arg
       530                     535                     540
   cca tca ttc taa                                                      1645
   Pro Ser Phe  *
   545
<210> 18
   <211> 547
   <212> PRT
   <213> Zea mays
<220>
   <221> VARIANT
   <222> (1)...(547)
   <223> Xaa = Any Amino Acid
<400> 18
   Ala Asp Gly Arg Arg Tyr Met Ile Arg Arg Arg Leu Leu Leu Thr Gly
    1               5                  10                  15
   Thr Pro Ile Gln Asn Ser Leu Gln Glu Leu Trp Ser Leu Leu Asn Phe
               20                  25                  30
   Ile Leu Pro Asn Ile Phe Asn Ser Ser Gln Asn Phe Glu Glu Trp Phe
           35                  40                  45
   Asn Ala Pro Phe Ala Cys Asp Val Ser Leu Asn Asp Glu Glu Gln Leu
       50                  55                  60
   Leu Ile Ile His Arg Leu His Gln Val Leu Arg Pro Phe Leu Leu Arg
   65                  70                  75                  80
   Arg Lys Lys Asp Glu Val Xaa Lys Tyr Leu Pro Val Lys Thr Gln Val
                   85                  90                  95
   Ile Leu Lys Cys Asp Met Ser Ala Trp Gln Lys Ala Tyr Tyr Glu Gln
               100                 105                 110
   Val Thr Ser Arg Glu Lys Val Ala Leu Gly Tyr Gly Ile Arg Lys Lys
           115                     120                     125
   Ala Leu Gln Asn Leu Ser Met Gln Leu Arg Lys Cys Cys Asn His Pro
       130                     135                     140
   Tyr Leu Phe Val Glu His Tyr Asn Met Tyr Gln Arg Glu Glu Ile Val
   145                     150                     155                     160
   Arg Ala Ser Gly Lys Phe Glu Leu Leu Asp Arg Leu Leu Pro Lys Leu
                   165                     170                     175
   Gln Arg Ala Gly His Arg Val Leu Leu Phe Ser Gln Met Thr Lys Leu
               180                     185                     190
   Leu Asp Val Leu Glu Ile Tyr Leu Gln Met Tyr Asn Phe Lys Tyr Met
           195                     200                     205
   Arg Leu Asp Gly Ser Thr Lys Thr Glu Glu Arg Gly Arg Leu Leu Ala
       210                     215                     220
   Asp Phe Asn Lys Lys Asn Ser Glu Tyr Phe Met Phe Leu Leu Ser Thr
   225                     230                     235                     240
   Arg Ala Gly Gly Leu Gly Leu Asn Leu Gln Thr Ala Asp Thr Val Ile
                   245                     250                     255
   Ile Phe Asp Ser Asp Trp Asn Pro Gln Met Asp Gln Gln Ala Glu Asp
               260                     265                     270
   Arg Ala His Arg Ile Gly Gln Lys Asn Glu Val Arg Val Phe Val Leu
           275                     280                     285
   Val Ser Val Gly Ser Ile Glu Glu Glu Ile Leu Asp Arg Ala Lys Gln
       290                     295                     300
   Lys Met Gly Ile Asp Ala Lys Val Ile Gln Ala Gly Leu Phe Asn Thr
   305                     310                     315                     320
   Thr Ser Thr Ala Gln Asp Arg Arg Ala Leu Leu Gln Glu Ile Leu Arg
                   325                     330                     335
   Arg Gly Thr Ser Ser Leu Gly Thr Asp Ile Pro Ser Glu Arg Glu Ile
               340                     345                     350
   Asn Arg Leu Ala Ala Arg Asn Asp Glu Glu Phe Arg Leu Phe Glu Lys
           355                     360                     365
   Met Asp Glu Glu Arg Arg Leu Lys Glu Asn Tyr Lys Ser Arg Leu Met
       370                     375                     380
   Asp Gly Asn Glu Val Pro Asp Trp Val Phe Ala Asn Asp Asn Glu Thr
   385                     390                     395                     400
   Leu Arg Lys Lys Thr Val Ala Asp Glu Phe Arg Asn Ile Ile Val Gly
                   405                     410                     415
   Ser Lys Arg Arg Arg Lys Glu Val Val Tyr Ser Asp Ser Phe Gly Asp
               420                     425                     430
   Gln Trp Met Lys Ser Asp Glu Gly Phe Glu Glu Ile Ala Lys Met Thr
           435                     440                     445
   Pro Arg Val Lys Arg Thr Ala Tyr Ser Pro Asp Ile Gln Val Glu Tyr
       450                     455                     460
   Asn Glu Arg Arg Lys Arg Pro Lys Ser Val Glu Asn Ser Ala Asp Gly
   465                     470                     475                     480
   Ala Ser Asn Pro Thr Arg Thr Pro Asp Lys Gly Arg Ala Gly Val Ser
                   485                     490                     495
   Ser Tyr Ser Lys Asp Glu Thr Glu Asp Asp Gly Glu Asp Glu Val Ile
               500                     505                     510
   Thr Ser Gly Leu Gln Lys Gly Asn Ser Phe Thr Trp Lys Thr Leu Gly
           515                     520                     525
   Arg Lys Arg Ser Ser His Leu Ser Ser Ser Ser Asp Ser Lys Gly Arg
       530                     535                     540
   Pro Ser Phe
   545
<210> 19
   <211> 23
   <212> DNA
   <213> Artificial Sequence
<220>
   <221> primer bind
   <222> (1) ... (23)
<400> 19
   acaggcactc ctatccaaaa cag 23
<210> 20
   <211> 23
   <212> DNA
   <213> Artificial Sequence
<220>
   <221> primer_bind
   <222> (1) ... (23)
<400> 20
   gaatgatggt cgcccttttg agt 23
<210> 21
   <211> 514
   <212> DNA
   <213> Glycine max
<220>
   <221> CDS
   <222> (6) ... (514)
   <221> misc feature
   <222> (1) ... (514)
   <223> n = A,T,C or G
<400> 21
   ccnta aat ttc ttg tta ccc aaa cnt nat caa ttt cat cca gga gga ctt      50
         Asn Phe Leu Leu Pro Lys Xaa Xaa Gln Phe His Pro Gly Gly Leu
          1               5                  10                  15
   ctc tca aat ggt tta ata agc cat ttg aga gtg ctt gga gat agc tcg      98
   Leu Ser Asn Gly Leu Ile Ser His Leu Arg Val Leu Gly Asp Ser Ser
                   20                  25                  30
   cct gat gaa gct tta ntg tcc gag gag gag aat ctc ttg att ata aat     146
   Pro Asp Glu Ala Leu Xaa Ser Glu Glu Glu Asn Leu Leu Ile Ile Asn
               35                  40                  45
   cgt ctg cac caa gtt ttg aga cca ttt gta ctt agg agg ctg aaa cac     194
   Arg Leu His Gln Val Leu Arg Pro Phe Val Leu Arg Arg Leu Lys His
           50                  55                  60
   aag gtt gaa aat gag ttg cct gag aag att gag aga cta ata aga tgt     242
   Lys Val Glu Asn Glu Leu Pro Glu Lys Ile Glu Arg Leu Ile Arg Cys
       65                  70                  75
   gag gcc tca tca tat caa aaa ctt ttg atg aag agg gtg gaa gaa aat      290
   Glu Ala Ser Ser Tyr Gln Lys Leu Leu Met Lys Arg Val Glu Glu Asn
    80                 85                  90                  95
   ctt ggt tct att ggc aat tca aag gct cga tca gta cac aac tct gtc      338
   Leu Gly Ser Ile Gly Asn Ser Lys Ala Arg Ser Val His Asn Ser Val
                   100                 105                 110
   atg gag ctt cgt aat ata tgc aat cat cca tat ctc agt cag ctt cat      386
   Met Glu Leu Arg Asn Ile Cys Asn His Pro Tyr Leu Ser Gln Leu His
               115                 120                 125
   gca gag gag gtg gat aac ttc ata cct aaa cat tat ctg cca cca att      434
   Ala Glu Glu Val Asp Asn Phe Ile Pro Lys His Tyr Leu Pro Pro Ile
           130                 135                 140
   att aga ctt tgt ggg aag ctt gag atg ttg gac cgt tta ttg cca aaa      482
   Ile Arg Leu Cys Gly Lys Leu Glu Met Leu Asp Arg Leu Leu Pro Lys
       145                 150                 155
   ttg aag gcg aca gat cat cgg gtt ctt ttc tt                           514
   Leu Lys Ala Thr Asp His Arg Val Leu Phe
   160                 165
<210> 22
   <211> 169
   <212> PRT
   <213> Glycine max
<220>
   <221> VARIANT
   <222> (1)...(169)
   <223> Xaa = Any Amino Acid
<400> 22
   Asn Phe Leu Leu Pro Lys Xaa Xaa Gln Phe His Pro Gly Gly Leu Leu
    1               5                  10                  15
   Ser Asn Gly Leu Ile Ser His Leu Arg Val Leu Gly Asp Ser Ser Pro
               20                  25                  30
   Asp Glu Ala Leu Xaa Ser Glu Glu Glu Asn Leu Leu Ile Ile Asn Arg
           35                  40                  45
   Leu His Gln Val Leu Arg Pro Phe Val Leu Arg Arg Leu Lys His Lys
       50                  55                  60
   Val Glu Asn Glu Leu Pro Glu Lys Ile Glu Arg Leu Ile Arg Cys Glu
   65                  70                  75                  80
   Ala Ser Ser Tyr Gln Lys Leu Leu Met Lys Arg Val Glu Glu Asn Leu
                   85                  90                  95
   Gly Ser Ile Gly Asn Ser Lys Ala Arg Ser Val His Asn Ser Val Met
               100                 105                 110
   Glu Leu Arg Asn Ile Cys Asn His Pro Tyr Leu Ser Gln Leu His Ala
           115                 120                 125
   Glu Glu Val Asp Asn Phe Ile Pro Lys His Tyr Leu Pro Pro Ile Ile
       130                 135                 140
   Arg Leu Cys Gly Lys Leu Glu Met Leu Asp Arg Leu Leu Pro Lys Leu
   145                 150                 155                 160
   Lys Ala Thr Asp His Arg Val Leu Phe
   165
<210> 23
   <211> 23
   <212> DNA
   <213> Artificial Sequence
<220>
   <221> primer bind
   <222> (1) ... (23)
<400> 23
   aacccgatga tctgtcgcct tca 23
<210> 24
   <211> 23
   <212> DNA
   <213> Artificial Sequence
<220>
   <221> primer_bind
   <222> (1) ... (23)
<400> 24
   tcatccagga ggacttctct caa 23
<210> 25
   <211> 403
   <212> DNA
   <213> Glycine max
<220>
   <221> CDS
   <222> (221) ... (403)
   <221> misc_feature
   <222> (1) ... (403)
   <223> n = A,T,C or G
<400> 25
   tgaatatntn cttgntttta atttatgcga ntaaggattt gtgcattngg agattagtgt     60
   cnatgaatca agtgattgnt attttatttc atgtgtcacc cagccatatt ggcagatgaa    120
   atgggtcttg gcaaaacagt tcaggtacgt attctgtttt ttattatttt aatatgtttc    180
   ntaatttgtt tgtnttccta atcctttact tttcaagtaa gaa atg cca tat gtt      235
                                               Glu Met Pro Tyr Val
                                                1               5
   ctt gtc ttc cag gcc atc aca tat tta act ttg ctg aaa cac ttg cac      283
   Leu Val Phe Gln Ala Ile Thr Tyr Leu Thr Leu Leu Lys His Leu His
                    10                  15                  20
   aat gat tct ggt cca cat ctt ata gta tgt cct gct tct gtt ctg gaa      331
   Asn Asp Ser Gly Pro His Leu Ile Val Cys Pro Ala Ser Val Leu Glu
                25                  30                  35
   aac tgg gaa agg gaa tta aaa agg tgg tgt cca tcc ttt tct gtt ctt      379
   Asn Trp Glu Arg Glu Leu Lys Arg Trp Cys Pro Ser Phe Ser Val Leu
            40                  45                  50
   caa tac cat ggg gcc gga cgt gca                                      403
   Gln Tyr His Gly Ala Gly Arg Ala
        55                  60
<210> 26
   <211> 61
   <212> PRT
   <213> Glycine max
<220>
   <221> VARIANT
   <222> (1)...(126)
   <223> Xaa = Any Amino Acid
<400> 26
   Glu Met Pro Tyr Val Leu Val Phe Gln Ala Ile Thr Tyr Leu Thr Leu
    1               5                  10                  15
   Leu Lys His Leu His Asn Asp Ser Gly Pro His Leu Ile Val Cys Pro
               20                  25                  30
   Ala Ser Val Leu Glu Asn Trp Glu Arg Glu Leu Lys Arg Trp Cys Pro
           35                  40                  45
   Ser Phe Ser Val Leu Gln Tyr His Gly Ala Gly Arg Ala
       50                  55                  60
<210> 27
   <211> 23
   <212> DNA
   <213> Artificial Sequence
<220>
   <221> primer_bind
   <222> (1)...(23)
<400> 27
   gccccatggt attgaagaac aga 23
<210> 28
   <211> 25
   <212> DNA
   <213> Artificial Sequence
<220>
   <221> primer bind
   <222> (1)...(25)
<400> 28
   attttatttc atgtgtcacc cagcc 25
<210> 29
   <211> 522
   <212> DNA
   <213> Oryza sativa
<220>
   <221> CDS
   <222> (1)...(522)
   <221> misc_feature
   <222> (1)...(522)
   <223> n = A,T,C or G
<400> 29
   gtt tct ggg agg aag gct cag tat tct aag aaa aac tca cgt aat gta      48
   Val Ser Gly Arg Lys Ala Gln Tyr Ser Lys Lys Asn Ser Arg Asn Val
    1               5                  10                  15
   gat tca ctc cct ttg atg gag ggt gaa ggg cgt gct tta aaa gtt tat      96
   Asp Ser Leu Pro Leu Met Glu Gly Glu Gly Arg Ala Leu Lys Val Tyr
               20                  25                  30
   gga ttc aat cac gtt caa cga aca caa ttc cta cag aca ctc atg agg     144
   Gly Phe Asn His Val Gln Arg Thr Gln Phe Leu Gln Thr Leu Met Arg
           35                  40                  45
   tat ggt ttt cag aac tat gat tgg aaa gag tat ctt cct cgt ttg aag     192
   Tyr Gly Phe Gln Asn Tyr Asp Trp Lys Glu Tyr Leu Pro Arg Leu Lys
       50                  55                  60
   ggg aaa agt gtt gag gaa att cag aga tat ggt gag ctt gtc atg gcc     240
   Gly Lys Ser Val Glu Glu Ile Gln Arg Tyr Gly Glu Leu Val Met Ala
   65                  70                  75                  80
   cat ctt gta gag gac aca aat gac tca cca acc tat gca gat ggt gtg     288
   His Leu Val Glu Asp Thr Asn Asp Ser Pro Thr Tyr Ala Asp Gly Val
                   85                  90                  95
   ccg aag aaa tgc gtg ctg atg aga cat tgg tca ggc tag cca aaa tat     336
   Pro Lys Lys Cys Val Leu Met Arg His Trp Ser Gly  *  Pro Lys Tyr
               100                 105                 110
   cac ttg tgg agg aga agg tgg tgc atg gag caa gga aaa tta caa aac     384
   His Leu Trp Arg Arg Arg Trp Cys Met Glu Gln Gly Lys Leu Gln Asn
               115                 120                 125
   tct tcc cca act act tga tgt atg aat tta ctg gct tat cag gtg gaa     432
   Ser Ser Pro Thr Thr  *  Cys Met Asn Leu Leu Ala Tyr Gln Val Glu
           130                 135                 140
   gaa tat gga aag ggg aac atg atc tac tgt nac tga agc ata ata agc     480
   Glu Tyr Gly Lys Gly Asn Met Ile Tyr Cys Xaa  *  Ser Ile Ile Ser
           145                 150                 155
   acg ggt tgc cag tgg cat aca tat cag atn cag aga tac ggg             522
   Thr Gly Cys Gln Trp His Thr Tyr Gln Xaa Gln Arg Tyr Gly
           160                 165                 170
<210> 30
   <211> 171
   <212> PRT
   <213> Oryza sativa
<220>
   <221> VARIANT
   <222> (1)...(171)
   <223> Xaa = Any Amino Acid
<400> 30
   Val Ser Gly Arg Lys Ala Gln Tyr Ser Lys Lys Asn Ser Arg Asn Val
    1               5                  10                  15
   Asp Ser Leu Pro Leu Met Glu Gly Glu Gly Arg Ala Leu Lys Val Tyr
               20                  25                  30
   Gly Phe Asn His Val Gln Arg Thr Gln Phe Leu Gln Thr Leu Met Arg
           35                  40                  45
   Tyr Gly Phe Gln Asn Tyr Asp Trp Lys Glu Tyr Leu Pro Arg Leu Lys
       50                  55                  60
   Gly Lys Ser Val Glu Glu Ile Gln Arg Tyr Gly Glu Leu Val Met Ala
   65                  70                  75                  80
   His Leu Val Glu Asp Thr Asn Asp Ser Pro Thr Tyr Ala Asp Gly Val
                   85                  90                  95
   Pro Lys Lys Cys Val Leu Met Arg His Trp Ser Gly Pro Lys Tyr His
               100                 105                 110
   Leu Trp Arg Arg Arg Trp Cys Met Glu Gln Gly Lys Leu Gln Asn Ser
           115                 120                 125
   Ser Pro Thr Thr Cys Met Asn Leu Leu Ala Tyr Gln Val Glu Glu Tyr
       130                 135                 140
   Gly Lys Gly Asn Met Ile Tyr Cys Xaa Ser Ile Ile Ser Thr Gly Cys
   145                 150                 155                 160
   Gln Trp His Thr Tyr Gln Xaa Gln Arg Tyr Gly
                   165                 170
<210> 31
   <211> 23
   <212> DNA
   <213> Artificial Sequence
<220>
   <221> primer_bind
   <222> (1)....(23)
<400> 31
   gtttctggga ggaaggctca gta 23
<210> 32
   <211> 23
   <212> DNA
   <213> Artificial Sequence
<400> 32
   tatgtatgcc actggcaacc cgt 23
<210> 33
   <211> 510
   <212> DNA
   <213> oryza sativa
<220>
   <221> CDS
   <222> (2)...(510)
<400> 33
   c tta cag gat ttc ggg gga ggt ggc tgc ggc tgt ttg gag cgg agg ggt       49
     Leu Gln Asp Phe Gly Gly Gly Gly Cys Gly Cys Leu Glu Arg Arg Gly
      1               5                  10                  15
   tta ata gct aca gca tgt gac gtt gat act cta atg atg aag gag cgg        97
   Leu Ile Ala Thr Ala Cys Asp Val Asp Thr Leu Met Met Lys Glu Arg
               20                  25                  30
   agc tct tta tgt gaa agt gcg gca gat gga agt tgg gtt ttg aaa tac       145
   Ser Ser Leu Cys Glu Ser Ala Ala Asp Gly Ser Trp Val Leu Lys Tyr
           35                  40                  45
   aaa agg aaa cgg agc aag cta aca gtt agt cca tca agt gag cat gat       193
   Lys Arg Lys Arg Ser Lys Leu Thr Val Ser Pro Ser Ser Glu His Asp
       50                  55                  60
   gct tcc tca cca ata ctg gat tct caa atg aac aat ggc tcc atc aaa       241
   Ala Ser Ser Pro Ile Leu Asp Ser Gln Met Asn Asn Gly Ser Ile Lys
   65                  70                  75                  80
   aag aag atc aaa cat gac act aac att tct cca tca acc aag aag ata       289
   Lys Lys Ile Lys His Asp Thr Asn Ile Ser Pro Ser Thr Lys Lys Ile
                   85                  90                  95
   aga gga cat gac ggg tac ttc tac gag tgt gta gaa tgt gat ctc ggt       337
   Arg Gly His Asp Gly Tyr Phe Tyr Glu Cys Val Glu Cys Asp Leu Gly
               100                 105                 110
   ggc aat ttg ctg tgc tgt gat agc tgt cca cga aca tac cac ttg gaa       385
   Gly Asn Leu Leu Cys Cys Asp Ser Cys Pro Arg Thr Tyr His Leu Glu
           115                 120                 125
   tgt ctt aat cct cct ctc aag cgt gca cca cct gga aat tgg caa tgc       433
   Cys Leu Asn Pro Pro Leu Lys Arg Ala Pro Pro Gly Asn Trp Gln Cys
       130                 135                 140
   cca aga tgt cgt aca aaa aaa gtt agc ttg aag ctc tta aac aat gct       481
   Pro Arg Cys Arg Thr Lys Lys Val Ser Leu Lys Leu Leu Asn Asn Ala
   145                 150                 155                 160
   gat gct gac acc tcc taa acg tga aag aa                                510
   Asp Ala Asp Thr Ser  *  Thr  *  Lys
                   165
<210> 34
   <211> 167
   <212> PRT
   <213> oryza sativa
<400> 34
   Leu Gln Asp Phe Gly Gly Gly Gly Cys Gly Cys Leu Glu Arg Arg Gly
    1               5                  10                  15
   Leu Ile Ala Thr Ala Cys Asp Val Asp Thr Leu Met Met Lys Glu Arg
                   20                      25                      30
   Ser Ser Leu Cys Glu Ser Ala Ala Asp Gly Ser Trp Val Leu Lys Tyr
               35                      40                      45
   Lys Arg Lys Arg Ser Lys Leu Thr Val Ser Pro Ser Ser Glu His Asp
           50                      55                      60
   Ala Ser Ser Pro Ile Leu Asp Ser Gln Met Asn Asn Gly Ser Ile Lys
   65                      70                      75                      80
   Lys Lys Ile Lys His Asp Thr Asn Ile Ser Pro Ser Thr Lys Lys Ile
                       85                      90                      95
   Arg Gly His Asp Gly Tyr Phe Tyr Glu Cys Val Glu Cys Asp Leu Gly
                   100                     105                     110
   Gly Asn Leu Leu Cys Cys Asp Ser Cys Pro Arg Thr Tyr His Leu Glu
               115                     120                     125
   Cys Leu Asn Pro Pro Leu Lys Arg Ala Pro Pro Gly Asn Trp Gln Cys
           130                     135                     140
   Pro Arg Cys Arg Thr Lys Lys Val Ser Leu Lys Leu Leu Asn Asn Ala
   145                     150                     155                     160
   Asp Ala Asp Thr Ser Thr Lys
                       165
<210> 35
   <211> 23
   <212> DNA
   <213> Artificial Sequence
<220>
   <221> primer_bind
   <222> (1) ... (23)
<400> 35
   cttacaggat ttcgggggag gtg 23
<210> 36
   <211> 23
   <212> DNA
   <213> Artificial Sequence
<220>
   <221> primer bind
   <222> (1) ... (23)
<400> 36
   ctttcacgtt taggaggtgt cag 23
<210> 37
   <211> 667
   <212> DNA
   <213> triticum aestivum
<220>
   <221> CDS
   <222> (2) ... (667)
   <221> misc feature
   <222> (1) ... (667)
   <223> n = A,T,C or G
<400> 37
   g ttg act gga acc cca tta cag aac aac att ggt gaa atg tat aat ttg      49
     Leu Thr Gly Thr Pro Leu Gln Asn Asn Ile Gly Glu Met Tyr Asn Leu
     1               5                  10                  15
   ttg aac ttc cta cag cct gct tct ttc cct tct cta gca tca ttt gag        97
   Leu Asn Phe Leu Gln Pro Ala Ser Phe Pro Ser Leu Ala Ser Phe Glu
               20                  25                  30
   gag aag ttt aat gaa ctt gca aca gca gag aaa gtg gag gag ctg aag       145
   Glu Lys Phe Asn Glu Leu Ala Thr Ala Glu Lys Val Glu Glu Leu Lys
           35                  40                  45
   aaa ctg gta gca cca cat atg ctt cga agg ctg aaa aaa gat gca atg       193
   Lys Leu Val Ala Pro His Met Leu Arg Arg Leu Lys Lys Asp Ala Met
       50                  55                  60
   aaa aat atc ccc ccg aag aca gag cga atg gtg cct gtc gaa ctg aca       241
   Lys Asn Ile Pro Pro Lys Thr Glu Arg Met Val Pro Val Glu Leu Thr
   65                  70                  75                  80
   tca atc cag gct gaa tac tac cgt gct atg ctt aca aag aac tac caa       289
   Ser Ile Gln Ala Glu Tyr Tyr Arg Ala Met Leu Thr Lys Asn Tyr Gln
                   85                  90                  95
   gta ctg cgt aat acc gga aaa ggt ggt gct cat cag tca ttg ctc aat       337
   Val Leu Arg Asn Thr Gly Lys Gly Gly Ala His Gln Ser Leu Leu Asn
               100                 105                 110
   ata gta atg cag ctt cgg aaa ttt gca acc atc cat atc tta tcc tgg       385
   Ile Val Met Gln Leu Arg Lys Phe Ala Thr Ile His Ile Leu Ser Trp
           115                 120                 125
   gaa ctg aac ccg aat caa gtt cac cag att ttt gca tga aat gag aat       433
   Glu Leu Asn Pro Asn Gln Val His Gln Ile Phe Ala  *  Asn Glu Asn
       130                 135                 140
   aaa ggc tca aca aat taa ctt tgt tgc att cta tgc tca aag tgt tac       481
   Lys Gly Ser Thr Asn  *  Leu Cys Cys Ile Leu Cys Ser Lys Cys Tyr
       145                 150                 155
   aca gtg atg ggc atc gtg ttc taa ttt tcc aga tga cta aac tct tga       529
   Thr Val Met Gly Ile Val Phe  *  Phe Ser Arg  *  Leu Asn Ser  *
       160                 165                         170
   cat ccc gaa gat anc gac ccg gaa ttg gca taa aca ntn aaa gag naa       577
   His Pro Glu Asp Xaa Asp Pro Glu Leu Ala  *  Thr Xaa Lys Glu Xaa
               175                 180                         185
   tgg tcg tgt cgt ggg tga cnc aag cac ata nct tca aca gaa ana cgt       625
   Trp Ser Cys Arg Gly  *  Xaa Lys His Ile Xaa Ser Thr Glu Xaa Arg
               190                 195                 200
   ttg att tgt aca acg gca tgc ntg tat tga cna nac gta can               667
   Leu Ile Cys Thr Thr Ala Cys Xaa Tyr  *  Xaa Xaa Val Xaa
               205                 210
<210> 38
   <211> 214
   <212> PRT
   <213> triticum aestivum
<220>
   <221> VARIANT
   <222> (1)...(214)
   <223> Xaa = Any Amino Acid
<400> 38
   Leu Thr Gly Thr Pro Leu Gln Asn Asn Ile Gly Glu Met Tyr Asn Leu
    1               5                  10                  15
   Leu Asn Phe Leu Gln Pro Ala Ser Phe Pro Ser Leu Ala Ser Phe Glu
               20                  25                  30
   Glu Lys Phe Asn Glu Leu Ala Thr Ala Glu Lys Val Glu Glu Leu Lys
           35                  40                  45
   Lys Leu Val Ala Pro His Met Leu Arg Arg Leu Lys Lys Asp Ala Met
       50                  55                  60
   Lys Asn Ile Pro Pro Lys Thr Glu Arg Met Val Pro Val Glu Leu Thr
   65                  70                  75                      80
   Ser Ile Gln Ala Glu Tyr Tyr Arg Ala Met Leu Thr Lys Asn Tyr Gln
                   85                  90                  95
   Val Leu Arg Asn Thr Gly Lys Gly Gly Ala His Gln Ser Leu Leu Asn
               100                 105                 110
   Ile Val Met Gln Leu Arg Lys Phe Ala Thr Ile His Ile Leu Ser Trp
           115                 120                 125
   Glu Leu Asn Pro Asn Gln Val His Gln Ile Phe Ala Asn Glu Asn Lys
       130                 135                 140
   Gly Ser Thr Asn Leu Cys Cys Ile Leu Cys Ser Lys Cys Tyr Thr Val
   145                 150                 155                     160
   Met Gly Ile Val Phe Phe Ser Arg Leu Asn Ser His Pro Glu Asp Xaa
                   165                 170                 175
   Asp Pro Glu Leu Ala Thr Xaa Lys Glu Xaa Trp Ser Cys Arg Gly Xaa
               180                 185                 190
   Lys His Ile Xaa Ser Thr Glu Xaa Arg Leu Ile Cys Thr Thr Ala Cys
           195                 200                 205
   Xaa Tyr Xaa Xaa Val Xaa
       210
<210> 39
   <211> 23
   <212> DNA
   <213> Artificial Sequence
<220>
   <221> primer_bind
   <222> (1)...(23)
<400> 39
   gttgactgga accccattac aga 23
<210> 40
   <211> 23
   <212> DNA
   <213> Artificial Sequence
<220>
   <221> primer_bind
   <222> (1) ... (23)
<400> 40
   catgccgttg tacaaatcaa acg 23
<210> 41
   <211> 12561
   <212> DNA
   <213> Zea mays
<220>
   <221> misc_feature
   <222> (1) ... (12561)
   <223> Zmpk1 genomic sequence
   <221> misc_feature
   <222> (1)... (12561)
   <223> n = A,T,C or G
<400> 41
   atactgtaat catctatgac aggtgaaaaa tctctctgtt tagacaaaac agttaacata 60
   atggattcgc ttcaatttct cacctatgta tgtacagtga ttggaaccca catgcggatt 120
   tgcaagctat ggcaagagct catcgcttag gacagactag taaggtattt taccttacac 180
   tttatattgt ataaaaaaac agattttcaa taagttttgt ggtgatttta taattttcat 240
   ctgtttttct tttaggtgat gatatacagg cttgttagcc gaggtacaat tgaggagcga 300
   atgatgcagc ttacaaaaaa gaagatttta ttggagcact tagttgttgg tcgactcacc 360
   aaagctaata atgtcaatca ggtatgttga ctacttttta atggtgaatt ttgtaaacca 420
   tcaacttagg ttgatctttt atggcctaag ctatttatga attcatttat ggattgaggg 480
   ttgagtagtt acatgttact ccctccattt tttatatttg tggtgtttta gttcaaaaat 540
   aaactaacgg gtgacaaata ttcgagaacg gaggtagtac tagtaccttc tgtctgggat 600
   gacatgaaat gaatgtagca tctgttagta tcatgtccat ttctttgtgt tacattttac 660
   aaggcttaaa accttacaca tattgccgga gttggtgact atttagtctt atctgtaaat 720
   ttagttgttt ctcttgatgt caatagcaat ttatggttgt atgagatttc gtgggtttgt 780
   tagcatgtgt gccatatagg tttagctccg ctgatgtgtt atgcacttat aattcagacc 840
   cattttggag ctgtgatgtg atacacaatg ctagttgtta aggccccatt tgtttgtttc 900
   atattcataa tctatgtgca tgcattagtc cagatcaggc tgggtgatct ggtgggcatt 960
   ggtacatgca tggctgaata agagtttgat gaccaagggt aaggcacccc acatctactc 1020
   ttggctggcc aactgggtag tatctgctag ttatcatgga aataaggttg gcaacctctt 1080
   taaggttgtc ttcaagaact aaacataaaa agaatgccat gaagatggaa cccaggatct 1140
   caggacctat ttgtcgtaag atttctgaat ctttgctgga gctggcaacg ggctggttgc 1200
   tggtctcatc cctacattat agttttgtgt atgtttcttc cagactttca acacttctca 1260
   acttctagaa catgtaaccg gcatgtacaa acagaacaat ttagaacagt tcaaagtgca 1320
   tgtccttact gcgatgggac cacacacttc tggtctcttt aggacttgat tgatttaatg 1380
   cagattaata attaacccaa tttgctcttc tgacctgcta gtgagccctt cattggtttt 1440
   taagtactaa ataataaata tgtgtctttg cctattttag gaggagttgg atgatattat 1500
   acgctatgga tcaaaggagc tttttgaaga cgagaatgat gaatctcgcc aaattcatta 1560
   tgacgaagct gcaattgaga ggtaaacacc taggcccttt tttgatctcc taagataaga 1620
   ttaatgagaa cgacgtagaa aaatgagtgt gatccaagca cagattctag aatccaacta 1680
   tctagctaaa ccttgctata catagattct cgctatgcaa aggccataat cactatgaaa 1740
   atgagatcca aacacccctt gtttttattt atctagaatg tagattctca aaagaagggc 1800
   aagcttggtg cattggtgag atctttctca ttcagtcatc aagtcgtggg ttcaaagcag 1860
   cctctcgaca tttgtgggag cctctagcac tgggtctacc cctttttatt aacaatctag 1920
   attctacatc atcatgtaga atcactatga taatgagatc taaacatggc cttaagcccc 1980
   taacttcacc ttgggaagtt atttcgtgca ttaagcatgc ctttttggtg tgaagttgtt 2040
   taaagtatta ccagatttat gtaacaattt acaaatgtat tggaatctgg agaccatttt 2100
   gatcatggat gcaaatctag aattctagag ataattagta tgtatgccct accaagtagg 2160
   atacgatgag agattgagag tgctaagaga cttttatggg aagacttaga tggtatgatt 2220
   agagttatgc ctattagtga gaagcttttc ataggagatc tcaacggaca tgtaggtaca 2280
   acaagtgtag gtttcaaggc ggttcatgga gggtttgagg aagagaatcc cattttgtga 2340
   catgtagtag tggcaaacgc tctagtcaga tttgttaagt aactgtgtat tccttgtaat 2400
   gggatcttgc gtaatgagca taggccctat aactctatta taaatacagc accgaaccct 2460
   gatgttaagc taggtttagc ctcctctctc ccacctagcc aatatggtat caagctaggt 2520
   ttagcctcct ctctcccacc cagccgtcgc cgccaccgct acagtagttg ccgctgtcgg 2580
   cattcttcct cccctccccc ttccctggtg tcggcgaccc tccttcgacc tccttccatc 2640
   ggcgcccctt tcccctcgtc cagccgtcgc cgcagtgtct ggacgcgagg cccttcctca 2700
   tccgtgcgct tgggggccgc tgggcttttc ctccccccgc ccgccagcag tcggccttgt 2760
   cctcgccacc gtcggagttg ccacatgaga ggcattgaaa gggtgaaagg aggtaatgcg 2820
   gtgggccctg atgttatccc aatcgaggca tggagatgtc ttgggggaca tagctataat 2880
   aggctaacta agttgttcag tctatcttcc ggtcaaacaa gatgcttgac aagtggagaa 2940
   gtatattggt accaatcgac aaaaataagg aagatattca aagttgtagc aattaatagg 3000
   gaataaagtt gatgagccat actacgaagc tatgggagag agttatcgag catcgagagg 3060
   aaaaacgagg acctgtatga accaatttgg tttcatgcaa gctcaaccat ggaagccatt 3120
   ttcttaataa gataagtact ttgtgactaa cataaaattt tatgtggtag gctttggaca 3180
   aacataaaat tccaacgaag tactttgtga ctaattaagg acatgtacaa taatggtgtg 3240
   actagtcact agtgtttgaa caagtcatgt gaacacaaat gacttactga ttagagtagg 3300
   gctacatcag gggtcaacct tgagctctta cctttttgcc ttggtgatgg atgaggtcac 3360
   aaaggacata caaatggata tcccttggtt tatgccttgc ggacgatata gcgttatttg 3420
   atgaaagtcg gatagagtta tttgatggaa gtcggatagg agtaaatagg aaactatagc 3480
   tgtggcggga gactctagag tccaaaggtt ttagactcaa tagaactaaa actgaataca 3540
   tgagatgtgt cttcggcact actacacata aggaaaacga tgttagtttg aaaggtcaag 3600
   tagtgcctac gaaacgataa tatacatgat agattagggg tgacaccaat tgaagaaaag 3660
   cttttccaac accgattgat atggtttgaa catgtctaac agagacctct agagacacca 3720
   atgtgtagtg gaattctaag tcatgatagt aatgagaaga gacaagagag gcagaggaag 3780
   gccaaagttg acatggaaat gggtagtaga agagatttca aaggatgaaa tataccaaag 3840
   atttagcctt aaataggagc gaatgggaaa caactcatcc atgtgcctga atcttgattt 3900
   gtggctttat taggtttcaa ctctagccta gcccaacttg attgggacta ataggctttg 3960
   ttgttgtttt tgcaaacctg gaaattttca tgaccatggc aataaatagc acagtttata 4020
   ctcaatatca ccctatacgt aagaacaaca tgagctgcat aattgatttt gtttgtttaa 4080
   ttccattttc ttatgaaatt ccttcttttc tcctattaca gtaattcata gtatggaatc 4140
   tgtcttcatg cagattatgc acaacactaa tccttgttgt tatggcccca atttttatct 4200
   tctttgttat gacttaaaac tgtagtgctc tatgttagag gagacaacgt aatatatctg 4260
   actggactca caggttgtta gaccgtgatc aagttgacgg tgatgaatct gtggaagatg 4320
   aagaagaaga tggattctta aaaggattca aggtattggg gtcttctttc aattattaca 4380
   agcataatgc ttgaggagct tcttcatttt aattatcctc ttgatattta ctgtggttta 4440
   cattgtttag tctttttctt gtttattatg tgcacatgta tttgttaaag tgcacataat 4500
   ctctatttgc acaagtacac ttgtgaggca gtgaggtctg actctgttat ttgtatgttt 4560
   gcgagtatgc atgtaatcaa gccactattg atatttgata ggattaacct atgcatcaga 4620
   ttttgttgat gaatgagata ttttatgtat gcattgcatg ctttctcatt tgaattatct 4680
   cgttagtttt acctgcccat ctaagatata caattgcgta gtgctgagta aaacactatg 4740
   caaataaaca atgtttcttg ttctctcatt catctgtagg cctattcttc caattcaaac 4800
   atgctatgtt actcataggg caatactatt tgtgtttcat ttattgttga aaatgctgca 4860
   tactacaact gcctcatgac tcatttttca ttctaagtgt ttggcacatt ctagcaaaca 4920
   aaagagtccc tttatttaac ccaaagcaaa acgattctcg cttcttgtat tactctatgc 4980
   taggttgcaa actttgaata tatcgatgag gcaaaggctc aggcagaaaa agaggaggca 5040
   cggagaaagg ctgcagctga ggctgaaaat tctgaaagaa actactggga tgaactattg 5100
   aaggatagat atgatgtaca gaaagttgaa gaacatactg ctatgggaaa agggaaaaga 5160
   agccgcaaac aggtttaatt tctaaccatt tccattgtta accttgtgac ttgtgcccct 5220
   tttcatatca ttttcccttg ttttgtgatc tgcattcatt gctttgcggt tggcaataga 5280
   tgtaatttca tatttgttcc ttcccaaaaa gaaaatcata gttgttgcct tgttggcaac 5340
   ttacagtatt tcacttgtag aatatctttt aaactcgatt taagcactgt aattgtaatt 5400
   aaattttagg aaatcatgct tatacaccaa ctaggataac tgatgcaacc aatcactgtt 5460
   gagcgttgac aatatgcatc aaagtcatat aactaatctt atcatgaaga taagcatgaa 5520
   attaaggaag aacgtagcca ttatttgtta tatctttctt ggtttaacgg cgcagtgcca 5580
   ttagtgcatt acttcttagc cagacatgac atctgctgct cagtgtagtg taatcttctt 5640
   ttgcgaaggt acttctcttt aaactagttt tgcatgacga tgaagttgag tatagacatt 5700
   ttgtgttaga tccttcttgc tgttactgta cagtatatat attagttcat tattcgtccc 5760
   accccacccc aaaccaatca ttgactatag cttaatgtac tatgtctgtt tggtaatgta 5820
   ttgtattgtt tttatttggt cgatttaaat tttagagtga ccatatacat tttcagtaag 5880
   agaaaagact gatgaacctt gtttcttttc tgcgtcatgt gcagatggct gccgctgatg 5940
   aagatgacat tcatgattta agttccgaag atgaggatta ctcattggag gatgacattt 6000
   cagataatga cacaagtttg caaggaaata tttctgggaa gaggggccaa tattctaaga 6060
   gaaaatcacg taagagagca atgtaaatac atcgcactat ggactattgt tacatgatga 6120
   atattctgta cttatatact ttgcaaatac attgtcatta ggtaatgttg attctattcc 6180
   attgatggag ggcgaaggac gtaccttgag agttcttgga ttcaaccatg ctcaacgagc 6240
   aatgttccta cagacactca ataggttagt tactgatatg cctcttgaac ctgtctggtc 6300
   agcgagtgag taccttgaac ctaaagttta tgtgcagatg ttgagatgct attacattgc 6360
   gtgataaagc aggcacacag aaatttctgt ttcatttgta tctcttggcc atgtgtacat 6420
   ttttaatcat ggaatccttt tttttttaca attgttggtt agtgtttaca tttttatcac 6480
   tcgtttctaa tagttgtgtg ctctgacctg tatctttgtc accaaattgt aatttctggt 6540
   ccaattttaa gcataaaata tttgcactga ggtcattggc tattccctag ttgtactatc 6600
   agaccaacat gaacgtgacc ccatgcaaca ttgtagattc ggttttcaga attatgactg 6660
   gaaagagtat cttcctcgtc ttaaaggaaa aagtgtcgag gaaatccaga ggtatgtgaa 6720
   atgtgctctc catgttttat gaccccaatt attttgagtt gtagaccaaa tagtgaagaa 6780
   tctggggcat agtttcgtaa gttagatgga tatgatgcac aacattttac attttattga 6840
   ttttctctta ggcgaagggc gggcctggtg cagcggtaga gcctaccgtc tgtaaccgga 6900
   aggtcttggg ttcgagcccc aacctctgca tattatgcgg gtaaggcttg gcgcttaaag 6960
   atacccttcc ccagaccccg cacagtgcga gaagcctatg gcactgggtt cgccctttta 7020
   ttgattttct cttaggcccc gtttgtttcc cttcatttta aggaattgga atctaactga 7080
   tggagtaagc tatttttttt ataatgtaat attccataac tttccaaagt ttatgtataa 7140
   gccaatctca aattcatggg gtgagagatg gaaattgatt ctatagattt acatgctact 7200
   tttcaaattt acaacttata gcacactctt ctacttgctt ctctatatga taaatgtagt 7260
   gtataactat ctctcttata tgatttagga taatatacaa atacattaca tagataaata 7320
   tattaactta atagttttat cttaaattat aattattata atggaattca attccaacga 7380
   aacaaacggg gccttaggta tatagacaat gaattcaagt ttgtgcttcg acgaagaatt 7440
   gatgagatgg gtaactgggc attcctggct tctgatagta cacatattta ggcaaagtcg 7500
   actgctggta gtagtgaaag tgtacacaca aatatgcttg gttggactct tgctttgttt 7560
   attatgttgc aaatttatat aactattgca cccttgccaa caaggaaaga tgtggcatca 7620
   tcacatatga agccaaatta agcaggcagt caccaaagtc agactatatc tgcagtgcta 7680
   gactgttagt agcaaattga gccaacagtc accaaagcta gactacctct gaagtgcgaa 7740
   tagcaggcgg ccataaccta accctattaa gttgtatgca ccaaccagtt caacccaaaa 7800
   gcttaagctg atggagagag gtggcaattc acttgtattc taacattctc cctcacatcg 7860
   aggctctctt agaccgtctc cagcagttca cccatacggt tatcaaaaca ctggttttca 7920
   ctgtagacta tactgtttgc atatggggat gtggatgagt aagctgctgg agatagcctt 7980
   aggtcttaga cgtggaataa gaacggacaa caattatttt atttaattgc gctaaccagg 8040
   attcgaactc aagatatctg gctttgatat catattaagt cgcatatacc agccagttca 8100
   acccaaaaac ttaagcttat agagaggtgg acaattcact tgtattctaa caaaccccag 8160
   tgctagtagc aaaatgagcc aacagttacc aaagcttgca gctgaagggc tagtttggca 8220
   gcgctttgtg gacagagcgc tgcgccgctg ccaaacactg ttgctccatc tcggaagtgc 8280
   tcgccctatg ctccagtcga tttgcattat gggtggggag cggaaaaaat ccgctctgtc 8340
   tgcagcatgc tcctctctcc acttcccgct ctgctgcctg ctcccctctc cacccacgcc 8400
   gctatccacc gcatctctgc ctgcacagcc cctctctggc tcattaacgg tggcgtggac 8460
   cacgagcact gggcagatcc gtggtggtga taaagagggc aggagggagg ccagaccctc 8520
   cgtggcgagg agtgctgcac ttggaggatg tgggccctgg cgagctggag ccgaagctga 8580
   tggagaagca ctaccaaaca ctggtatcgg caggtgggga gcaacctgag cgggagccta 8640
   ggggagctgg agccgctggg agcttcgtga cagtagtacc aaacatgatc taagtaaacg 8700
   tcatatgaac atggaacagg catcaagatg ctgtccttca tatagctcta tcattacttt 8760
   ttccttgtag agcccataac cacaagaatg ttgtacaccc acttatgtat agggtaggct 8820
   tgtataacac ccatttaagt caagtacacc tcttatattc ccaccaaaca acccagtttt 8880
   aggcaattat ttttgtggag atcctcaaag tgattcatag caataacctt tgtgcaattt 8940
   attttcgtat atataatatg ttcttgcaga tatgctgaac ttgtcatggc acatcttgtt 9000
   gaagaaatta atgattctga ctatttttca ggtaattgag cttagtaatg gctactatca 9060
   tttttacgca tccaattctt atcctccata tgcatgaatg cagatggcgt tccaaaggaa 9120
   atgatgcgtg ttgatgatgt actagtcagg atagcaaaca tatcccttat tgaggagaag 9180
   gtgcatgtgg ctttcatttg ttatttgcat cttaacatga cttagaactc aaaagaaact 9240
   tatgagcatc ctgatgatgt tcaatacaaa catgattgtg ctgtattctt tcttgcgtaa 9300
   agtgcaagga ttgtcgagag ctgaagttta gtatttaaac ttgcacctta tagaattcgg 9360
   ttgattacaa taccttgata tgattctgct atcaatgagg caacctttgt gtactgtatt 9420
   ctttcttact tttgtattgt ggttccagat ggctgccaca ggaccaggaa aaattacaaa 9480
   catttttcct aattacttgc tctatgagtt ccaaggctta tctggtggaa gaatatggaa 9540
   agcggagcat gatctactgt tactgagagg catactgaag tacgaaacta cgaatatttt 9600
   ctttgttaca atcagctcta caaaattacc cctccatgaa actagcagtg gctggaacag 9660
   ttctggcaag agtagcctta aaaagtcgat gtttgtgtca gcagcagtgg cgttgcaatg 9720
   acagaactga attgtttggc tcacttgttg gttgatggat tattagtttt ttagtatagc 9780
   agcagtggct tgcatcttgg cccgagtgga cctattgtgc tcagaagtta gggttaccct 9840
   aacgggtatc ttttcgcata gcttgatagc ttccaggtgg tgcctgtcca cccagtgctc 9900
   tagtgatttt ggagacatcg ggttcaggaa ctgtttaatt aaggcaacaa attcaactta 9960
   tcttaacgtt gagtaagcaa actgaagtat cacatgcaaa ccagatgagc tccacatttg 10020
   atttgatatc taacttaaat tatttatttt gattctgttt aatactttta caattgtaat 10080
   catgtggaac agcttaatga aaaatgtagg ttttcaaggg gtacacttga cctgcacagc 10140
   gatctccttt gcttttatag actttgtgtt ttttccttgt ggataatgca ttttttacca 10200
   actattgttt cacactgtaa caaaactata tttaaggtat taacacaatg catttgtttt 10260
   gccttcacca ggcatggata tgcaaggtgg cagtatatat cagatgacag agagaatggg 10320
   ctttttgagg ctgcacgacg agagcttcat ctcccttcgg ttaatgaaat aattggtgct 10380
   cagttgaacg aggcaaatgt tagcatgtgc tcactatgtc ccttcctcca aattttgaag 10440
   tgttgcattt cttatttctt ccattccttt tctaggggaa tttggaaggt gcacaggaag 10500
   gccaagcgaa cacaacaagc atgtcgcatt acaaggagac ccagagaaag atagttgagt 10560
   tcttgagaaa gagatatcat cttatggaga gagccttgaa tatggaatat gctgtggtac 10620
   ggtactaggc ttttttccct gaacaggcat gcactcaaag ctcacatggg agtgcacagt 10680
   agctgttcat gtagtatctt gcagcatttt ttaaagcgta cctttttctc ttgcagataa 10740
   agaaaaaaat tcctgttcct gatgatatta ctgaacaagg tgttccagca ggacatgctc 10800
   cttttattcc agatatcagt gaactgttgc gggaattgcc caatcttgag ccaatttgta 10860
   agtgatctct tattccctat atatatattc cttctctcca cctatgcagg aagagacagt 10920
   cacatagtgg catagaacat gctggggcag atgctcacaa tttttaatca tgtgtcatgc 10980
   atgactcttg tcttactaaa ctctctctaa agatgtgttg ttgttgacat atactcttga 11040
   gtcttgaggt ttgttcattt gtttgttttg ataactggga tcctcgtttc cagctaccaa 11100
   tgaattggtt tctgagggca cagctggtca gttacaagtt ccccatctct acaataaggt 11160
   gtgtaagcgc aatggcacac gttttcatgg gtcatgggcg aacgcatcat ttgttttcct 11220
   cgagttaaca ttataaccag actgtgtacg tatgctctgc agatgtgtgg agtgcttgaa 11280
   gagagtggtg cttatgcgct cagttccttc tttggagaca agtccgcatc ttctagtttg 11340
   gccaatagcc ttcgacagtt tgaaactgtg tgcgggaatg tcgtcgaggc cttgcgacca 11400
   caccaaaatg gtactggcag tgccatcaaa gaggaattgg tagatgcagc caccaaagca 11460
   gcagcagcag cagctcctca acaagattca ggccatgatg caccgcatgg gcagtcttcg 11520
   acagccaagg cggacatgga aatcgatggt tgatttgtag gttccagagt ggtaagaaag 11580
   ggaatccccc tctaatcatt atgtatactg tggtcagaat gtgcgctata tattgtaaca 11640
   tcaaagaaag cacctccagg cctgagggtg ttactgctaa tgcgtttggt ttacttgttg 11700
   tccttgtaat atgcatacac atttagaact catgcagcca ttttgtgtgc tcgaatncgg 11760
   tggatcgctg ccctgttgtc ttgtactgtg tttaagggcc tgtttggnat acgaatggtt 11820
   aattataaga cggggctaaa gataagtacg gattaactna tagttggcta gcttgttggg 11880
   taagaaatta caaaatagtt tgcaaaacaa aacatgttgg tgcaagcgtg gtcccaaaat 11940
   gttaaaaacg aagaaacgat ccatgcatat cttgtaagta tttacattgg ctcaattcca 12000
   agcaaccttt gcacttacat tatacaaact agttcaatta tgcatttcta tacttgcttt 12060
   ggtttgtgtt ggcatcaatc accaaaaagg gggagattga aagggaatta ggcttacacc 12120
   tatagtccct aattaatttt ggtggttgaa ttgcccaaca caaataattg gactaactaa 12180
   tttgcccaag tgtatagaat atacaggtgt aaaaggttca cactcagcca ataaaaagat 12240
   caagttttgg attcaacaaa ggagcaaaga gacaaccgaa ggcacctctg gtctgggggc 12300
   accggactgt ccggtgcacc agaggactca aactcaaact tgccaccttc gggaattttc   12360
   aaaggcactc cgctataatt caccggactg tccggtgtac accggacagt gtccggtgct   12420
   ccaaggaaga gcggcctctg gaactcgcca gcctcgggaa aacgcagcgg ctgctccgct   12480
   ataattcacc ggactgtccg gtgtacaccg gactgtccgg tgaaccagca gagcaatggc   12540
   tacttcacgc caacggtcac c                                            12561

## Description

### TECHNICAL FIELD

The present invention relates generally to plant molecular biology. More specifically, it relates to nucleic acids and methods for modulating their expression in plants.

### BACKGROUND OF THE INVENTION

Major advances in plant transformation have occurred over the last few years. However, in major crop plants, such as maize and soybeans, serious genotype limitations still exist. Transformation of agronomically important maize inbred lines continues to be both difficult and time consuming. Traditionally, the only way to elicit a culture response has been by optimizing medium components and/or explant material and source. This has led to success in some genotypes, but most elite hybrids fail to produce a favorable culture response. While, transformation of model genotypes is efficient, the process of introgressing transgenes into production inbreds is laborious, expensive and time consuming. It would save considerable time and money if genes could be introduced into and evaluated directly in production inbreds or commercial hybrids.

Current methods for genetic engineering in maize require a specific cell type as the recipient of foreign DNA. These cells are found in relatively undifferentiated, rapidly growing callus cells or on the scutellar surface of the immature embryo (which gives rise to callus). Irrespective of the delivery method currently used, DNA is introduced into literally thousands of cells, yet transformants are recovered at frequencies of 10⁻⁵ relative to transiently-expressing cells. Exacerbating this problem, the trauma that accompanies DNA introduction directs recipient cells into cell cycle arrest and accumulating evidence suggests that many of these cells are directed into apoptosis or programmed cell death. (Reference Bowen et al, Third International Congress of the International Society for Plant Molecular Biology, 1991, Abstract 1093*).* Therefore it would be desirable to provide improved methods capable of increasing transformation efficiency in a number of cell types.

Typically a selectable marker is used to recover transformed cells. Traditional selection schemes expose all cells to a phytotoxic agent and rely on the introduction of a resistance gene to recover transformants. Unfortunately, the presence of dying cells may reduce the efficiency of stable transformation. It would therefore be useful to provide a positive selection system for recovering transformants.

In spite of increases in yield and harvested area worldwide, it is predicted that over the next ten years, meeting the demand for corn will require an additional 20% increase over current production (Dowswell, C.R., Paliwal, R.L., Cantrell, R.P., 1996, Maize in the Third World, Westview Press, Boulder, CO).

In hybrid crops, including grains, oil seeds, forages, fruits and vegetables, there are problems associated with the development and production of hybrid seeds. The process of cross-pollination of plants is laborious and expensive. In the cross-pollination process, the female plant must be prevented from being fertilized by its own pollen. Many methods have been developed over the years, such as detasseling in the case of corn, developing and maintaining male sterile lines, and developing plants that are incompatible with their own pollen, to name a few. Since hybrids do not breed true, the process must be repeated for the production of every hybrid seed lot.

To further complicate the process, inbred lines are crossed. For example in the case of corn, the inbreds can be low yielding. This provides a major challenge in the production of hybrid seed corn. In fact, certain hybrids cannot be commercialized at all due to the performance of the inbred lines. The production of hybrid seeds is consequently expensive, time consuming and provides known and unknown risks. It would therefore be valuable to develop new methods that contribute to the increase of production efficiency of hybrid seed.

As new traits are added to commercial crops by means of genetic engineering, problems arise in "stacking" traits. In order to develop heritable stacked traits, the traits must be linked because of segregating populations. Improved methods for developing hybrid seed that would not require linking of the traits would significantly shorten the time for developing commercial hybrid seeds.

Gene silencing is another problem in developing heritable traits with genetic engineering. Frequently gene silencing is seen following meiotic divisions. Elimination or reduction of this problem would advance the state of science and industry in this area.

### SUMMARY OF THE INVENTION

### DETAILED DESCRIPTION OF THE INVENTION

### DEFINITIONS

The term "isolated" refers to material, such as a nucleic acid or a protein, which is: (1) substantially or essentially free from components which normally accompany or interact with the material as found in its naturally occurring environment or (2) if the material is in its natural environment, the material has been altered by deliberate human intervention to a composition and/or placed at a locus in the cell other than the locus native to the material.

As used herein, "nucleic acid" means a polynucleotide and includes single or double-stranded polymer of deoxyribonucleotide or ribonucleotide bases. Nucleic acids may also include fragments and modified nucleotides.

As used herein, "CHD polynucleotide" means a nucleic acid sequence encoding a CHD polypeptide.

As used herein, "polypeptide" means proteins, protein fragments, modified proteins, amino acid sequences and synthetic amino acid sequences. The polypeptide can be glycosylated or not.

As used herein, "CHD polypeptide" means a polypeptide containing 3 domains, a chromatin organization modifier, a helicase SNF-2 related/ATP domain, and a DNA binding domain.

As used herein, "plant" includes plants and plant parts including but not limited to plant cells, plant tissue such as leaves, stems, roots, flowers, and seeds.

As used herein, "promoter" includes reference to a region of DNA upstream from the start of transcription and involved in recognition and binding of RNA polymerase and other proteins to initiate transcription.

By "fragment" is intended a portion of the nucleotide sequence or a portion of the amino acid sequence and hence protein encoded thereby. Fragments of a nucleotide sequence may encode protein fragments that retain the biological activity of the native nucleic acid. Alternatively, fragments of a nucleotide sequence that are useful as hybridization probes may not encode fragment proteins retaining biological activity. Thus, fragments of a nucleotide sequence are generally greater than 25, 50, 100, 200, 300, 400, 500, 600, or 700 nucleotides and up to and including the entire nucleotide sequence encoding the proteins of the invention. Generally the probes are less than 1000 nucleotide and preferably less than 500 nucleotide. Fragments of the invention include antisense sequences used to decrease expression of the inventive polynucleotides. Such antisense fragments may vary in length ranging from greater than 25, 50, 100, 200, 300, 400, 500, 600, or 700 nucleotides and up to and including the entire coding sequence.

By "functional equivalent" as applied to a polynucleotide or a protein is intended a polynucleotide or a protein of sufficient length to modulate the level of CHD protein activity in a plant cell. A polynucleotide functional equivalent can be in sense or antisense orientation.

By "variants" is intended substantially similar sequences. Generally, nucleic acid sequence variants of the invention will have at least

90%, 95% or 98% sequence identity to the native nucleotides sequence, wherein the % sequence identity is based on the entire inventive sequence and is determined by GAP 10 analysis using default parameters. Generally, polypeptide sequence variants of the invention will have at least about

90%, 95% or 98% sequence identity to the native protein, wherein the % sequence identity is based on the entire sequence and is determined by GAP 10 analysis using default parameters. GAP uses the algorithm of Needleman and Wunsch (J. Mol. Biol. 48:443-453,1970) to find the alignment of two complete sequences that maximizes the number of matches and minimizes the number of gaps.

As used herein a "responsive cell" refers to a cell that exhibits a positive response to the introduction of CHD polypeptide or CHD polynucleotide compared to a cell that has not been introduced with CHD polypeptide or CHD polynucleotide. The response can be to enhance tissue culture response, induce somatic embryogenesis, induce apomixis, increase transformation efficiency or increase recovery of regenerated plants.

As used herein a "recalcitrant plant cell" is a plant cell that exhibits unsatisfactory tissue culture response, transformation efficiency or recovery of regenerated plants compared to model systems. In maize such a model system is GS3. Elite maize inbreds are typically recalcitrant. In soybeans such model systems are Peking or Jack.

As used herein "Transformation" includes stable transformation and transient transformation unless indicated otherwise.

As used herein "Stable Transformation" refers to the transfer of a nucleic acid fragment into a genome of a host organism (this includes both nuclear and organelle genomes) resulting in genetically stable inheritance. In addition to traditional methods, stable transformation includes the alteration of gene expression by any means including chimerplasty or transposon insertion.

As used herein "Transient Transformation" refers to the transfer of a nucleic acid fragment or protein into the nucleus (or DNA-containing organelle) of a host organism resulting in gene expression without integration and stable inheritance.

As used herein, a "CHD-silencing" construct as an expression cassette whose transcribed mRNA or translated protein will diminish the functional expression of active CHD in the cell. Such silencing can be achieved through expression of an antisense construct targeted against the CHD structural gene, a vector in which the CHD structural gene or a portion of this sequence is used to make a silencing hairpin (or where silencing hairpin is conjoined to the CHD sequence in some fashion), or where a CHD-overexpression cassette is used to co-suppress endogenous CHD levels. Reducing activity of endogenous CHD protein can also be achieved through expression of a transgene encoding an antibody (including single chain antibodies) directed against a critical functional domain within the CHD molecule (for example, an antibody that was raised against the chromo-domain of CHD).

### NUCLEIC ACIDS

Expression of CHD genes and their localization within the cell modulate their chromatin-organizing function. Several CHD1-binding sites have been found in the nuclear matrix attachment region from mouse chromosomes, suggesting that this protein binds to chromosomes, at least during certain stages of the cell cycle. When cells enter mitosis, CHD1 has been shown in mouse cells to be released into the cytoplasm.

In an effort to elucidate the effect of gibberellic acid on *Arabidopsis* root development, a group of scientists in UC Berkely (Sung's lab) and Carnegie Institute of Washington (Sommerville's lab) discovered an *Arabidopsis* mutant called pickle (pkl). The primary root meristem of the pkl plant has embryonic characteristics. Root tissues from pickle plants can regenerate new embryos and plants without hormone induction (Ogas et al., Science 277: 91-94, 1997). This observation suggested that the pkl gene serves as a key repressor for plant embryogenesis. The gene was mapped to a position near 48.4 on chromosome 2. The sequence of AtPickle was then published (Ogas et al., PNAS 96: 13839-13844, 1999) and was found to be a CHD3 homologue. Interestingly, the *Arabidopsis* gymnos (gym) mutant was recently found to be allelic to pkl. GYM (PKL) acts as a suppressor to repress genes that promote meristematic activities (Eshed et al., Cell 99: 199-209, 1999).

Since the identification of the first CHD gene (MmCHD1, Delmas et al., PNAS 90:2414-2418, 1993), a total of 13 highly conserved genes have so far been isolated. AtPKL and AtPKL-related genes are the only CHD genes isolated from plants.

CHD genes are required for appropriate inhibition of the transcription of important genes during development. Most likely, they are also required to be nonfunctional during embryogenesis and/or cell division. For those cells in which the key repressors are still on, overexpression of downstream, stimulatory genes may not be able to overcome the repression and consequently, no enhancement of transformation would be observed. Thus, manipulation of key repressor genes such that the repressor activity is transiently inhibited (antisense, cosuppression, antibody, etc.) may be an approach to establish an environment of embryogenesis and/or organogenesis. Working alone or together with LEC1, RepA or CycD, this approach may improve transformation.

In addition, modulating specific aspects of developmental pathways such as embryogenesis can be used to create high oil crops. Moreover, the family of CHD genes can be used to specifically shut down gene expression by engineering of specific DNA binding domains.

In many cases of apomixis maternal tissues such as the nucellus or inner integument "bud off" producing somatic embryos. These embryos then develop normally into seed. Since meiosis and fertilization are circumvented, the plants developing from such seed are genetically identical to the maternal plant. Suppression of expression of the CHD gene in the nucellus integument, or in the megaspore mother cell is expected to trigger embryo formation from maternal tissues.

Producing a seed identical to the parent has many advantages. For example high yielding hybrids could be used in seed production to multiply identical copies of high yielding hybrid seed. This would greatly reduce seed cost as well as increase the number of genotypes that are commercially available. Genes can be evaluated directly in commercial hybrids since the progeny would not segregate. This would save years of back crossing.

Apomixis would also provide a method of containment of transgenes when coupled with male sterility. The construction of male sterile autonomous agamospermy would prevent genetically engineered traits from hybridizing with weedy relatives.

Gene stacking would be relatively easy with apomixis. Hybrids could be successively re-transformed with various new traits and propagated via apomixis. The traits would not need to be linked since apomixis avoids the problems associated with segregation.

Apomixis can provide a reduction in gene silencing. Gene silencing is frequently seen following meiotic divisions. Since meiotic divisions never occur, it may be possible to eliminate or reduce the frequency of gene silencing. Apomixis can also be used to stabilize desirable phenotypes with complex traits such as hybrid vigor. Such traits could easily be maintained and multiplied indefinitely via apomixis.

Suppression of the CHD gene in transformed cells appears to initiate embryo development and stimulate development of pre-existing embryos. Reduced expression of the CHD gene should stimulate growth of transformed cells, but also insure that transformed somatic embryos develop in a normal, viable fashion (increasing the capacity of transformed somatic embryos to germinate vigorously).

Suppression of the CHD gene will stimulate growth in cells with the potential to initiate or maintain embryogenic growth. Cells in established meristems or meristem-derive cell lineages may be less prone to undergo the transition to embryos.

The isolated nucleic acids of the present invention can be made using (a) standard recombinant methods, (b) synthetic techniques, or combinations thereof. In some embodiments, the polynucleotides of the present invention will be cloned, amplified, or otherwise constructed from a monocot or dicot. Typical examples of monocots are corn, sorghum, barley, wheat, millet, rice, or turf grass. Typical dicots include soybeans, sunflower, canola, alfalfa, potato, or cassava.

Functional fragments included in the invention can be obtained using primers that selectively hybridize under stringent conditions. Primers are generally at least 12 bases in length and can be as high as 200 bases, but will generally be from 15 to 75, preferably from 15 to 50 bases. Functional fragments can be identified using a variety of techniques such as restriction analysis, Southern analysis, primer extension analysis, and DNA sequence analysis.

The present invention includes a plurality of polynucleotides that encode for the identical amino acid sequence. The degeneracy of the genetic code allows for such "silent variations" which can be used, for example, to selectively hybridize and detect allelic variants of polynucleotides of the present invention. Additionally, the present invention includes isolated nucleic acids comprising allelic variants. The term "allele" as used herein refers to a related nucleic acid of the same gene.

Variants of nucleic acids included in the invention can be obtained, for example, by oligonucleotide-directed mutagenesis, linker-scanning mutagenesis, mutagenesis using the polymerase chain reaction, and the like. See, for example, Ausubel, pages 8.0.3 - 8.5.9. Also, see generally, McPherson (ed.), DIRECTED MUTAGENESIS: A Practical Approach, (IRL Press, 1991). Thus, the present invention also encompasses DNA molecules comprising nucleotide sequences that have substantial sequence similarity with the inventive sequences.

Variants included in the invention may contain individual substitutions, deletions or additions to the nucleic acid or polypeptide sequences which alters, adds or deletes a single amino acid or a small percentage of amino acids in the encoded sequence is a "conservatively modified variant" where the alteration results in the substitution of an amino acid with a chemically similar amino acid. When the nucleic acid is prepared or altered synthetically, advantage can be taken of known codon preferences of the intended host.

The present invention also includes "shufflents" produced by sequence shuffling of the inventive polynucleotides to obtain a desired characteristic. Sequence shuffling is described in PCT publication No. 96/19256. See also, Zhang, J. H., et al., Proc. Natl. Acad. Sci. USA 94:4504-4509 (1997).

The present invention also includes the use of 5' and/or 3' UTR regions for modulation of translation of heterologous coding sequences. Positive sequence motifs include translational initiation consensus sequences (Kozak, Nucleic Acids Res.15:8125 (1987)) and the 7-methylguanosine cap structure (Drummond et al., Nucleic Acids Res. 13:7375 (1985)). Negative elements includes stable intramolecular 5' UTR stem-loop structures (Muesing et al., Cell 48:691 (1987)) and AUG sequences or short open reading frames preceded by an appropriate AUG in the 5' UTR (Kozak, *supra,* Rao et al., Mol. and Cell. Biol. 8:284 (1988)).

Further, the polypeptide-encoding segments of the polynucleotides of the present invention can be modified to alter codon usage. Altered codon usage can be employed to alter translational efficiency. Codon usage in the coding regions of the polynucleotides of the present invention can be analyzed statistically using commercially available software packages such as "Codon Preference" available from the University of Wisconsin Genetics Computer Group (see Devereaux et al., Nucleic Acids Res. 12:387-395 (1984)) or MacVector 4.1 (Eastman Kodak Co., New Haven, Conn.).

For example, the inventive nucleic acids can be optimized for enhanced expression in plants of interest. See, for example, EPA0359472; WO91/16432; Perlak et al. (1991) Proc. Natl. Acad. Sci. USA 88:3324-3328; and Murray et al. (1989) Nucleic Acids Res.17:477-498. In this manner, the polynucleotides can be synthesized utilizing plant-preferred codons. See, for example, Murray et al. (1989) Nucleic Acids Res. 17:477-498.

The present invention provides subsequences comprising isolated nucleic acids containing at least 20 contiguous bases of the inventive sequences. For example the isolated nucleic acid includes those comprising at least 20, 30, 40, 50, 60, 70, 80, 90, 100, 200, 300, 400, or 500 contiguous nucleotides of the inventive sequences. Subsequences of the isolated nucleic acid can be used to modulate or detect gene expression by introducing into the subsequences compounds which bind, intercalate, cleave and/or crosslink to nuclei acids.

The nucleic acids of the invention may conveniently comprise a multicloning site comprising one or more endonuclease restriction sites inserted into the nucleic acid to aid in isolation of the polynucleotide. Also, translatable sequences may be inserted to aid in the isolation of the translated polynucleotide of the present invention. For example, a hexa-histidine marker sequence provides a convenient means to purify the proteins of the present invention.

A polynucleotide of the present invention can be attached to a vector, adapter, promoter, transit peptide or linker for cloning and/or expression of a polynucleotide of the present invention. Additional sequences may be added to such cloning and/or expression sequences to optimize their function in cloning and/or expression, to aid in isolation of the polynucleotide, or to improve the introduction of the polynucleotide into a cell. Use of cloning vectors, expression vectors, adapters, and linkers is well known and extensively described in the art. For a description of such nucleic acids see, for example, Stratagene Cloning Systems, Catalogs 1995, 1996, 1997 (La Jolla, CA); and, Amersham Life Sciences, Inc, Catalog '97 (Arlington Heights, IL).

The isolated nucleic acid compositions of this invention, such as RNA, cDNA, genomic DNA, or a hybrid thereof, can be obtained from plant biological sources using any number of cloning methodologies known to those of skill in the art. In some embodiments, oligonucleotide probes that selectively hybridize, under stringent conditions, to the polynucleotides of the present invention are used to identify the desired sequence in a cDNA or genomic DNA library.

Exemplary total RNA and mRNA isolation protocols are described in Plant Molecular Biology: A Laboratory Manual, Clark, Ed., Springer-Verlag, Berlin (1997); and, Current Protocols in Molecular Biology, Ausubel, et al., Eds., Greene Publishing and Wiley-Interscience, New York (1995). Total RNA and mRNA isolation kits are commercially available from vendors such as Stratagene (La Jolla, CA), Clonetech (Palo Alto, CA), Pharmacia (Piscataway, NJ), and 5'-3' (Paoli, PA). See also, U.S. Patent Nos. 5,614,391; and, 5,459,253.

Typical cDNA synthesis protocols are well known to the skilled artisan and are described in such standard references as: Plant Molecular Biology: A Laboratory Manual, Clark, Ed., Springer-Verlag, Berlin (1997); and, Current Protocols in Molecular Biology, Ausubel, et al., Eds., Greene Publishing and Wiley-Interscience, New York (1995). cDNA synthesis kits are available from a variety of commercial vendors such as Stratagene or Pharmacia.

An exemplary method of constructing a greater than 95% pure full-length cDNA library is described by Caminci et al., Genomics, 37:327-336 (1996). Other methods for producing full-length libraries are known in the art. See, e.g., Edery et al., Mol. Cell Biol.15(6):3363-3371 (1995); and PCT Application WO 96/34981.

It is often convenient to normalize a cDNA library to create a library in which each clone is more equally represented. A number of approaches to normalize cDNA libraries are known in the art. Construction of normalized libraries is described in Ko, Nucl. Acids. Res. 18(19):5705-5711 (1990); Patanjali et al., Proc. Natl. Acad. U.S.A. 88:1943-1947 (1991); U.S. Patents 5,482,685 and 5,637,685; and Soares et al., Proc. Natl. Acad. Sci. USA 91:9228-9232 (1994).

Subtracted cDNA libraries are another means to increase the proportion of less abundant cDNA species. See, Foote et al. in, Plant Molecular Biology: A Laboratory Manual, Clark, Ed., Springer-Verlag, Berlin (1997); Kho and Zarbl, Technique 3(2):58-63 (1991); Sive and St. John, Nucl. Acids Res. 16(22):10937 (1988); Current Protocols in Molecular Biology, Ausubel, et al., Eds., Greene Publishing and Wiley-Interscience, New York (1995); and, Swaroop et al., Nucl. Acids Res. 19(8):1954 (1991). cDNA subtraction kits are commercially available. See, e.g., PCR-Select (Clontech).

To construct genomic libraries, large segments of genomic DNA are generated by random fragmentation. Examples of appropriate molecular biological techniques and instructions are found in Sambrook, et al., Molecular Cloning: A Laboratory Manual, 2nd Ed., Cold Spring Harbor Laboratory, Vols. 1-3 (1989), Methods in Enzymology, Vol. 152: Guide to Molecular Cloning Techniques, Berger and Kimmel, Eds., San Diego: Academic Press, Inc. (1987), Current Protocols in Molecular Biology, Ausubel, et al., Eds., Greene Publishing and Wiley-Interscience, New York (1995); Plant Molecular Biology: A Laboratory Manual, Clark, Ed., Springer-Verlag, Berlin (1997). Kits-for construction of genomic libraries are also commercially available.

The cDNA or genomic library can be screened using a probe based upon the sequence of a nucleic acid of the present invention such as those disclosed herein. Probes may be used to hybridize with genomic DNA or cDNA sequences to isolate homologous polynucleotides in the same or different plant species. Those of skill in the art will appreciate that various degrees of stringency of hybridization can be employed in the assay; and either the hybridization or the wash medium can be stringent. The degree of stringency can be controlled by temperature, ionic strength, pH and the presence of a partially denaturing solvent such as formamide.

Typically, stringent hybridization conditions will be those in which the salt concentration is less than about 1.5 M Na ion, typically about 0.01 to 1.0 M Na ion concentration (or other salts) at pH 7.0 to 8.3 and the temperature is at least about 30°C for short probes (e.g., 10 to 50 nucleotides) and at least about 60°C for long probes (e.g., greater than 50 nucleotides). Stringent conditions may also be achieved with the addition of destabilizing agents such as formamide.

Exemplary low stringency conditions include hybridization with a buffer solution of 30 to 35% formamide, 1 M NaCl, 1% SDS (sodium dodecyl sulfate) at 37°C, and a wash in 1X to 2X SSC (20X SSC = 3.0 M NaCl/0.3 M trisodium citrate) at 50°C. Exemplary moderate stringency conditions include hybridization in 40 to 45% formamide, 1 M NaCl, 1% SDS at 37°C, and a wash in 0.5X to 1X SSC at 55°C. Exemplary high stringency conditions include hybridization in 50% formamide, 1 M NaCl, 1% SDS at 37°C, and a wash in 0.1 X SSC at 60°C. Typically the time of hybridization is from 4 to 16 hours.

An extensive guide to the hybridization of nucleic acids is found in Tijssen, Laboratory Techniques in Biochemistry and Molecular Biology-Hybridization with Nucleic Acid Probes, Part I, Chapter 2 "Overview of principles of hybridization and the strategy of nucleic acid probe assays", Elsevier, New York (1993); and Current Protocols in Molecular Biology, Chapter 2, Ausubel, et al., Eds., Greene Publishing and Wiley-Interscience, New York (1995). Often, cDNA libraries will be normalized to increase the representation of relatively rare cDNAs.

The nucleic acids of the invention can be amplified from nucleic acid samples using amplification techniques. For instance, polymerase chain reaction (PCR) technology can be used to amplify the sequences of polynucleotides of the present invention and related polynucleotides directly from genomic DNA or cDNA libraries. PCR and other *in vitro* amplification methods may also be useful, for example, to clone nucleic acid sequences that code for proteins to be expressed, to make nucleic acids to use as probes for detecting the presence of the desired mRNA in samples, for nucleic acid sequencing, or for other purposes.

Examples of techniques useful for *in vitro* amplification methods are found in Berger, Sambrook, and Ausubel, as well as Mullis et al., U.S. Patent No. 4,683,202 (1987); and, PCR Protocols A Guide to Methods and Applications, Innis et al., Eds., Academic Press Inc., San Diego, CA (1990). Commercially available kits for genomic PCR amplification are known in the art. See, e.g., Advantage-GC Genomic PCR Kit (Clontech). The T4 gene 32 protein (Boehringer Mannheim) can be used to improve yield of long PCR products. PCR-based screening methods have also been described. Wilfinger et al. describe a PCR-based method in which the longest cDNA is identified in the first step so that incomplete clones can be eliminated from study. BioTechniques, 22(3):481-486 (1997).

In one aspect of the invention, nucleic acids can be amplified from a plant nucleic acid library. The nucleic acid library may be a cDNA library, a genomic library, or a library generally constructed from nuclear transcripts at any stage of intron processing. Libraries can be made from a variety of plant tissues. Good results have been obtained using mitotically active tissues such as shoot meristems, shoot meristem cultures, embryos, callus and suspension cultures, immature ears and tassels, and young seedlings. The cDNAs of the present invention were obtained from immature zygotic embryo and regenerating callus libraries.

Alternatively, the sequences of the invention can be used to isolate corresponding sequences in other organisms, particularly other plants, more particularly, other monocots. In this manner, methods such as PCR, hybridization, and the like can be used to identify such sequences having substantial sequence similarity to the sequences of the invention. See, for example, Sambrook et al. (1989) Molecular Cloning: A Laboratory Manual (2d ed., Cold Spring Harbor Laboratory Press, Plainview, New York). and Innis et al. (1990), PCR Protocols: A Guide to Methods and Applications (Academic Press, New York). Coding sequences isolated based on their sequence identity to the entire inventive coding sequences set forth herein or to fragments thereof are encompassed by the present invention.

The isolated nucleic acids of the present invention can also be prepared by direct chemical synthesis by methods such as the phosphotriester method of Narang et al., Meth. Enzymol. 68:90-99 (1979); the phosphodiester method of Brown et al., Meth. Enzymol. 68:109-151 (1979); the diethylphosphoramidite method of Beaucage et al., Tetra. Lett. 22:1859-1862 (1981); the solid phase phosphoramidite triester method described by Beaucage and Caruthers, Tetra. Letts. 22(20):1859-1862 (1981), *e.g.*, using an automated synthesizer, *e.g.*, as described in Needham-VanDevanter et al., Nucleic Acids Res. 12:6159-6168 (1984); and, the solid support method of U.S. Patent No. 4,458,066. Chemical synthesis generally produces a single stranded oligonucleotide. This may be converted into double stranded DNA by hybridization with a complementary sequence, or by polymerization with a DNA polymerase using the single strand as a template. One of skill will recognize that while chemical synthesis of DNA is limited to sequences of about 100 bases, longer sequences may be obtained by the ligation of shorter sequences.

The nucleic acids described herein include those amplified using the following primer pairs: SEQ ID NOS: 3 and 4; 7 and 8; 11 and 12; 15 and 16; 19 and 20; 23 and 24; 27 and 28; 31 and 32; 35 and 36; and 39 and 40.

### EXPRESSION CASSETTES

In another embodiment expression cassettes comprising isolated nucleic acids of the present invention are provided. An expression cassette will typically comprise a polynucleotide of the present invention operably linked to transcriptional initiation regulatory sequences which will direct the transcription of the polynucleotide in the intended host cell, such as tissues of a transformed plant.

The construction of such expression cassettes which can be employed in conjunction with the present invention is well known to those of skill in the art in light of the present disclosure. See, e.g., Sambrook, et al.; Molecular Cloning: A Laboratory Manual Cold Spring Harbor, New York; (1989); Gelvin, et al.; Plant Molecular Biology Manual (1990); Plant Biotechnology: Commercial Prospects and Problems, eds. Prakash, et al.; Oxford & IBH Publishing Co.; New Delhi. India; (1993); and Heslot, et al.; Molecular Biology and Genetic Engineering of Yeasts; CRC Press, Inc., USA; (1992).

For example, plant expression vectors may include (1) a cloned plant gene under the transcriptional control of 5' and 3' regulatory sequences and (2) a dominant selectable marker. Such plant expression vectors may also contain, if desired, a promoter regulatory region (e.g., one conferring inducible, constitutive, environmentally- or developmentally-regulated, or cell- or tissue-specific/selective expression), a transcription initiation start site, a ribosome binding site, an RNA processing signal, a transcription termination site, and/or a polyadenylation signal.

Constitutive, tissue-preferred or inducible promoters can be employed. Examples of constitutive promoters include the cauliflower mosaic virus (CaMV) 35S transcription initiation region, the 1'- or 2'- promoter derived from T-DNA of *Agrobacterium tumefaciens,* the actin promoter, the ubiquitin promoter, the histone H2B promoter (Nakayama et al., 1992, FEBS Lett 30:167-170), the Smas promoter, the cinnamyl alcohol dehydrogenase promoter (U.S. Patent No. 5,683,439), the *Nos* promoter, the pEmu promoter, the rubisco promoter, the GRP1-8 promoter, and other transcription initiation regions from various plant genes known in the art.

Examples of inducible promoters are the Adh1 promoter which is inducible by hypoxia or cold stress, the Hsp70 promoter which is inducible by heat stress, the PPDK promoter which is inducible by light, the In2 promoter which is safener induced, the ERE promoter which is estrogen induced and the Pepcarboxylase promoter which is light induced.

Examples of promoters under developmental control include promoters that initiate transcription preferentially in certain tissues, such as leaves, roots, fruit, seeds, or flowers. An exemplary promoter is the anther specific promoter 5126 (U.S. Patent Nos. 5,689,049 and 5,689,051). Examples of seed-preferred promoters include, but are not limited to, 27 kD gamma zein promoter and waxy promoter, Boronat, A., Martinez, M.C., Reina, M., Puigdomenech, P. and Palau, J.; Isolation and sequencing of a 28 kD glutelin-2 gene from maize: Common elements in the 5' flanking regions among zein and glutelin genes; Plant Sci. 47:95-102 (1986) and Reina, M., Ponte, I., Guillen, P., Boronat, A. and Palau, J., Sequence analysis of a genomic clone encoding a Zc2 protein from Zea mays W64 A, Nucleic Acids Res. 18(21):6426 (1990). See the following site relating to the waxy promoter: Kloesgen, R.B., Gierl, A., Schwarz-Sommer, Z.S. and Saedier, H., Molecular analysis of the waxy locus of Zea mays, Mol. Gen. Genet. 203:237-244 (1986).

The barley or maize Nuc1 promoter, the maize Cim 1 promoter or the maize LTP2 promoter can be used to preferentially express in the nucellus. See for example US Serial No. 60/097,233 filed August 20, 1998 the disclosure of which is incorporated herein by reference. Either heterologous or non-heterologous (i.e., endogenous) promoters can be employed to direct expression of the nucleic acids of the present invention. These promoters can also be used, for example, in expression cassettes to drive expression of antisense nucleic acids to reduce, increase, or alter concentration and/or composition of the proteins of the present invention in a desired tissue.

If polypeptide expression is desired, it is generally desirable to include a polyadenylation region at the 3'-end of a polynucleotide coding region. The polyadenylation region can be derived from the natural gene, from a variety of other plant genes, or from T-DNA. The 3' end sequence to be added can be derived from, for example, the nopaline synthase or octopine synthase genes, or alternatively from another plant gene, or less preferably from any other eukaryotic gene.

An intron sequence can be added to the 5' untranslated region or the coding sequence of the partial coding sequence to increase the amount of the mature message that accumulates. See for example Buchman and Berg, Mol. Cell Biol. 8:4395-4405 (1988); Callis et al., Genes Dev. 1:1183-1200 (1987). Use of maize introns Adh1-S intron 1, 2, and 6, the Bronze-1 intron are known in the art. See generally, The Maize Handbook, Chapter 116, Freeling and Walbot, Eds., Springer, New York (1994).

The vector comprising the sequences from a polynucleotide of the present invention will typically comprise a marker gene which confers a selectable phenotype on plant cells. Usually, the selectable marker gene will encode antibiotic or herbicide resistance. Suitable genes include those coding for resistance to the antibiotics spectinomycin and streptomycin (e.g., the aada gene), the streptomycin phosphotransferase (SPT) gene coding for streptomycin resistance, the neomycin phosphotransferase (NPTII) gene encoding kanamycin or geneticin resistance, the hygromycin phosphotransferase (HPT) gene coding for hygromycin resistance.

Suitable genes coding for resistance to herbicides include those which act to inhibit the action of acetolactate synthase (ALS), in particular the sulfonylurea-type herbicides (e.g., the acetolactate synthase (ALS) gene containing mutations leading to such resistance in particular the S4 and/or Hra mutations), those which act to inhibit action of glutamine synthase, such as phosphinothricin or basta (e.g., the *bar* gene), or other such genes known in the art. The *bar* gene encodes resistance to the herbicide basta and the ALS gene encodes resistance to the herbicide chlorsulfuron.

While useful in conjunction with the above antibiotic and herbicide-resistance selective markers (i.e. use of the CHD gene can increase transformation frequencies when using chemical selection), use of the CHD gene confers a growth advantage to transformed cells without the need for inhibitory compounds to retard non-transformed growth. Thus, CHD transformants are recovered based solely on their differential growth advantage.

Typical vectors useful for expression of genes in higher plants are well known in the art and include vectors derived from the tumor-inducing (Ti) plasmid of *Agrobacterium tumefaciens* described by Rogers et al., Meth. In Enzymol. 153:253-277 (1987). Exemplary *A. tumefaciens* vectors useful herein are plasmids pKYLX6 and pKYLX7 of Schardl et al., Gene, 61:1-11 (1987) and Berger et al., Proc. Natl. Acad. Sci. USA 86:8402-8406 (1989). Another useful vector herein is plasmid pBl101.2 that is available from Clontech Laboratories, Inc. (Palo Alto, CA).

A variety of plant viruses that can be employed as vectors are known in the art and include cauliflower mosaic virus (CaMV), geminivirus, brome mosaic virus, and tobacco mosaic virus.

A polynucleotide of the present invention can be expressed in either sense or anti-sense orientation as desired. In plant cells, it has been shown that antisense RNA inhibits gene expression by preventing the accumulation of mRNA which encodes the enzyme of interest, see, e.g., Sheehy et al., Proc. Natl. Acad. Sci. USA 85:8805-8809 (1988); and Hiatt et al., U.S. Patent No. 4,801,340.

Another method of suppression is sense suppression. Introduction of nucleic acid configured in the sense orientation has been shown to be an effective means by which to block the transcription of target genes. For an example of the use of this method to modulate expression of endogenous genes see, Napoli et al., The Plant Cell 2:279-289 (1990) and U.S. Patent No. 5,034,323. Recent work has shown suppression with the use of double stranded RNA. Such work is described in Tabara et al., Science 282:5388:430-431 (1998). Hairpin approaches of gene suppression are disclosed in WO 98/53083 and WO 99/53050.

Catalytic RNA molecules or ribozymes can also be used to inhibit expression of plant genes. The inclusion of ribozyme sequences within antisense RNAs confers RNA-cleaving activity upon them, thereby increasing the activity of the constructs. The design and use of target RNA-specific ribozymes is described in Haseloff et al., Nature 334:585-591 (1988).

A variety of cross-linking agents, alkylating agents and radical generating species as pendant groups on polynucleotides of the present invention can be used to bind, label, detect, and/or cleave nucleic acids. For example, Vlassov, V. V., et al., Nucleic Acids Res (1986) 14:4065-4076, describe covalent bonding of a single-stranded DNA fragment with alkylating derivatives of nucleotides complementary to target sequences. A report of similar work by the same group is that by Knorre, D. G., et al., Biochimie (1985) 67:785-789. Iverson and Dervan also showed sequence-specific cleavage of single-stranded DNA mediated by incorporation of a modified nucleotide which was capable of activating cleavage (J. Am. Chem. Soc. (1987) 109:1241-1243). Meyer, R. B., et al., J. Am. Chem. Soc. (1989) 111:8517-8519, effect covalent crosslinking to a target nucleotide using an alkylating agent complementary to the single-stranded target nucleotide sequence. A photoactivated crosslinking to single-stranded oligonucleotides mediated by psoralen was disclosed by Lee, B. L., et al., Biochemistry (1988) 27:3197-3203. Use of crosslinking in triple-helix forming probes was also disclosed by Home, et al., J. Am. Chem. Soc. (1990) 112:2435-2437. Use of N4, N4-ethanocytosine as an alkylating agent to crosslink to single-stranded oligonucleotides has also been described by Webb and Matteucci, J. Am. Chem. Soc. (1986) 108:2764-2765; Nucleic Acids Res (1986) 14:7661-7674; Feteritz et al., J. Am. Chem. Soc. 113:4000 (1991). Various compounds to bind, detect, label, and/or cleave nucleic acids are known in the art. See, for example, U.S. Patent Nos. 5,543,507; 5,672,593; 5,484,908; 5,256,648; and, 5,681941.

### Proteins

CHD proteins are named for the three functional domains they contain. These include: a modifier of chromatin organization, a helicase/ATPase domain (similar to the chromatin-remodeling factor (SNF2) first found in yeast, named after a "sucrose non-fermenting" mutant, and a DNA-binding domain. CHD proteins are suggested to be involved in a range of basic processes including modification of chromatin structure, DNA repair, regulation of transcription, etc. In particular. CHD proteins inhibit transcription probably by binding to relatively long AT tracts in double-stranded DNA via minor-groove interactions. CHD proteins fall into two sub-families. CHD1 and CHD2 belong to the first sub-family while CHD3 and CHD4 belong to the second sub-family. A major difference between these two sub-families is that the CHD of the second sub-family has a zinc-finger domain in the N-terminal end which was thought to interact with histone deacetylases. Another feature is that the DNA-binding regions of the second sub-family members are more divergent than those of the first sub-family members.

Proteins of the present invention include proteins having the disclosed sequences as well proteins coded by the disclosed polynucleotides. In addition proteins of the present invention include proteins derived from the native protein by deletion (so-called truncation), addition or substitution of one or more amino acids at one or more sites in the native protein. Such variants may result from, for example, genetic polymorphism or from human manipulation. Methods for such manipulations are generally known in the art.

For example, amino acid sequence variants of the polypeptide can be prepared by mutations in the cloned DNA sequence encoding the native protein of interest. Methods for mutagenesis and nucleotide sequence alterations are well known in the art. See, for example, Walker and Gaastra, eds. (1983) Techniques in Molecular Biology (MacMillan Publishing Company, New York); Kunkel (1985) Proc. Natl. Acad. Sci. USA 82:488-492; Kunkel et al. (1987) Methods Enzymol. 154:367-382; Sambrook et al. (1989) Molecular Cloning: A Laboratory Manual (Cold Spring Harbor, New York): U.S. Patent No. 4,873,192; and the references cited therein. Guidance as to appropriate amino acid substitutions that do not affect biological activity of the protein of interest may be found in the model of Dayhoff et al. (1978) Atlas of Protein Sequence and Structure (Natl. Biomed. Res. Found., Washington, D.C.). Conservative substitutions, such as exchanging one amino acid with another having similar properties, may be preferred.

In constructing variants of the proteins of interest, modifications to the nucleotide sequences encoding the variants will generally be made such that variants continue to possess the desired activity.

The isolated proteins of the present invention include a polypeptide comprising at least 30 contiguous amino acids encoded by any one of the nucleic acids of the present invention, or polypeptides that are conservatively modified variants thereof. The proteins of the present invention or variants thereof can comprise any number of contiguous amino acid residues from a polypeptide of the present invention, wherein that number is selected from the group of integers consisting of from 25 to the number of residues in a full-length polypeptide of the present invention. Optionally, this subsequence of contiguous amino acids is at least 25, 30, 40, 50, 60, 70, 80, 90, 100, 150, 200, 250, 300, 350, 400, 450, or 500 amino acids in length.

The present invention includes catalytically active polypeptides (i.e., enzymes). Catalytically active polypeptides will generally have a specific activity of at least about 20%, 30%, 40%, 50%, 60%, 70%, 80%, 90%, or 95% that of the native (non-synthetic), endogenous polypeptide. Further, the substrate specificity (k_{cat}/Kₘ) is optionally substantially similar to the native (non-synthetic), endogenous polypeptide. Typically, the Kₘ will be at least about 30%, 40%, 50%, 60%, 70%, 80%, 90%, or 95% that of the native (non-synthetic), endogenous polypeptide. Methods of assaying and quantifying measures of enzymatic activity and substrate specificity (k_{cat}/Kₘ), are well known to those of skill in the art.

The present invention includes modifications that can be made to an inventive protein. In particular, it may be desirable to diminish the activity of the gene. Other modifications may be made to facilitate the cloning, expression, or incorporation of the targeting molecule into a fusion protein. Such modifications are well known to those of skill in the art and include, for example, a methionine added at the amino terminus to provide an initiation site, or additional amino acids (*e.g.*, poly His) placed on either terminus to create conveniently located restriction sites or termination codons or purification sequences.

Using the nucleic acids of the present invention, one may express a protein of the present invention in recombinantly engineered cells such as bacteria, yeast, insect, mammalian, or plant cells. The cells produce the protein in a non-natural condition (e.g., in quantity, composition, location, and/or time), because they have been genetically altered through human intervention to do so.

Typically, an intermediate host cell will be used in the practice of this invention to increase the copy number of the cloning vector. With an increased copy number, the vector containing the gene of interest can be isolated in significant quantities for introduction into the desired plant cells.

Host cells that can be used in the practice of this invention include prokaryotes and eukaryotes. Prokaryotes include bacterial hosts such as *Eschericia coli, Salmonella typhimurium,* and *Serratia marcescens.* Eukaryotic hosts such as yeast or filamentous fungi may also be used in this invention. Since these hosts are also microorganisms, it will be essential to ensure that plant promoters which do not cause expression of the polypeptide in bacteria are used in the vector.

Commonly used prokaryotic control sequences include such commonly used promoters as the beta lactamase (penicillinase) and lactose (lac) promoter systems (Chang et al., Nature 198:1056 (1977)), the tryptophan (trp) promoter system (Goeddel et al., Nucleic Acids Res. 8:4057 (1980)) and the lambda derived P L promoter and N-gene ribosome binding site (Shimatake et al., Nature 292:128 (1981)). The inclusion of selection markers in DNA vectors transfected in *E. coli* is also useful. Examples of such markers include genes specifying resistance to ampicillin, tetracycline, or chloramphenicol.

The vector is selected to allow introduction into the appropriate host cell. Bacterial vectors are typically of plasmid or phage origin. Expression systems for expressing a protein of the present invention are available using *Bacillus sp.* and *Salmonella* (Palva, et al., Gene 22:229-235 (1983); Mosbach, et al., Nature 302:543-545 (1983)).

Synthesis of heterologous proteins in yeast is well known. See Sherman, F., et al., Methods in Yeast Genetics, Cold Spring Harbor Laboratory (1982). Two widely utilized yeast for production of eukaryotic proteins are *Saccharomyces cerevisiae* and *Pichia pastoris.* Vectors, strains, and protocols for expression in Saccharomyces and *Pichia* are known in the art and available from commercial suppliers (e.g., Invitrogen). Suitable vectors usually have expression control sequences, such as promoters, including 3-phosphoglycerate kinase or alcohol oxidase, and an origin of replication, termination sequences and the like as desired.

A protein of the present invention, once expressed, can be isolated from yeast by lysing the cells and applying standard protein isolation techniques to the lysates. The monitoring of the purification process can be accomplished by using Western blot techniques or radioimmunoassay of other standard immunoassay techniques.

The proteins of the present invention can also be constructed using non-cellular synthetic methods. Solid phase synthesis of proteins of less than about 50 amino acids in length may be accomplished by attaching the C-terminal amino acid of the sequence to an insoluble support followed by sequential addition of the remaining amino acids in the sequence. Techniques for solid phase synthesis are described by Barany and Merrifield, Solid-Phase Peptide Synthesis, pp. 3-284 in The Peptides: Analysis, Synthesis, Biology. Vol. 2: Special Methods in Peptide Synthesis, Part A*.;* Merrifield, et al., J. Am. Chem. Soc. 85:2149-2156 (1963), and Stewart et al., Solid Phase Peptide Synthesis, 2nd ed., Pierce Chem. Co., Rockford, III. (1984). Proteins of greater length may be synthesized by condensation of the amino and carboxy termini of shorter fragments. Methods of forming peptide bonds by activation of a carboxy terminal end (e.g., by the use of the coupling reagent N,N'-dicycylohexylcarbodiimide)) is known to those of skill.

The proteins of this invention, recombinant or synthetic, may be purified to substantial purity by standard techniques well known in the art, including detergent solubilization, selective precipitation with such substances as ammonium sulfate, column chromatography, immunopurification methods, and others. See, for instance, R. Scopes, Protein Purification: Principles and Practice, Springer-Verlag: New York (1982); Deutscher, Guide to Protein Purification, Academic Press (1990). For example, antibodies may be raised to the proteins as described herein. Purification from *E*. *coli* can be achieved following procedures described in U.S. Patent No. 4,511,503. Detection of the expressed protein is achieved by methods known in the art and include, for example, radioimmunoassays, Western blotting techniques or immunoprecipitation.

The present invention further provides a method for modulating (i.e., increasing or decreasing) the concentration or composition of the polypeptides of the present invention in a plant or part thereof. Modulation can be effected by increasing or decreasing the concentration and/or the composition (i.e., the ratio of the polypeptides of the present invention) in a plant.

The method comprises transforming a plant cell with an expression cassette comprising a polynucleotide of the present invention to obtain a transformed plant cell, growing the transformed plant cell under conditions allowing expression of the polynucleotide in the plant cell in an amount sufficient to modulate concentration and/or composition in the plant cell.

In some embodiments, the content and/or composition of polypeptides of the present invention in a plant may be modulated by altering, *in vivo* or *in vitro,* the promoter of a non-isolated gene of the present invention to up- or down-regulate gene expression. In some embodiments, the coding regions of native genes of the present invention can be altered via substitution, addition, insertion, or deletion to decrease activity of the encoded enzyme. See, e.g., Kmiec, U.S. Patent 5,565,350; Zarling et al., PCT/US93/03868 (WO 93/22443). One method of down-regulation of the protein involves using PEST sequences that provide a target for degradation of the protein.

In some embodiments, an isolated nucleic acid (e.g., a vector) comprising a promoter sequence is transfected into a plant cell. Subsequently, a plant cell comprising the promoter operably linked to a polynucleotide of the present invention is selected for by means known to those of skill in the art such as, but not limited to, Southern blot, DNA sequencing, or PCR analysis using primers specific to the promoter and to the gene and detecting amplicons produced therefrom. A plant or plant part altered or modified by the foregoing embodiments is grown under plant forming conditions for a time sufficient to modulate the concentration and/or composition of polypeptides of the present invention in the plant Plant forming conditions are well known in the art.

In general, content of the polypeptide is increased or decreased by at least 5%, 10%, 20%, 30%, 40%, 50%, 60%, 70%, 80%, or 90% relative to a native control plant, plant part, or cell lacking the aforementioned expression cassette. Modulation in the present invention may occur during and/or subsequent to growth of the plant to the desired stage of development Modulating nucleic acid expression temporally and/or in particular tissues can be controlled by employing the appropriate promoter operably linked to a polynucleotide of the present invention in, for example, sense or antisense orientation as discussed in greater detail, *supra.* Induction of expression of a polynucleotide of the present invention can also be controlled by exogenous administration of an effective amount of inducing compound. Inducible promoters and inducing compounds which activate expression from these promoters are well known in the art. In preferred embodiments, the polypeptides of the present invention are modulated in monocots or dicots, preferably maize, soybeans, sunflower, sorghum, canola, wheat, alfalfa, rice, barley and millet.

Means of detecting the proteins of the present invention are not critical aspects of the present invention. In a preferred embodiment, the proteins are detected and/or quantified using any of a number of well recognized immunological binding assays (see, *e.g.,* U.S. Patents 4,366,241; 4,376,110; 4,517,288; and 4,837,168). For a review of the general immunoassays, see also Methods in Cell Biology, Vol. 37: Antibodies in Cell Biology, Asai, Ed., Academic Press, Inc. New York (1993); Basic and Clinical Immunology 7th Edition, Stites & Terr, Eds. (1991). Moreover, the immunoassays of the present invention can be performed in any of several configurations, *e.g.*, those reviewed in Enzyme Immunoassay, Maggio, Ed., CRC Press, Boca Raton, Florida (1980); Tijan, Practice and Theory of Enzyme Immunoassays, Laboratory Techniques in Biochemistry and Molecular Biology, Elsevier Science Publishers B.V., Amsterdam (1985); Harlow and Lane, *supra;* Immunoassay: A Practical Guide, Chan, Ed., Academic Press, Orlando, FL (1987); Principles and Practice of Immunoassays, Price and Newman Eds., Stockton Press, NY (1991); and Non-isotopic Immunoassays, Ngo, Ed., Plenum Press, NY (1988).

Typical methods include Western blot (immunoblot) analysis, analytic biochemical methods such as electrophoresis, capillary electrophoresis, high performance liquid chromatography (HPLC), thin layer chromatography (TLC), hyperdiffusion chromatography, and the like, and various immunological methods such as fluid or gel precipitin reactions, immunodiffusion (single or double), immunoelectrophoresis, radioimmunoassays (RIAs), enzyme-linked immunosorbent assays (ELISAs), immunofluorescent assays, and the like.

Non-radioactive labels are often attached by indirect means. Generally, a ligand molecule (*e.g.*, biotin) is covalently bound to the molecule. The ligand then binds to an anti-ligand (*e.g.*, streptavidin) molecule which is either inherently detectable or covalently bound to a signal system, such as a detectable enzyme, a fluorescent compound, or a chemiluminescent compound. A number of ligands and anti-ligands can be used. Where a ligand has a natural anti-ligand, for example, biotin, thyroxine, and cortisol, it can be used in conjunction with the labeled, naturally occurring anti-ligands. Alternatively, any haptenic or antigenic compound can be used in combination with an antibody.

The molecules can also be conjugated directly to signal generating compounds, *e.g.*, by conjugation with an enzyme or fluorophore. Enzymes of interest as labels will primarily be hydrolases, particularly phosphatases, esterases and glycosidases, or oxidoreductases, particularly peroxidases. Fluorescent compounds include fluorescein and its derivatives, rhodamine and its derivatives, dansyl, umbelliferone, etc. Chemiluminescent compounds include luciferin, and 2,3-dihydrophthalazinediones, e.g., luminol. For a review of various labeling or signal producing systems which may be used, see, U.S. Patent No. 4,391,904.

Some assay formats do not require the use of labeled components. For instance, agglutination assays can be used to detect the presence of the target antibodies. In this case, antigen-coated particles are agglutinated by samples comprising the target antibodies. In this format, none of the components need be labeled and the presence of the target antibody is detected by simple visual inspection.

The proteins of the present invention can be used for identifying compounds that bind to (e.g., substrates), and/or increase or decrease (i.e., modulate) the enzymatic activity of, catalytically active polypeptides of the present invention. The method comprises contacting a polypeptide of the present invention with a compound whose ability to bind to or modulate enzyme activity is to be determined. The polypeptide employed will have at least 20%, 30%, 40%, 50%, 60%, 70%, 80%, 90% or 95% of the specific activity of the native, full-length polypeptide of the present invention (e.g., enzyme). Methods of measuring enzyme kinetics are well known in the art. See, e.g., Segel, Biochemical Calculations, 2nd ed., John Wiley and Sons, New York (1976).

Antibodies can be raised to a protein of the present invention, including individual, allelic, strain, or species variants, and fragments thereof, both in their naturally occurring (full-length) forms and In recombinant forms. Additionally, antibodies are raised to these proteins in either their native configurations or in non-native configurations. Anti-idiotypic antibodies can also be generated. Many methods of making antibodies are known to persons of skill.

In some instances, it is desirable to prepare monoclonal antibodies from various mammalian hosts, such as mice, rodents, primates, humans, *etc.* Description of techniques for preparing such monoclonal antibodies are found in, *e.g.,* Basic and Clinical Immunology, 4th ed., Stites et al., Eds., Lange Medical Publications, Los Altos, CA, and references cited therein; Harlow and Lane, *Supra;* Goding, Monoclonal Antibodies: Principles and Practice, 2nd ed., Academic Press, New York, NY (1986); and Kohler and Milstein, Nature 256:495-497 (1975).

Other suitable techniques involve selection of libraries of recombinant antibodies in phage or similar vectors (*see, e.g.,* Huse et al., Science 246:1275-1281 (1989); and Ward, et al., Nature 341:544-546 (1989); and Vaughan et al., Nature Biotechnology, 14:309-314 (1996)). Alternatively, high avidity human monoclonal antibodies can be obtained from transgenic mice comprising fragments of the unrearranged human heavy and light chain Ig loci (i.e., minilocus transgenic mice). Fishwild et al., Nature Biotech., 14:845-851 (1996). Also, recombinant immunoglobulins may be produced. See, Cabilly, U.S. Patent No. 4,816,567; and Queen et al., Proc. Natl. Acad. Sci. 86:10029-10033 (1989).

The antibodies of this invention can be used for affinity chromatography in isolating proteins of the present invention, for screening expression libraries for particular expression products such as normal or abnormal protein or for raising anti-idiotypic antibodies which are useful for detecting or diagnosing various pathological conditions related to the presence of the respective antigens.

Frequently, the proteins and antibodies of the present invention will be labeled by joining, either covalently or non-covalently, a substance which provides for a detectable signal. A wide variety of labels and conjugation techniques are known and are reported extensively in both the scientific and patent literature. Suitable labels include radionucleotides, enzymes, substrates, cofactors, inhibitors, fluorescent moieties, chemiluminescent moieties, magnetic particles, and the like.

### Transformation of Cells

The method of transformation is not critical to the present invention; various methods of transformation are currently available. As newer methods are available to transform crops or other host cells they may be directly applied. Accordingly, a wide variety of methods have been developed to insert a DNA sequence into the genome of a host cell to obtain the transcription and/or translation of the sequence to effect phenotypic changes in the organism. Thus, any method which provides for efficient transformation/transfection may be employed.

A DNA sequence coding for the desired polynucleotide of the present invention, for example a cDNA or a genomic sequence encoding a full length protein, can be used to construct an expression cassette which can be introduced into the desired plant. Isolated nucleic acid acids of the present invention can be introduced into plants according techniques known in the art. Generally, expression cassettes as described above and suitable for transformation of plant cells are prepared.

Techniques for transforming a wide variety of higher plant species are well known and described in the technical, scientific, and patent literature. See, for example, Weising et al., Ann. Rev. Genet. 22:421-477 (1988). For example, the DNA construct may be introduced directly into the genomic DNA of the plant cell using techniques such as electroporation, PEG poration, particle bombardment, silicon fiber delivery, or microinjection of plant cell protoplasts or embryogenic callus. See, e.g., Tomes et al., Direct DNA Transfer into Intact Plant Cells Via Microprojectile Bombardment. pp.197-213 in Plant Cell, Tissue and Organ Culture, Fundamental Methods. eds. O. L. Gamborg and G.C. Phillips. Springer-Verlag Berlin Heidelberg New York, 1995. Alternatively, the DNA constructs may be combined with suitable T-DNA flanking regions and introduced into a conventional *Agrobacterium tumefaciens* host vector. The virulence functions of the *Agrobacterium tumefaciens* host will direct the insertion of the construct and adjacent marker into the plant cell DNA when the cell is infected by the bacteria. See, U.S. Patent No. 5,591,616.

The introduction of DNA constructs using polyethylene glycol precipitation is described in Paszkowski et al., Embo J. 3:2717-2722 (1984). Electroporation techniques are described in Fromm et al., Proc. Natl. Acad. Sci. 82:5824 (1985). Ballistic transformation techniques are described in Klein et al., Nature 327:70-73 (1987).

*Agrobacterium tumefaciens*-meditated transformation techniques are well described in the scientific literature. See, for example Horsch et al., Science 233:496-498 (1984), and Fraley et al., Proc. Natl. Acad. Sci. 80:4803 (1983). For instance, *Agrobacterium* transformation of maize is described in US 5,981,840. *Agrobacterium* transformation of soybean is described in US Pat. No. 5,563,055.

Other methods of transformation include (1) *Agrobacterium rhizogenes-*mediated transformation (see, e.g., Lichtenstein and Fuller In: Genetic Engineering, Vol. 6, PWJ Rigby, Ed., London, Academic Press, 1987; and Lichtenstein, C. P., and Draper, J,. In: DNA Cloning, Vol. II, D. M. Glover, Ed., Oxford, IRI Press, 1985), Application PCT/US87/02512 (WO 88/02405 published Apr. 7, 1988) describes the use of *A. rhizogenes* strain A4 and its Ri plasmid along with *A. tumefaciens* vectors pARC8 or pARC16 (2) liposome-mediated DNA uptake (see, e.g., Freeman et al., Plant Cell Physiol. 25:1353, (1984)), (3) the vortexing method (see, e.g., Kindle, Proc. Natl. Acad. Sci. USA 87:1228, (1990)).

DNA can also be introduced into plants by direct DNA transfer into pollen as described by Zhou et al., Methods in Enzymology, 101:433 (1983); D. Hess, Intern Rev. Cytol., 107:367 (1987); Luo et al., Plant Mol. Biol. Reporter, 6:165 (1988). Expression of polypeptide coding polynucleotides can be obtained by injection of the DNA into reproductive organs of a plant as described by Pena et al., Nature, 325:274 (1987). DNA can also be injected directly into the cells of immature embryos and the rehydration of desiccated embryos as described by Neuhaus et al., Theor. Appl. Genet., 75:30 (1987); and Benbrook et al., in Proceedings Bio Expo 1986, Butterworth, Stoneham, Mass., pip. 27-54 (1986).

Animal and lower eukaryotic (e.g., yeast) host cells are competent or rendered competent for transformation by various means. There are several well-known methods of introducing DNA into animal cells. These include: calcium phosphate precipitation, fusion of the recipient cells with bacterial protoplasts containing the DNA, treatment of the recipient cells with liposomes containing the DNA, DEAE dextran, electroporation, biolistics, and micro-injection of the DNA directly into the cells. The transfected cells are cultured by means well known in the art. Kuchler, R.J., Biochemical Methods in Cell Culture and Virology, Dowden, Hutchinson and Ross, Inc. (1977).

### Altering the culture medium to suppress somatic embryogenesis in non-transformed plant cells and/or tissues to provide for a positive section means of transformed plant cells

Using the following methods for controlling somatic embryogenesis, it is possible to alter plant tissue culture media components to suppress somatic embryogenesis in a plant species of interest (often having multiple components that potentially could be adjusted to impart this effect). Such conditions would not impart a negative or toxic *in vitro* environment for wild-type tissue, but instead would simply not produce a somatic embryogenic growth form. Suppressing the expression of the CHD gene will stimulate somatic embryogenesis and growth in the transformed cells or tissue, providing a clear differential growth screen useful for identifying transformants.

Altering a wide variety of media components can modulate somatic embryogenesis (either stimulating or suppressing embryogenesis depending on the species and particular media component). Examples of media components which, when altered, can stimulate or suppress somatic embryogenesis include;
1) the basal medium itself (macronutrient, micronutrients and vitamins; see T.A. Thorpe, 1981 for review, "Plant Tissue Culture: Methods and Applications in Agriculture", Academic Press, NY),
2) plant phytohormones such as auxins (indole acetic acid, indole butyric acid, 2,4-dichlorophenoxyacetic acid, naphthaleneacetic acid, picloram, dicamba and other functional analogues), cytokinins (zeatin, kinetin, benzyl amino purine, 2-isopentyl adenine and functionally-related compounds) abscisic acid, adenine, and gibberellic acid,
3) and other compounds that exert "growth regulator" effects such as coconut water, casein hydrolysate, and proline, and
4) the type and concentration of gelling agent, pH and sucrose concentration.

Changes in the individual components listed above (or in some cases combinations of components) have been demonstrated in the literature to modulate *in vitro* somatic embryogenesis across a wide range of dicotyledonous and monocotyledonous species. For a compilation of examples, see E.F. George et al. 1987. Plant Tissue Culture Media, Vol. 1: Formulations and Uses. Exergetics, Ltd., Publ., Edington, England.

### Transgenic Plant Regeneration

Transformed plant cells which are derived by any of the above transformation techniques can be cultured to regenerate a whole plant which possesses the transformed genotype. Such regeneration techniques often rely on manipulation of certain phytohormones in a tissue culture growth medium, typically relying on a biocide and/or herbicide marker that has been introduced together with a polynucleotide of the present invention. For transformation and regeneration of maize see, Gordon-Kamm et al., The Plant Cell, 2:603-618 (1990).

Plants cells transformed with a plant expression vector can be regenerated, e.g., from single cells, callus tissue or leaf discs according to standard plant tissue culture techniques. It is well known in the art that various cells, tissues, and organs from almost any plant can be successfully cultured to regenerate an entire plant. Plant regeneration from cultured protoplasts is described in Evans et al., Protoplasts Isolation and Culture, Handbook of Plant Cell Culture, Macmillan Publishing Company, New York, pp. 124-176 (1983); and Binding, Regeneration of Plants, Plant Protoplasts, CRC Press, Boca Raton, pp. 21-73 (1985).

The regeneration of plants containing the foreign gene introduced by *Agrobacterium* can be achieved as described by Horsch et al., Science, 227:1229-1231 (1985) and Fraley et al., Proc. Natl. Acad. Sci. U.S.A. 80:4803 (1983). This procedure typically produces shoots within two to four weeks and these transformant shoots are then transferred to an appropriate root-inducing medium containing the selective agent and an antibiotic to prevent bacterial growth. Transgenic plants of the present invention may be fertile or sterile.

Regeneration can also be obtained from plant callus, explants, organs, or parts thereof. Such regeneration techniques are described generally in Klee et al., Ann. Rev. of Plant Phys. 38:467-486 (1987). The regeneration of plants from either single plant protoplasts or various explants is well known in the art. See, for example, Methods for Plant Molecular Biology, A. Weissbach and H. Weissbach, eds., Academic Press, Inc., San Diego, Calif. (1988). For maize cell culture and regeneration see generally, The Maize Handbook, Freeling and Walbot, Eds., Springer, New York (1994); Com and Com Improvement, 3rd edition, Sprague and Dudley Eds., American Society of Agronomy, Madison, Wisconsin (1988).

One of skill will recognize that after the expression cassette is stably incorporated in transgenic plants and confirmed to be operable, it can be introduced into other plants by sexual crossings Any of a number of standard breeding techniques can be used, depending upon the species to be crossed.

In vegetatively propagated crops, mature transgenic plants can be propagated by the taking of cuttings, via production of apomictic seed, or by tissue culture techniques to produce multiple identical plants. Selection of desirable transgenics is made and new varieties are obtained and propagated vegetatively for commercial use. In seed propagated crops, mature transgenic plants can be self crossed to produce a homozygous inbred plant. The inbred plant produces seed containing the newly introduced heterologous nucleic acid. These seeds can be grown to produce plants that would produce the selected phenotype.

Parts obtained from the regenerated plant, such as flowers, seeds, leaves, branches, fruit, and the like are included in the invention, provided that these parts comprise cells comprising the isolated nucleic acid of the present invention. Progeny and variants, and mutants of the regenerated plants are also included within the scope of the invention, provided that these parts comprise the introduced nucleic acid sequences.

Transgenic plants expressing a selectable marker can be screened for transmission of the nucleic acid of the present invention by, for example, standard immunoblot and DNA detection techniques. Transgenic lines are also typically evaluated on levels of expression of the heterologous nucleic acid. Expression at the RNA level can be determined initially to identify and quantitate expression-positive plants. Standard techniques for RNA analysis can be employed and include PCR amplification assays using oligonucleotide primers designed to amplify only the heterologous RNA templates and solution hybridization assays using heterologous nucleic acid-specific probes. The RNA-positive plants can then be analyzed for protein expression by Western immunoblot analysis using the specifically reactive antibodies of the present invention. In addition, *in situ* hybridization and immunocytochemistry according to standard protocols can be done using heterologous nucleic acid specific polynucleotide probes and antibodies, respectively, to localize sites of expression within transgenic tissue. Generally, a number of transgenic lines are usually screened for the incorporated nucleic acid to identify and select plants with the most appropriate expression profiles.

A preferred embodiment is a transgenic plant that is homozygous for the added heterologous nucleic acid; i.e., a transgenic plant that contains two added nucleic acid sequences, one gene at the same locus on each chromosome of a chromosome pair. A homozygous transgenic plant can be obtained by sexually mating (selfing) a heterozygous transgenic plant that contains a single added heterologous nucleic acid, germinating some of the seed produced and analyzing the resulting plants produced for altered expression of a polynucleotide of the present invention relative to a control plant (i.e., native, non-transgenic). Backcrossing to a parental plant and out-crossing with a non- transgenic plant are also contemplated. Alternatively, propagation of heterozygous transgenic plants could be accomplished through apomixis.

The present invention provides a method of genotyping a plant comprising a polynucleotide of the present invention. Genotyping provides a means of distinguishing homologs of a chromosome pair and can be used to differentiate segregants in a plant population. Molecular marker methods can be used for phylogenetic studies, characterizing genetic relationships among crop varieties, identifying crosses or somatic hybrids, localizing chromosomal segments affecting monogenic traits, map based cloning, and the study of quantitative inheritance. See, e.g., Plant Molecular Biology: A Laboratory Manual, Chapter 7, Clark, Ed., Springer-Verlag, Berlin (1997). For molecular marker methods, see generally, The DNA Revolution by Andrew H. Paterson 1996 (Chapter 2) in: Genome Mapping in Plants (ed. Andrew H. Paterson) by Academic Press/R. G. Landis Company, Austin, Texas, pp.7-21.

The particular method of genotyping in the present invention may employ any number of molecular marker analytic techniques such as, but not limited to, restriction fragment length polymorphisms (RFLPs). RFLPs are the product of allelic differences between DNA restriction fragments caused by nucleotide sequence variability. Thus, the present invention further provides a means to follow segregation of a gene or nucleic acid of the present invention as well as chromosomal sequences genetically linked to these genes or nucleic acids using such techniques as RFLP analysis.

### EXAMPLES

### Example 1

### Library construction used for the maize CHD EST's

### A. Total RNA Isolation

Total RNA was isolated from maize embryo and regenerating callus tissues with TRizol Reagent (Life Technology Inc. Gaithersburg, MD) using a modification of the guanidine isothiocyanate/acid-phenol procedure described by Chomczynski and Sacchi (Chomczynski, P., and Sacchl, N. Anal. Biochem. 162,156 (1987)). In brief, plant tissue samples were pulverized In liquid nitrogen before the addition of the TRizol Reagent; and then were further homogenized with a mortar and pestle. Addition of chloroform followed by centrifugation was conducted for separation of an aqueous phase and an organic phase. The total RNA was recovered by precipitation with isopropyl alcohol from the aqueous phase.

### B. Poly(A)+ RNA Isolation

The selection of poly(A)+ RNA from total RNA was performed using PolyATact system (Promega Corporation. Madison, WI). In brief, blotinylated oligo(dT) primers were used to hybridize to the 3' poly(A) tails on mRNA. The hybrids were captured using streptavidin coupled to paramagnetic particles and a magnetic separation stand. The mRNA was washed at high stringent condition and eluted by RNase-free deionized water.

### C. cDNA Library Construction

cDNA synthesis was performed and unidirectional cDNA libraries were constructed using the SuperScript Plasmid System (Life Technology Inc. Gaithersburg, MD). The first stand of cDNA was synthesized by priming an oligo(dT) primer containing a Not I site. The reaction was catalyzed by SuperScript Reverse Transcriptase II at 45°C. The second strand of cDNA was labeled with alpha-³²P-dCTP and a portion of the reaction was analyzed by agarose gel electrophoresis to determine cDNA sizes. cDNA molecules smaller than 500 base pairs and unligated adapters were removed by Sephacryl-S400 chromatography. The selected cDNA molecules were ligated into pSPORT1 vector in between of Not I and Sal I sites.

### D. Genomic library Construction into BAC (Bacterial Artificial chromosome) vectors.

BAC library were constructed according Texas A&M BAC center protocol. High molecular weight DNA isolated from line Mo17 embedded in LMP agarose microbeads were partially digested by HindIII. After partial digestion, the DNA was size-selected pulsed-field gel electrophoresis to remove the smaller DNA fragments that can compete more effectively than the larger DNA fragments for vector ends. The size-selected DNA fragments were ligated into pBeloBAC11 in HindIIII site.

### Example 2

### Sequencing and cDNA subtraction procedures used for maize CHD EST's

### A. Sequencing Template Preparation

Individual colonies were picked and DNA was prepared either by PCR with M13 forward primers and M13 reverse primers, or by plasmid isolation. All the cDNA clones were sequenced using M13 reverse primers.

### B. Q-bot Subtraction Procedure

cDNA libraries subjected to the subtraction procedure were plated out on 22 x 22 cm² agar plate at density of about 3,000 colonies per plate. The plates were incubated in a 37°C incubator for 12-24 hours. Colonies were picked into 384-well plates by a robot colony picker, Q-bot (GENETIX Limited). These plates were incubated overnight at 37°C.

Once sufficient colonies were picked, they were pinned onto 22 x 22 cm² nylon membranes using Q-bot. Each membrane contained 9,216 colonies or 36,864 colonies. These membranes were placed onto agar plate with appropriate antibiotic. The plates were incubated at 37°C for overnight.

After colonies were recovered on the second day, these filters were placed on filter paper prewetted with denaturing solution for four minutes, then were incubated on top of a boiling water bath for additional four minutes. The filters were then placed on filter paper prewetted with neutralizing solution for four minutes. After excess solution was removed by placing the filters on dry filter papers for one minute, the colony side of the filters were place into Proteinase K solution, incubated at 37°C for 40-50 minutes. The filters were placed on dry filter papers to dry overnight. DNA was then cross-linked to nylon membrane by UV light treatment.

Colony hybridization was conducted as described by Sambrook, J., Fritsch, E.F. and Maniatis, T., (in Molecular Cloning: A laboratory Manual, 2nd Edition). The following probes were used in colony hybridization:
1. First strand cDNA from the same tissue from which the library was made to remove the most redundant clones.
2. 48-192 most redundant cDNA clones from the same library based on previous sequencing data.
3. 192 most redundant cDNA clones in the entire corn sequence database.
4. A Sal-A20 oligo nucleotide: TCG ACC CAC GCG TCC GAA AAA AAA AAA AAA AAA AAA, removes clones containing a poly A tail but no cDNA.
5. cDNA clones derived from rRNA.

The image of the autoradiography was scanned into computer and the signal intensity and cold colony addresses of each colony was analyzed. Re-arraying of cold-colonies from 384 well plates to 96 well plates was conducted using Q-bot.

### Example 3

### Identification of Maize CHD EST's from a Computer Homology Search

Gene identities were determined by conducting BLAST (Basic Local Alignment Search Tool; Altschul, S. F., et al., (1993) J. Mol. Biol. 215:403-410; see also www.ncbi.nim.nih.gov/BLAST/) searches under default parameters for similarity to sequences contained in the BLAST "nr" database (comprising all non-redundant GenBank CDS translations, sequences derived from the 3-dimensional structure Brookhaven Protein Data Bank, the last major release of the SWISS-PROT protein sequence database, EMBL, and DDBJ databases). The cDNA sequences were analyzed for similarity to all publicly available DNA sequences contained in the "nr" database using the BLASTN algorithm. The DNA sequences were translated in all reading frames and compared for similarity to all publicly available protein sequences contained in the "nr" database using the BLASTX algorithm (Gish, W. and States, D. J. (1993) Nature Genetics 3:266-272) provided by the NCBI. In some cases, the sequencing data from two or more clones containing overlapping segments of DNA were used to construct contiguous DNA sequences.

### Example 4

### Transformation and Regeneration of Maize Callus

Expression vectors useful for modulating CHD expression are those that down-regulate CHD levels or activity (abbreviated hereafter as CHD-DR constructs). A CHD-DR construct is an expression cassette in which the transcribed RNA results in decreased levels of CHD protein in the cell. Examples would include expressing antisense, expressing an inverted-repeat sequence (which will form a hairpin) constructed from a portion of the CHD sequence, expressing the CHD sequence fused to another such "hairpin" forming sequence, or expressing CHD in a manner that will favor co-suppression of endogenous CHD.

Transformation of a CHD-DR construct (whether antisense, hairpin, or co-suppression-based) along with a marker-expression cassette (for example, UBI::moPAT-GPFm::pinII) into genotype Hi-II follows a well-established bombardment transformation protocol used for introducing DNA into the scutellum of immature maize embryos (Songstad, D.D. et al., In Vitro Cell Dev. Biol. Plant 32:179-183, 1996). It is noted that any suitable method of transformation can be used, such as *Agrobacterium*-mediated transformation and many other methods. To prepare suitable target tissue for transformation, ears are surface sterilized in 50% Chlorox bleach plus 0.5% Micro detergent for 20 minutes, and rinsed two times with sterile water. The immature embryos (approximately 1-1.5mm in length) are excised and placed embryo axis side down (scutellum side up), 25 embryos per plate. These are cultured onto medium containing N6 salts, Erikkson's vitamins, 0,69 g/l proline, 2 mg/l 2,4-D and 3% sucrose. After 4-5 days of incubation in the dark at 28°C, embryos are removed from the first medium and cultured onto similar medium containing 12% sucrose. Embryos are allowed to acclimate to this medium for 3 h prior to transformation. The scutellar surface of the immature embryos is targeted using particle bombardment. Embryos are transformed using the PDS-1000 Helium Gun from Bio-Rad at one shot per sample using 650PSI rupture disks. DNA delivered per shot averages approximately 0.1667µg. Following bombardment, all embryos are maintained on standard maize culture medium (N6 salts, Erikkson's vitamins, 0.69 g/l proline, 2 mg/l 2,4-D, 3% sucrose) for 2-3 days and then transferred to N6-based medium containing 3mg/L Bialaphos®. Plates are maintained at 28°C in the dark and are observed for colony recovery with transfers to fresh medium every two to three weeks. After approximately 10 weeks of selection, selection-resistant GFP positive callus clones were sampled for PCR and activity of the polynucleotide of interest. Positive lines were transferred to 288J medium, an MS-based medium with lower sucrose and hormone levels, to initiate plant regeneration. Following somatic embryo maturation (2-4 weeks), well-developed somatic embryos were transferred to medium for germination and transferred to the lighted culture room. Approximately 7-10 days later, developing plantlets were transferred to medium in tubes for 7-10 days until plantlets were well established. Plants were then transferred to inserts in flats (equivalent to 2.5" pot) containing potting soil and grown for 1 week in a growth chamber, subsequently grown an additional 1-2 weeks in the greenhouse, then transferred to Classic™ 600 pots (1.6 gallon) and grown to maturity. Plants are monitored for expression of the polynucleotide of interest. Recovered colonies and plants are scored based on GFP visual expression, leaf painting sensitivity to a 1% application of Ignite® herbicide, and molecular characterization via PCR and Southern analysis.

Transformation of a CHD-DR cassette along with UBI::moPAT∼moGFP::pinII into a maize genotype such as Hi-II (or inbreds such as Pioneer Hi-Bred International, Inc. proprietary inbreds N46 and P38) is also done using the *Agrobacterium* mediated DNA delivery method, as described by United States Patent #5,981,840 with the following modifications. Again, it is noted that any suitable method of transformation can be used, such as particle-mediated transformation, as well as many other methods. Agrobacteria are grown to log phase in liquid minimal-A medium containing 100µM spectinomycin. Embryos are immersed in a log phase suspension of Agrobacteria adjusted to obtain an effective concentration of 5 x 10⁸ cfu/ml. Embryos are infected for 5 minutes and then co-cultured on culture medium containing acetosyringone for 7 days at 20°C in the dark. After 7 days, the embryos are transferred to standard culture medium (MS salts with N6 macronutrients, 1 mg/L 2,4-D, 1 mg/L Dicamba, 20g/L sucrose, 0.6g/L glucose, 1 mg/L silver nitrate, and 100mg/L carbenicillin) with 3mg/L Bialaphos® as the selective agent. Plates are maintained at 28°C in the dark and are observed for colony recovery with transfers to fresh medium every two to three weeks. Positive lines are transferred to an MS-based medium with lower sucrose and hormone levels, to initiate plant regeneration. Following somatic embryo maturation (2-4 weeks), well-developed somatic embryos are transferred to medium for germination and transferred to the lighted culture room. Approximately 7-10 days later, developed plantlets are transferred to medium in tubes for 7-10 days until plantlets are well established. Plants are then transferred to inserts in flats (equivalent to 2.5" pot) containing potting soil and grown for 1 week in a growth chamber, subsequently grown an additional 1-2 weeks in the greenhouse, then transferred to Classic™ 600 pots (1.6 gallon) and grown to maturity. Recovered colonies and plants are scored based on GFP visual expression, leaf painting sensitivity to a 1% application of Ignite® herbicide, and molecular characterization via PCR and Southern analysis.

### A. Introducing CHD-DR to improve transformation frequency using Agrobacterium or particle bombardment.

Plasmids described in Example 4 are used to transform Hi-II immature embryos using particle delivery or the *Agrobacterium.* Bialaphos resistant GFP+ colonies are counted using a GFP microscope and transformation frequencies are determined (percentage of initial target embryos from which art least one GFP-expressing, bialaphos-resistant multicellular transformed event grows). In both particle gun experiments and Agrobacterium experiments, transformation frequencies are expected to increase with CHD treatment.

### B. Down-regulation of CHD to improve the embryogenic phenotype and regeneration capacity of inbreds.

Immature embryos from the inbred P38 are isolated, cultured and transformed as described above, with the following changes. Embryos are initially cultured on 601 H medium (a MS based medium with 0.1 mg/l zeatin, 2mgll 2,4-D, MS and SH vitamins, proline, silver nitrate, extra potassium nitrate, casein hydrolysate, gelrite, 10g/l glucose and 20g/l sucrose). Prior to bombardment embryos are moved to a high osmoticum medium (modified Duncan's with 2mg/l 2,4-D and 12% sucrose). Post bombardment, embryos are moved to 601 H medium with 3mg/l bialaphos for two weeks. Embryos are then moved to 601 H medium without proline and casein hydrolysate with 3mg/l bialaphos and transferred every two weeks. Transformation frequency is determined by counting the numbers of bialaphos-resistant GFP-positive colonies. Colonies are also scored on whether they have an embryogenic (regenerable) or non-embryogenic phenotype. Compared to the control treatment (UBI::moPAT∼moGFP::pinII alone), treatments including the marker cassette (UBI::moPAT∼moGFP::pinII) + CHD-DR is expected to result in consistently higher transformation frequencies, the transformants having a more embryogenic callus phenotype and the frequency of successful regeneration from transformed callus should be substantially improved.

### Example 5

### Transient Suppression of the CHD Polynucleotide Product to Induce Somatic Embryogenesis

It may be desirable to "kick start" somatic embryogenesis by transiently expressing a CHD-DR polynucleotide product. This can be done by delivering CHD-DR 5'capped polyadenylated RNA or expression cassettes containing CHD-DR DNA. These molecules can be delivered using a biolistics particle gun. For example 5' capped polyadenylated CHD-DR RNA can easily be made *in vitro* using Ambion's mMessage mMachine kit. Following the procedure outline above RNA is co-delivered along with DNA containing an agronomically useful expression cassette. The cells receiving the RNA will form somatic embryos and a large portion of these will have integrated the agronomic gene. Plants regenerated from these embryos can then be screened for the presence of the agronomic gene.

### Example 6

### Use of the maize CHD to induce apomixis

Maize expression cassettes down-regulating CHD expression in the inner integument or nucellus can easily be constructed. An expression cassette directing expression of the CHD-DR polynucleotide to the nucellus is made using the barley Nuc1 promoter. Embryos are co-bombarded with the selectable marker PAT fused to the GFP gene (UBI::moPAT∼moGFP) along with the nucellus specific CHD-DR expression cassette described above. Both inbred (P38) and GS3 transformants are obtained and regenerated as described in examples 4.

It is expected that the regenerated plants will then be capable of producing *de novo* embryos from CHD-DR expressing nucellar cells. This is complemented by pollinating the ears to promote normal central cell fertilization and endosperm development. In another variation of this scheme, nuc1:CHD-DR transformations could be done using a FIE-null genetic background which would promote both de novo embryo development and endosperm development without fertilization (see Ohad et al. 1999 The Plant Cell 11:407-415; also pending US application Serial No. 60/151575 filed August 31, 1999). Upon microscopic examination of the developing embryos it will be apparent that apomixis has occurred by the presence of embryos budding off the nucellus. In yet another variation of this scheme the CHD-DR polynucleotide could be delivered as described above into a homozygous zygotic-embryo-lethal genotype. Only the adventive embryos produced from somatic nucellus tissue would develop in the seed.

### EXAMPLE 7

### Expression of Chimeric Genes in Microbial Cells

The cDNAs encoding the instant transcription factors can be inserted into the T7 *E. coli* expression vector pBT430. This vector is a derivative of pET-3a (Rosenberg et al. (1987) Gene 56:125-135) which employs the bacteriophage T7 RNA polymerase/T7 promoter system. Plasmid pBT430 was constructed by first destroying the EcoR I and Hind III sites in pET-3a at their original positions. An oligonucleotide adaptor containing EcoR I and Hind III sites was inserted at the BamH I site of pET-3a. This created pET-3aM with additional unique cloning sites for insertion of genes into the expression vector. Then, the Nde I site at the position of translation initiation was converted to an Nco I site using oligonucleotide-directed mutagenesis. The DNA sequence of pET-3aM in this region, 5'-CATATGG, was converted to 5'-CCCATGG in pBT430.

Plasmid DNA containing a cDNA may be appropriately digested to release a nucleic acid fragment encoding the protein. This fragment may then be purified on a 1 % NuSieve GTG™ low melting agarose gel (FMC). Buffer and agarose contain 10 µg/ml ethidium bromide for visualization of the DNA fragment. The fragment can then be purified from the agarose gel by digestion with GELase™ (Epicentre Technologies) according to the manufacturer's instructions, ethanol precipitated, dried and resuspended in 20 µL of water. Appropriate oligonucleotide adapters may be ligated to the fragment using T4 DNA ligase (New England Biolabs, Beverly, MA). The fragment containing the ligated adapters can be purified from the excess adapters using low melting agarose as described above. The vector pBT430 is digested, dephosphorylated with alkaline phosphatase (NEB) and deproteinized with phenol/chloroform as described above. The prepared vector pBT430 and fragment can then be ligated at 16°C for 15 hours followed by transformation into DH5 electrocompetent cells (GIBCO BRL). Transformants can be selected on agar plates containing LB media and 100 µg/mL ampicillin. Transformants containing the polynucleotide encoding the transcription factor are then screened for the correct orientation with respect to the T7 promoter by restriction enzyme analysis.

For high level expression, a plasmid clone with the cDNA insert in the correct orientation relative to the T7 promoter can be transformed into *E. coli* strain BL21 (DE3) (Studier et al. (1986) J. Mol. Biol. 189:113-130). Cultures are grown in LB medium containing ampicillin (100 mg/L) at 25°C. At an optical density at 600 nm of approximately 1, IPTG (isopropylthio-β-galactoside, the inducer) can be added to a final concentration of 0.4 mM and incubation can be continued for 3 hours at 25°C. Cells are then harvested by centrifugation and re-suspended in 50 µL of 50 mM Tris-HCl at pH 8.0 containing 0.1 mM DTT and 0.2 mM phenyl methylsulfonyl fluoride. A small amount of 1 mm glass beads can be added and the mixture sonicated 3 times for about 5 seconds each time with a microprobe sonicator. The mixture is centrifuge and the protein concentration of the supernatant determined. One µg of protein from the soluble fraction of the culture can be separated by SDS-polyacrylamide gel electrophoresis. Gels can be observed for protein bands migrating at the expected molecular weight.

### EXAMPLE 8

### Evaluating Compounds for Their Ability to Inhibit the Activity of Plant Transcription Factors

The transcription factors described herein may be produced using any number of methods known to those skilled in the art. Such methods include, but are not limited to, expression in bacteria as described in Example 7, or expression in eukaryotic cell culture, *in planta,* and using viral expression systems in suitably infected organisms or cell lines. The instant transcription factors may be expressed either as mature forms of the proteins as observed *in vivo* or as fusion proteins by covalent attachment to a variety of enzymes, proteins or affinity tags. Common fusion protein partners include glutathione S-transferase ("GST"), thioredoxin ("Trx"), maltose binding protein, and C- and/or N-terminal hexahistidine polypeptide ("(His)₆"). The fusion proteins may be engineered with a protease recognition site at the fusion point so that fusion partners can be separated by protease digestion to yield intact mature enzyme. Examples of such proteases include thrombin, enterokinase and factor Xa. However, any protease can be used which specifically cleaves the peptide connecting the fusion protein and the enzyme.

Purification of the instant transcription factors, if desired, may utilize any number of separation technologies familiar to those skilled in the art of protein purification. Examples of such methods include, but are not limited to, homogenization, filtration, centrifugation, heat denaturation, ammonium sulfate precipitation, desalting, pH precipitation, ion exchange chromatography, hydrophobic interaction chromatography and affinity chromatography, wherein the affinity ligand represents a substrate, substrate analog or inhibitor. When the transcription factors are expressed as fusion proteins, the purification protocol may include the use of an affinity resin which is specific for the fusion protein tag attached to the expressed enzyme or an affinity resin containing ligands which are specific for the enzyme. For example, a transcription factor may be expressed as a fusion protein coupled to the C-terminus of thioredoxin. In addition, a (His)₆ peptide may be engineered into the N-terminus of the fused thioredoxin moiety to afford additional opportunities for affinity purification. Other suitable affinity resins could be synthesized by linking the appropriate ligands to any suitable resin such as Sepharose-4B. In an alternate embodiment, a thioredoxin fusion protein may be eluted using dithiothreitol; however, elution may be accomplished using other reagents which interact to displace the thioredoxin from the resin. These reagents include β-mercaptoethanol or other reduced thiol. The eluted fusion protein may be subjected to further purification by traditional means as stated above, if desired. Proteolytic cleavage of the thioredoxin fusion protein and the enzyme may be accomplished after the fusion protein is purified or while the protein is still bound to the ThioBond™ affinity resin or other resin.

Crude, partially purified or purified enzyme, either alone or as a fusion protein, may be utilized in assays for the evaluation of compounds for their ability to inhibit enzymatic activation of the transcription factors disclosed herein. Assays may be conducted under well-known experimental conditions that permit optimal enzymatic activity.

### Example 9

### CHD down-regulation to increase growth rates, which could be used as a screening criterion for positive selection of transformants

Using two promoters of increasing strength to drive expression of CHD-DR cassettes in maize, it appears that CHD-DR stimulates callus growth over control treatments and the stronger promoter driving CHD-DR results in faster growth than with the low-level promoter. For example, an experiment is performed to compare the ln2 and nos promoters. As noted above, based on our experience with these two promoters driving other genes, the In2 promoter (in the absence of an inducer other than auxin from the medium) would drive expression at very low levels. The nos promoter has been shown to drive moderately-low levels of transgene expression (approximately 10- to 30-fold lower than the maize ubiquitin promoter, but still stronger than ln2 under the culture conditions used in this experiment). One control treatment is used in this experiment, the UBI:PAT∼GFPmo:pinII construct by itself (with no CHD-DR). Hi-II immature embryos are bombarded as previously described, and transgenic, growing events are scored at 3 and 6 weeks. The control treatment results in a typical transformation frequency, for example of 0.8%. The ln2 and nos-driven CHD down-regulator treatments are expected to result in progressively higher transformation frequencies, for example 25 and 40%, respectively.

Within these treatments there is also expected an increase in the overall frequency of large, rapidly growing calli, relative to the control treatment. For this data, the fresh weight of transformed calli is recorded 2 months after bombardment. Assuming that all the transgenic events started as single transformed cells within a few days after bombardment, these weights represent the relative growth rate of these transformants during this period (all tissue is sub-cultured and weighed for each transformant; mean weights and standard deviations are calculated for each treatment). For the control treatment, the mean transformant weight after two months is expected to be 37 +/-15 mg (n=6). For the In2:CHD-down-regulator and nos:CHD-down-regulator treatments, the mean transformant weights are expected to be 126 +/-106 and 441 +/-430 mg, respectively. If the control treatment is set at a relative growth value of 1.0, this means that transformants in the ln2: CHD-down-regulator and nos: CHD-down-regulator treatments are expected to grow 3.4 and 12-fold faster than the control. Increasing CHD down regulation should result in a concomitant increase in callus growth rate.

### Example 10

### The use of CHD polynucleotide as a positive selection system for wheat transformation and for improving the regeneration capacity of wheat tissues

### Method

### Plant Material

Seeds of wheat Hybrinova lines NH535 and BO 014 are sown into soil in plug trays for vernalisation at 6°C for eight weeks. Vernalized seedlings are transferred in 8" pots and grown in a controlled environment room. The growth conditions used are; 1) soil composition: 75% L&P fine-grade peat, 12% screened sterilized loam, 10% 6 mm screened, lime-free grit, 3% medium grade vermiculite, 3.5 kg Osmocote per m³ soil (slow-release fertilizer, 15-11-13 NPK plus micronutrients), 0.5 kg PG mix per m³ (14-16-18 NPK granular fertilizer plus micronutrients, 2) 16 h photoperiod (400W sodium lamps providing irradiance of ca. 750 µE s⁻¹ m⁻²), 18 to 20°C day and 14 to 16°C night temperature, 50 to 70% relative air humidity and 3) pest control: sulfur spray every 4 to 6 weeks and biological control of thrips using *Amblyseius caliginosus* (Novartis BCM Ltd, UK).

### Isolation of Explants and Culture Initiation

Two sources of primary explants are used; scutellar and inflorescence tissues. For scutella, early-medium milk stage grains containing immature translucent embryos are harvested and surface-sterilized in 70% ethanol for 5 min. and 0.5% hypochlorite solution for 15-30 min. For inflorescences, tillers containing 0.5-1.0 cm inflorescences are harvested by cutting below the inflorescence-bearing node (the second node of a tiller). The tillers are trimmed to approximately 8-10 cm length and surface-sterilized as above with the upper end sealed with Nescofilm (Bando Chemical Ind. Ltd, Japan).

Under aseptic conditions, embryos of approximately 0.5-1.0 mm length are isolated and the embryo axis removed. Inflorescences are dissected from the tillers and cut into approximately 1 mm pieces. Thirty scutella or 1 mm inflorescence explants are placed in the center (18 mm target circle) of a 90 mm Petri dish containing MD0.5 or L7D2 culture medium. Embryos are placed with the embryo-axis side in contact with the medium exposing the scutellum to bombardment whereas inflorescence pieces are placed randomly. Cultures are incubated at 25±°C in darkness for approximately 24 h before bombardment. After bombardment, explants from each bombarded plate are spread across three plates for callus induction.

### Culture Media

The standard callus induction medium for scutellar tissues (MDO.5) consists of solidified (0.5% Agargel, Sigma A3301) modified MS medium supplemented with 9% sucrose, 10 mg l⁻¹ AgNO₃ and 0.5 mg l⁻¹ 2,4-D (Rasco-Gaunt *et al.,* 1999). Inflorescence tissues are cultured on L7D2 which consists of solidified (0.5% Agargel) L3 medium supplemented with 9% maltose and 2 mg l⁻¹ 2,4-D (Rasco-Gaunt and Barcelo, 1999). The basal shoot induction medium, RZ contains L salts, vitamins and inositol, 3% w/v maltose, 0.1 mg l⁻¹ 2,4-D and 5 mg l⁻¹ zeatin (Rasco-Gaunt and Barcelo, 1999). Regenerated plantlets are maintained in RO medium with the same composition as RZ, but without 2,4-D and zeatin.

### DNA Precipitation Procedure and Particle Bombardment

Submicron gold particles (0.6 µm Micron Gold, Bio-Rad) are coated with a plasmid containing a CHD-DR construct following the protocol modified from the original Bio-Rad procedure (Barcelo and Lazzeri, 1995). The standard precipitation mixture consists of 1 mg of gold particles in 50 µl SDW, 50 µl of 2.5 M calcium chloride, 20 µl of 100 mM spermidine free base and 5 µl DNA (concentration 1 µg µl⁻¹). After combining the components, the mixture is vortexed and the supernatant discarded. The particles are then washed with 150 µl absolute ethanol and finally resuspended in 85 µl absolute ethanol. The DNA/gold ethanol solution is kept on ice to minimize ethanol evaporation. For each bombardment, 5 µl of DNA/gold ethanol solution (ca. 60 µg gold) is loaded onto the macrocarrier.

Particle bombardments are carried out using DuPont PDS 1000/He gun with a target distance of 5.5 cm from the stopping plate at 650 psi acceleration pressure and 28 in. Hg chamber vacuum pressure.

### Regeneration of Transformants

For callus induction, bombarded explants are distributed over the surface of the medium in the original dish and two other dishes and cultured at 25±1°C in darkness for three weeks. Development of somatic embryos from each callus are periodically recorded. For shoot induction, calluses are transferred to RZ medium and cultured under 12 h light (250 µE s⁻¹m⁻², from cool white fluorescent tubes) at 25±1°C for three weeks for two rounds. All plants regenerating from the same callus are noted. Plants growing more vigorously than the control cultures are potted in soil after 6-9 weeks in R0 medium. The plantlets are acclimatized in a propagator for 1-2 weeks. Thereafter, the plants are grown to maturity under growth conditions described above.

### DNA Isolation from Callus and Leaf Tissues

Genomic DNA as extracted from calluses or leaves using a modification of the CTAB (cetyltriethylammonium bromide, Sigma H5882) method described by Stacey and Isaac cite (1994). Approximately 100-200 mg of frozen tissues is ground into powder in liquid nitrogen and homogenized in 1 ml of CTAB extraction buffer (2% CTAB, 0.02 M EDTA, 0.1 M Tris-Cl pH 8, 1.4 M NaCl, 25 mM DTT) for 30 min at 65°C. Homogenized samples are allowed to cool at room temperature for 15 min before a single protein extraction with approximately 1 ml 24:1 v/v chloroform:octanol is done. Samples are centrifuged for 7 min at 13,000 rpm and the upper layer of supernatant collected using wide-mouthed pipette tips. DNA is precipitated from the supernatant by incubation in 95% ethanol on ice for 1 h. DNA threads are spooled onto a glass hook, washed in 75% ethanol containing 0.2 M sodium acetate for 10 min, air-dried for 5 min and resuspended in TE buffer. Five µl RNAse A is added to the samples and incubated at 37°C for 1 h.

For quantification of genomic DNA, gel electrophoresis is performed using an 0.8% agarose gel in 1x TBE buffer. One microliter of the samples are fractionated alongside 200, 400, 600 and 800 ng µl⁻¹ λ uncut DNA markers.

### Polymerase chain reaction (PCR) analysis

The presence of the maize CHD-DR polynucleotide is analyzed by PCR using 100-200 ng template DNA in a 30 ml PCR reaction mixture containing 1X concentration enzyme buffer (10 mM Tris-HCl pH 8.8, 1.5 mM magnesium chloride, 50 mM potassium chloride, 0.1% Triton X-100), 200 µM dNTPs, 0.3 µM primers and 0.022 U TaqDNA polymerase (Boehringer Mannheim). Thermocycling conditions are as follows (30 cycles): denaturation at 95°C for 30 s, annealing at 55°C for 1 min and extension at 72°C for 1 min. After particle-mediated delivery of either a UBI::PAT∼GFPmo::pinII construct alone (control treatment), or the UBI::PAT∼GFPmo::pinII + CHD-DR, in the treatments containing the CDH-DR construct there should be a higher frequency of embryogenic transformants recovered and the regeneration capacity of these transformants should be substantially improved over the control treatment. In addition, in the CDH-DR treatment the frequency of escape colonies should be reduced.

### EXAMPLE 11

### Expression of Chimeric Genes in Dicot Cells

The CHD-DR polynucleotide can also be used to improve the transformation of soybean. To demonstrate this the construct consisting of the ln2 promoter and CHD-DR sequence are introduced into embryogenic suspension cultures of soybean by particle bombardment using essentially the methods described in Parrott, W.A., L.M. Hoffman, D.F. Hildebrand, E.G. Williams, and G.B. Collins, (1989) Recovery of primary transformants of soybean, Plant Cell Rep. 7:615-617. This method with modifications is described below.

Seed is removed from pods when the cotyledons are between 3 and 5 mm in length. The seeds are sterilized in a Chlorox solution (0.5%) for 15 minutes after which time the seeds are rinsed with sterile distilled water. The immature cotyledons are excised by first excising the portion of the seed that contains the embryo axis. The cotyledons are then removed from the seed coat by gently pushing the distal end of the seed with the blunt end of the scalpel blade. The cotyledons are then placed (flat side up) SB1 initiation medium (MS salts, B5 vitamins, 20 mg/L 2,4-D, 31.5 g/l sucrose, 8 g/L TC Agar, pH 5.8). The Petri plates are incubated in the light (16 hr day; 75-80 µE) at 26° C. After 4 weeks of incubation the cotyledons are transferred to fresh SB1 medium. After an additional two weeks, globular stage somatic embryos that exhibit proliferative areas are excised and transferred to FN Lite liquid medium (Samoylov, V.M., D.M. Tucker, and W.A. Parrott (1998) Soybean [Glycine max (L.) Merrill] embryogenic cultures: the role of sucrose and total nitrogen content on proliferation. In Vitro Cell Dev. Biol.- Plant 34:8-13). About 10 to 12 small clusters of somatic embryos are placed in 250 ml flasks containing 35 ml of SB172 medium. The soybean embryogenic suspension cultures are maintained in 35 mL liquid media on a rotary shaker, 150 rpm, at 26°C with florescent lights (20 µE) on a 16:8 hour day/night schedule. Cultures are sub-cultured every two weeks by inoculating approximately 35 mg of tissue into 35 mL of liquid medium.

Soybean embryogenic suspension cultures are then transformed using particle gun bombardment (Klein et al. (1987) Nature (London) 327:70, U.S. Patent No. 4,945,050). A BioRad Biolistic™ PDS1000/HE instrument is used for these transformations. A selectable marker gene which is used to facilitate soybean transformation is a chimeric gene composed of the 35S promoter from Cauliflower Mosaic Virus (Odell et al. (1985) Nature 313:810-812), the hygromycin phosphotransferase gene from plasmid pJR225 (from *E. coli*; Gritz et al. (1983) Gene 25:179-188) and the 3' region of the nopaline synthase gene from the T-DNA of the Ti plasmid of *Agrobacterium tumefaciens.*

To 50 µL of a 60 mg/mL 1 µm gold particle suspension is added (in order): 5 µL DNA (1 µg/µL), 20 µl spermidine (0.1 M), and 50 µL CaCl₂ (2.5 M). The particle preparation is agitated for three minutes, spun in a microfuge for 10 seconds and the supernatant removed. The DNA-coated particles are washed once in 400 µL 70% ethanol and resuspended in 40 µL of anhydrous ethanol. The DNA/particle suspension is sonicated three times for one second each. Five µL of the DNA-coated gold particles are then loaded on each macro carrier disk.

Approximately 300-400 mg of a two-week-old suspension culture is placed in an empty 60x15 mm petri dish and the residual liquid removed from the tissue with a pipette. Membrane rupture pressure is set at 1100 psi and the chamber is evacuated to a vacuum of 28 inches mercury. The tissue is placed approximately 8 cm away from the retaining screen, and is bombarded three times. Following bombardment, the tissue is divided in half and placed back into 35 ml of FN Lite medium.

Five to seven days after bombardment, the liquid medium is exchanged with fresh medium. Eleven days post bombardment the medium is exchanged with fresh medium containing 50 mg/mL hygromycin. This selective medium is refreshed weekly. Seven to eight weeks post bombardment, green, transformed tissue is observed growing from untransformed, necrotic embryogenic clusters. Isolated green tissue is removed and inoculated into individual flasks to generate new, clonally propagated, transformed embryogenic suspension cultures. Each new line is treated as an independent transformation event. These suspensions are then subcultured and maintained as clusters of immature embryos, or tissue is regenerated into whole plants by maturation and germination of individual embryos.

Two different genotypes are used in these experiments: 92B91 and 93B82. Samples of tissue are either bombarded with the hygromycin resistance gene alone or with a 1:1 mixture of the hygromycin resistance gene and the CHD-DR construct. Embryogenic cultures generated from 92B91 generally produce transformation events while cultures from 93B82 are much more difficult to transform. For both genotypes, the CHD-DR construct resulted in increased transformation frequencies.

### Example 12

### Use of antibodies raised against CHD to transiently stimulate embryogenesis and enhance transformation

Antibodies directed against CHD can also be used to mitigate CHD's activity, thus stimulating somatic embryogenic growth. Genes encoding single chain antibodies expressed behind a suitable promoter, for example the ubiquitin promoter, could be used in such a fashion. Transient expression of an anti-CHD antibody could temporarily disrupt normal CHD function and thus stimulate somatic embryogenic growth. Alternatively, antibodies raised against CHD could be purified and used for direct introduction into maize cells. The antibody is introduced into maize cells using physical methods such as microinjection, bombardment, electroporation or silica fiber methods.

Alternatively, single chain anti-CHD is delivered from *Agrobacterium tumefaciens* into plant cells in the form of fusions to *Agrobacterium* virulence proteins (see co-pending applications US Serial Nos. 09/316,914 filed May 19, 1999 and 09/570,319 filed May 12, 2000). Fusions are constructed between the anti-CHD single chain antibody and bacterial virulence proteins such as VirE2, VirD2, or VirF which are known to be delivered directly into plant cells. Fusion's are constructed to retain both those properties of bacterial virulence proteins required to mediate delivery into plant cells and the anti-CHD activity required for stimulating somatic embryogenic growth and enhancing transformation. This method ensures a high frequency of simultaneous co-delivery of T-DNA and functional anti-CHD protein into the same host cell. Direct delivery of anti-CHD antibodies using physical methods such as particle bombardment can also be used to inhibit CHD activity and transiently stimulate somatic embryogenic growth.

### Example 13

### Use dominant-negative mutagenized CHD gene to transiently stimulate embryogenesis and enhance transformation

Using directed mutagenesis to disrupt critical functional domains within the CHD gene will create a dominant negative mutant. For example, single amino-acid changes in the helicase/ATPase motifs IV and VI will abolish ATPase function in this protein. In *Arabidopsis,* the nucleotide sequence can be modified to encode an altered amino-acid sequence, either changing LLRRVKK to LLRKVKK or changing AMARAHR to AMAKAHR. In either amino-acid stretch, changing the central arginine to a lysine or alanine residue completely destroys ATPase function in this protein. These sequences tend to be highly conserved, so altering the maize gene (or any other plant CHD gene) should have a similar effect. When such an altered CHD gene is over-expressed in the plant cell, it acts as a dominant-negative resulting in a reduction of endogenous CHD activity (and in some cases can result in essentially down-regulating CHD to the point where there is no activity).

Deletion or domain swapping techniques can also be employed to create a dominant negative mutant. For example, one of the transcriptional repression activities of CHD is achieved through deacetylation of histones. In mammalian system, CHD3/CHD4 binds to histone deacetylase through zinc-finger motif that is present in the N-terminal of the protein. Deletion of the zinc-finger motif, i.e. CQACGESTNLVSCNTCTYAFHAKCL of Arabidopsis CHD3, in this protein will change the accessibility to the histones and result in a reduction of the nucleosome remodeling activity of this protein and lead to a release of the transgenic cell from transcriptional repression.

Transient overexpression of such a dominant-negative CHD construct will result in depressed CHD activity in the transiently expressing plant cells. Genes and/or pathways normally suppressed by CHD will be transiently activated. Such a stimulation in cells receiving the foreign DNA will result in increased growth, and in species such as corn in which growth of transgenic cell clusters relative to wild-type (non-transformed) cells can be limiting, this growth stimulation will translate into increased recovery of transformants (i.e. increased transformation frequency).

### Example 14

### Increase of oil content in crop seed by co-suppression of CHD gene

Expression cassettes suppressing CHD expression in seeds can easily be constructed. For example, maize oleosin promoter, or gamma-zein promoter can be used to co-suppress CHD in seed only. Transgenic seeds can be obtained by either *Agrobacteria* transformation or particle gun methods as discussed above. Repression of CHD expression in seed will lead to expression of many embryonic genes and change the cell differentiation. This may increase oil accumulation in endosperm or increase embryo size. Oil content in embryo and endosperm can be determined easily by hexane extraction.

## Claims

1. An isolated nucleic acid encoding a protein having CHD (chromatin organization modifier-helicase-DNA binding) activity, and comprising:
(a) a polynucleotide sequence which encodes a polypeptide of SEQ ID NO: 2;
(b) a polynucleotide sequence having as set forth in SEQ ID. NO: 1; or
(c) a polynucleotide sequence having at least 90% sequence identity to SEQ ID NO: 1, wherein the % sequence identity is based on the entire sequence of SEQ.ID NO: 1 and is determined by GAP 10 analysis using default parameters.

2. The isolated nucleic acid of claim 1, wherein the polynucleotide is from a monocot or dicot.

3. The isolated nucleic acid of claim 1 wherein said percentage sequence identity is at least 95%.

4. A vector comprising at least one nucleic acid of any one of the preceding claims.

5. An expression cassette comprising at least one nucleic acid of any one of claims 1 to 3 operably linked to a promoter, wherein the nucleic acid is in sense orientation.

6. A host cell containing at least one expression cassette, vector or nucleic acid of any one of the preceding claims.

7. The host cell of claim 3 that is a plant cell.

8. A transgenic plant comprising at least one expression cassette of claim 5.

9. The transgenic plant of claim 8, wherein the plant is corn, soybean, sorghum, wheat, rice, alfalfa, sunflower, canola, cotton, or turf grass.

10. A seed from the transgenic plant of claim 8 or 9 which comprises said expression cassette of claim 5.

11. An isolated protein having CHD (chromatin organization modifier-helicase-DNA binding) activity, and comprising:
(a) a polypeptide having the sequence set forth in SEQ ID NO: 2;
(b) a polypeptide sequence having at least 90% sequence identity to SEQ ID NO: 2, wherein the % sequence identity is based on the entire sequence of SEQ ID NO: 2 and is determined by GAP 10 analysis using default parameters; or
(c) a polypeptide encoded by a nucleic acid of claim 1.

12. A protein of claim 11 wherein said percentage identity is at least 95%.

13. An isolated ribonucleic acid sequence encoding a protein of claim 11.

14. A method for modulating CHD (chromatin organization modifier-helicase-DNA binding) activity in a host cell, comprising:
(a) transforming a host cell with at least one expression cassette of claim 5; and
(b) growing the transformed host cell.

15. A method for transiently modulating the level of CHD (chromatin organization modifier-helicase-DNA binding) activity in host cells comprising introducing at least one CHD nucleic acid of claim 1 to produce a transformed cell and growing the transformed host cell.

16. Use of
a CHD (chromatin organization modifier-helicase-DNA binding) - silencing construct as an expression construct whose transcribed mRNA or translated protein acts to diminish the functional expression of active CHD,
or of
an antibody directed against a critical functional domain within a CHD molecule
to reduce activity of endogenous CHD protein in a plant or plant cell;
wherein the CHD whose expression or activity is reduced or diminished is a CHD as defined in claim 11 or 12.

17. Use according to claim 16 wherein said diminishment is achieved through the expression of an antisense construct targeted against the CHD structural gene; through, a vector in which the CHD structural gene or a portion thereof is used to make a silencing hairpin; or where a CHD-overexpression cassette comprising a nucleic acid of any one of claims 1 or 3 is used to co-suppress endogenous CHD levels.

18. Use according to claim 16 or 17 to modulate, optionally transiently, CHD activity in a host plant cell.

19. Use according to claim 16 or 17 to enhance tissue culture response.

20. Use according to claim 16 or 17 to induce somatic embryogenesis.

21. Use according to claim 16 or 17 for positive selection of a transformed plant cell.

22. Use according to claim 21, comprising introducing a CHD-silencing construct as defined in claim 16 or 17 into said cell, wherein said CHD-silencing construct comprises a promoter capable of driving expression in a plant cell, to produce a transformed cell, growing the transformed cell to produce a transformed embryo, and selecting for the transformed embryo.

23. Use according to claim 21 or 22 further comprising introducing a gene of interest into the transformed cell.

24. Use according to claim 21, 22 or 23 further comprising altering media components to favor the growth of transformed cells.

25. Use according to claim 24 wherein the media components are altered to reduce somatic embryogenesis in non-transformed cells.

26. Use according to claim 22, 23, 24 or 25 wherein said CHD-silencing construct is excised.

27. Use according to claim 26 wherein said polynucleotide is flanked by FRT sequences to allow FLP-mediated excision of the polynucleotide.

28. Use according to claim 16 or 17 to induce apomixis in a plant cell.

29. Use according to claim 28, comprising introducing into a responsive plant cell a CHD-silencing construct as defined in claim 16 or 17 wherein said construct optionally comprises an inducible promoter, to produce a transformed plant cell and growing the transformed plant cell to produce a transformed somatic embryo.

30. Use according to claim 28 or 29 further comprising suppressing the expression of an FIE polycomb polynucleotide in the plant cell using sense or antisense methods.

31. Use according to claim 28, 29 or 30 further comprising growing the embryo to produce a regenerated plant.

32. Use according to claim 29, 30 or 31 wherein said CHD-silencing construct is expressed in integument or nucellus tissue.

33. Use according to claim 16 or 17 for increasing transformation efficiency, comprising introducing a CHD-silencing construct and a gene of interest into a responsive host cell.

34. Use according to claim 16 or 17 for increasing recovery of regenerated plants, comprising introducing a CHD-silencing construct as defined in claim 16 or 17 into a responsive host cell and growing said cell to produce a regenerated plant.

35. Use according to claim 16 or 17 for decreasing gene silencing comprising stably transforming a CHD-silencing construct as defined in claim 16 or 17 and a gene of interest into a host cell and growing the transformed host cell.

36. Use according to claim 16 or 17 for increasing oil production in a host cell, comprising stably transforming a host cell with a CHD-silencing construct as defined in claim 16 or 17 and growing the transformed cell to produce elevated levels of oil compared to a corresponding non-transformed cell.

37. Use according to any one of claims 17 to 36 wherein said plant cell is from a monocot or a dicot.

38. Use according to claim 37 wherein said monocot or dicot is corn, soybean, sorghum, wheat, rice, alfalfa, sunflower, canola, cotton, or turf grass.

39. A plant in which functional expression of a CHD (chromatin organization modifier-helicase-DNA binding) is diminished through a CHD-silencing construct, or in which an antibody directed against a critical functional domain of CHD reduces activity of endogenous CHD protein; wherein the CHD whose expression or activity is reduced or diminished is a CHD as defined in any one of claims 11 or 12.

40. A plant according to claim 39 wherein said diminishment or reduction is achieved in a manner defined in claim 17.

41. A plant according claim 39 or 40 which is a monocot or a dicot.

42. A plant according any one of claims 40 to 42 wherein said monocot or dicot is corn, soybean, sorghum, wheat, rice, alfalfa, sunflower, canola, cotton, or turf grass.

## Patentansprüche

1. Isolierte Nucleinsäure, codierend ein Protein mit CHD (chromatin organization modifier-helicase-DNA binding)-Aktivität und umfassend:
(a) eine Polynucleotidsequenz, die ein Polypeptid von SEQ ID NO: 2 codiert;
(b) eine Polynucleotidsequenz, wie sie in SEQ ID NO: 1 angegeben ist; oder
(c) eine Polynucleotidsequenz, die wenigstens 90% Sequenzidentität zu SEQ ID NO: 1 hat, wobei die % Sequenzidentität auf der gesamten Sequenz von SEQ ID NO: 1 basiert und durch GAP 10-Analyse unter Verwendung von Default-Parametern bestimmt wird.

2. Isolierte Nucleinsäure gemäß Anspruch 1, wobei das Polynucleotid aus einer Monokotyle oder Dikotyle ist.

3. Nucleinsäure gemäß Anspruch 1, wobei der Prozentwert der Sequenzidentität wenigstens 95% ist.

4. Vektor, der wenigstens eine Nucleinsäure gemäß einem der vorangehenden Ansprüche umfasst.

5. Expressionskassette, umfassend wenigstens eine Nucleinsäure gemäß einem der Ansprüche 1 bis 3, funktionell verknüpft mit einem Promotor, wobei die Nucleinsäure in Sense-Orientierung ist.

6. Wirtszelle, die wenigstens eine Expressionskassette, einen Vektor oder eine Nucleinsäure gemäß einem der vorangehenden Ansprüche enthält.

7. Wirtszelle gemäß Anspruch 3, die eine Pflanzenzelle ist.

8. Transgene Pflanze, umfassend wenigstens eine Expressionskassette gemäß Anspruch 5.

9. Transgene Pflanze gemäß Anspruch 8, wobei die Pflanze Mais, Sojabohne, Sorghum, Weizen, Reis, Alfalfa, Sonnenblume, Canola bzw. Raps, Baumwolle oder Rasengras ist.

10. Samen aus der transgenen Pflanze gemäß Anspruch 8 oder 9, der die Expressionskassette gemäß Anspruch 5 umfasst.

11. Isoliertes Protein, das CHD (chromatin organization modifier-helicase-DNA binding)-Aktivität hat und umfasst:
(a) ein Polypeptid mit der in SEQ ID NO: 2 angegebenen Sequenz;
(b) eine Polypeptidsequenz, die wenigstens 90% Sequenzidentität zu SEQ ID NO: 2 hat, wobei die % Sequenzidentität auf der gesamten Sequenz von SEQ ID NO: 2 basiert und durch GAP 10-Analyse unter Verwendung von Default-Parametern bestimmt wird; oder
(c) ein Polypeptid, das durch eine Nucleinsäure gemäß Anspruch 1 codiert wird.

12. Protein gemäß Anspruch 11, wobei der Prozentwert der Identität wenigstens 95% ist.

13. Isolierte Ribonucleinsäuresequenz, die ein Protein gemäß Anspruch 11 codiert.

14. Verfahren zur Modulierung der CHD (chromatin organization modifier-helicase-DNA binding)-Aktivität in einer Wirtszelle, umfassend:
(a) Transformieren einer Wirtszelle mit wenigstens einer Expressionskassette gemäß Anspruch 5 und
(b) Wachsenlassen der transformierten Wirtszelle.

15. Verfahren zur zeitweiligen Modulierung des Levels der CHD (chromatin organization modifier-helicase-DNA binding)-Aktivität in Wirtszellen, umfassend ein Einführen wenigstens einer CHD-Nucleinsäure gemäß Anspruch 1 unter Erzeugung einer transformierten Zelle und Wachsenlassen der transformierten Wirtszelle.

16. Verwendung eines
CHD (chromatin organization modifier-helicase-DNA binding)-Silencing-Konstrukts als Expressionskonstrukt, dessen transskribierte mRNA oder translatiertes Protein unter Verringerung der funktionellen Expression von aktivem CHD wirkt, oder eines Antikörpers, der gegen eine kritische funktionelle Domäne innerhalb eines CHD-Moleküls gerichtet ist,
um die Aktivität von endogenem CHD-Protein in einer Pflanze oder einer Pflanzenzelle zu reduzieren;
wobei das CHD, dessen Expression oder Aktivität reduziert oder verringert wird, ein CHD ist, wie es in Anspruch 11 oder 12 definiert ist.

17. Verwendung gemäß Anspruch 16, wobei die Verringerung durch die Expression eines Antisense-Konstrukts, das gegen das CHD-Strukturgen gerichtet ist; durch einen Vektor, in dem das CHD-Strukturgen oder ein Teil davon verwendet wird, um eine Silencing-Haarnadel herzustellen, erreicht wird oder wobei eine CHD-Überexpressionskassette, die eine Nucleinsäure gemäß einem der Ansprüche 1 oder 3 umfasst, verwendet wird, um endogene CHD-Level einer Cosuppression zu unterwerfen.

18. Verwendung gemäß Anspruch 16 oder 17, um CHD-Aktivität in einer Wirtspflanzenzelle, gegebenenfalls vorübergehend, zu modulieren.

19. Verwendung gemäß Anspruch 16 oder 17, um eine Gewebekulturantwort zu verstärken.

20. Verwendung gemäß Anspruch 16 oder 17, um eine somatische Embryogenese zu induzieren.

21. Verwendung gemäß Anspruch 16 oder 17 zur positiven Selektion einer transformierten Pflanzenzelle.

22. Verwendung gemäß Anspruch 21, umfassend Einführen eines CHD-Silencing-Konstrukts, wie es in Anspruch 16 oder 17 definiert ist, in die Zelle, wobei das CHD-Silencing-Konstrukt einen Promotor umfasst, der fähig ist, eine Expression in einer Pflanzenzelle zu steuern, um eine transformierte Zelle zu produzieren, Wachsenlassen der transformierten Zelle unter Herstellung eines transformierten Embryos und Selektieren auf den transformierten Embryo.

23. Verwendung gemäß Anspruch 21 oder 22, außerdem umfassend Einführen eines Gens von Interesse in die transformierte Zelle.

24. Verwendung gemäß Anspruch 21, 22 oder 23, außerdem umfassend Ändern der Mediumkomponenten, um das Wachstum von transformierten Zellen zu begünstigen.

25. Verwendung gemäß Anspruch 24, wobei die Mediumkomponenten zur Verringerung der somatischen Embryogenese in nicht-transformierten Zellen geändert werden.

26. Verwendung gemäß Anspruch 22, 23, 24 oder 25, wobei das CHD-Silencing-Konstrukt ausgeschnitten wird.

27. Verwendung gemäß Anspruch 26, wobei das Polynucleotid von FRT-Sequenzen flankiert wird, um eine FLP-vermittelte Exzision des Polynucleotids zu erlauben.

28. Verwendung gemäß Anspruch 16 oder 17, um Apomixis in einer Pflanzenzelle zu induzieren.

29. Verwendung gemäß Anspruch 28, umfassend Einführen eines CHD-Silencing-Konstrukts, wie es in Anspruch 16 oder 17 definiert ist, in eine reaktionsfähige Pflanzenzelle, wobei das Konstrukt gegebenenfalls einen induzierbaren Promotor umfasst, um eine transformierte Pflanzenzelle zu produzieren, und Wachsenlassen der transformierten Pflanzenzelle, um einen transformierten somatischen Embryo zu produzieren.

30. Verwendung gemäß Anspruch 28 oder 29, ferner umfassend Supprimieren der Expression eines FIE-Polycomb-Polynucleotids in der Pflanzenzelle unter Verwendung von Sense- oder Anti-Sense-Verfahren.

31. Verwendung gemäß Anspruch 28, 29 oder 30, außerdem umfassend Wachsenlassen des Embryos, um eine regenerierte Pflanze zu produzieren.

32. Verwendung gemäß Anspruch 29, 30 oder 31, wobei das CHD-Silencing-Konstrukt in Integument- oder Kerngewebe exprimiert wird.

33. Verwendung gemäß Anspruch 16 oder 17 zur Erhöhung der Transformationseffizienz, umfassend Einführen eines CHD-Silencing-Konstrukts und eines Gens von Interesse in eine reaktionsfähige Wirtszelle.

34. Verwendung gemäß Anspruch 16 oder 17 zur Erhöhung der Gewinnung von regenerierten Pflanzen, umfassend Einführen eines CHD-Silencing-Konstrukts, wie es in Anspruch 16 oder 17 definiert ist, in eine reaktionsfähige Wirtszelle und Wachsenlassen der Zelle, um eine regenerierte Pflanze zu produzieren.

35. Verwendung gemäß Anspruch 16 oder 17 zur Verringerung des Gen-Silencings, umfassend stabiles Transformieren eines CHD-Silencing-Konstrukts, wie es in Anspruch 16 oder 17 definiert ist, und eines Gens von Interesse in eine Wirtszelle und Wachsenlassen der transformierten Wirtszelle.

36. Verwendung gemäß Anspruch 16 oder 17 zur Erhöhung der Ölproduktion in einer Wirtszelle, umfassend stabiles Transformieren einer Wirtszelle mit einem CHD-Silencing-Konstrukt, wie es in Anspruch 16 oder 17 definiert ist, und Wachsenlassen der transformierten Zelle, um erhöhte Öllevel, verglichen mit einer entsprechenden nicht-transformierten Zelle, zu produzieren.

37. Verwendung gemäß einem der Ansprüche 17 bis 36, wobei die Pflanzenzelle aus einer Monokotyle oder einer Dikotyle stammt.

38. Verwendung gemäß Anspruch 37, wobei die Monokotyle oder Dikotyle Mais, Sojabohne, Sorghum, Weizen, Reis, Alfalfa, Sonnenblume, Canola bzw. Raps, Baumwolle oder Rasengras ist.

39. Pflanze, in der eine funktionelle Expression eines CHD (chromatin organization modifier-helicase-DNA binding) durch ein CHD-Silencing-Konstrukt vermindert ist oder in der ein Antikörper, der gegen eine kritische funktionelle Domäne von CHD gerichtet ist, die Aktivität von endogenem CHD-Protein reduziert, wobei das CHD, dessen Expression oder Aktivität reduziert oder vermindert ist, ein CHD ist, wie es in einem der Ansprüche 11 oder 12 definiert ist.

40. Pflanze gemäß Anspruch 39, wobei die Verminderung oder Reduktion in einer Art, wie sie in Anspruch 17 definiert ist, erreicht wird.

41. Pflanze gemäß Anspruch 39 oder 40, die eine Monokotyle oder eine Dikotyle ist.

42. Pflanze gemäß einem der Ansprüche 40 bis 42, wobei die Monokotyle oder Dikotyle Mais, Sojabohne, Sorghum, Weizen, Reis, Alfalfa, Sonnenblume, Canola bzw. Raps, Baumwolle oder Rasengras ist.

## Revendications

1. Acide nucléique isolé codant pour une protéine ayant une activité CHD (hélicase se liant à l'ADN modificatrice de l'organisation de la chromatine) et comprenant :
a) une séquence polynucléotidique qui code pour un polypeptide de SEQ ID NO : 2 ;
b) une séquence polynucléotidique telle que présentée en SEQ ID NO : 1 ou
c) une séquence polynucléotidique ayant au moins 90 % d'identité de séquence avec SEQ ID NO : 1, dans laquelle le % d'identité de séquence est basé sur la séquence entière de SEQ ID NO : 1 et est déterminé par analyse de GAP 10 en utilisant les paramètres par défaut.

2. Acide nucléique isolé de la revendication 1, dans lequel le polynucléotide provient d'une monocotylédone ou d'une dicotylédone.

3. Acide nucléique isolé de la revendication 1, dans lequel ledit pourcentage d'identité de séquence est d'au moins 95 %.

4. Vecteur comprenant au moins un acide nucléique de l'une quelconque des revendications précédentes.

5. Cassette d'expression comprenant au moins un acide nucléique de l'une quelconque des revendications 1 à 3 liée de manière fonctionnelle à un promoteur, dans laquelle l'acide nucléique est dans l'orientation sens.

6. Cellule hôte contenant au moins une cassette d'expression, un vecteur ou un acide nucléique de l'une quelconque des revendications précédentes.

7. Cellule hôte de la revendication 3 qui est une cellule de plante.

8. Plante transgénique comprenant au moins une cassette d'expression de la revendication 5.

9. Plante transgénique de la revendication 8, dans laquelle la plante est le maïs, soja, sorgho, blé, riz, luzerne, tournesol, colza, coton, ou graminée gazonnante.

10. Graine de la plante transgénique de la revendication 8 ou 9 qui comprend ladite cassette d'expression de la revendication 5.

11. Protéine isolée ayant une activité CHD (hélicase se liant à l'ADN modificatrice de l'organisation de la chromatine), et comprenant :
a) un polypeptide ayant la séquence présentée en SEQ ID NO : 2 ;
b) une séquence polypeptidique ayant au moins 90 % d'identité de séquence avec SEQ ID NO :2, dans laquelle le % d'identité de séquence est basé sur la séquence entière de SEQ ID NO :2 et est déterminé par une analyse de GAP 10 en utilisant les paramètres par défaut ; ou
c) un polypeptide codé par un acide nucléique de la revendication 1.

12. Protéine de la revendication 11, dans laquelle ledit pourcentage d'identité est d'au moins 95 %.

13. Séquence d'acide ribonucléique isolé codant pour une protéine de la revendication 11.

14. Méthode pour moduler une activité CHD (hélicase se liant à l'ADN modificatrice de l'organisation de la chromatine) dans une cellule hôte, comprenant :
a) la transformation d'une cellule hôte avec au moins une cassette d'expression de la revendication 5 ; et
b) la culture de la cellule hôte transformée.

15. Méthode pour moduler de manière transitoire le niveau d'une activité CHD (hélicase se liant à l'ADN modificatrice de l'organisation de la chromatine) dans des cellules hôtes, comprenant l'introduction d'au moins un acide nucléique de CHD de la revendication 1 pour produire une cellule transformée et la culture de la cellule hôte transformée.

16. Utilisation
d'une construction inactivant CHD (hélicase se liant à l'ADN modificatrice de l'organisation de la chromatine) en tant que construction d'expression dont l'ARNm transcrit ou la protéine traduite agit en diminuant l'expression fonctionnelle de CHD active,
ou
d'un anticorps dirigé contre un domaine fonctionnel critique dans une molécule de CHD
pour réduire l'activité de la protéine CHD endogène dans une plante ou une cellule de plante ;
dans laquelle la CHD dont l'expression ou l'activité est réduite ou diminuée est une CHD telle que définie dans la revendication 11 ou 12.

17. Utilisation selon la revendication 16 dans laquelle ladite diminution est obtenue par l'expression d'une construction antisens dirigée de manière ciblée contre le gène de structure de CHD ; par un vecteur dans lequel le gène de structure de CHD ou une portion de celui-ci est utilisé pour faire une épingle à cheveux inactivante ; ou dans laquelle une cassette de surexpression de CHD comprenant un acide nucléique de l'une quelconque des revendications 1 ou 3 est utilisée pour co-supprimer les nivaux endogènes de CHD.

18. Utilisation selon la revendication 16 ou 17 pour moduler, éventuellement de manière transitoire, l'activité CHD dans une cellule de plante hôte.

19. Utilisation selon la revendication 16 ou 17 pour stimuler la réponse d'une culture de tissu.

20. Utilisation selon la revendication 16 ou 17 pour induire une embryogenèse somatique.

21. Utilisation selon la revendication 16 ou 17 pour une sélection positive d'une cellule de plante transformée.

22. Utilisation selon la revendication 21, comprenant l'introduction d'une construction inactivant CHD telle que définie dans la revendication 16 ou 17 dans ladite cellule, dans laquelle ladite construction inactivant CHD comprend un promoteur capable de commander l'expression dans une cellule de plante, pour produire une cellule transformée, la culture de la cellule transformée pour produire un embryon transformé, et la sélection de l'embryon transformé.

23. Utilisation selon la revendication 21 ou 22 comprenant en outre l'introduction d'un gène d'intérêt dans la cellule transformée.

24. Utilisation selon la revendication 21, 22 ou 23 comprenant en outre la modification de composants de milieux pour favoriser la croissance des cellules transformées.

25. Utilisation selon la revendication 24 dans laquelle les composants de milieux sont modifiés pour réduire l'embryogenèse somatique dans des cellules non transformées.

26. Utilisation selon la revendication 22, 23, 24 ou 25 dans laquelle ladite construction inactivant CHD est excisée.

27. Utilisation selon la revendication 26 dans laquelle ledit polynucléotide est flanqué par des séquences FRT pour permettre une excision du polynucléotide sous la médiation de FLP.

28. Utilisation selon la revendication 16 ou 17 pour induire une apomixie dans une cellule de plante.

29. Utilisation selon la revendication 28, comprenant l'introduction dans une cellule de plante compatible d'une construction inactivant CHD telle que définie dans la revendication 16 ou 17 dans laquelle ladite construction comprend éventuellement un promoteur inductible pour produire une cellule de plante transformée et la culture de la cellule de plante transformée pour produire un embryon somatique transformé.

30. Utilisation selon la revendication 28 ou 29 comprenant en outre la suppression de l'expression d'un polynucléotide Polycomb FIE dans la cellule de plante en utilisant des méthodes sens ou antisens.

31. Utilisation selon la revendication 28, 29 ou 30 comprenant en outre la culture de l'embryon pour produire une plante régénérée.

32. Utilisation selon la revendication 29, 30 ou 31 dans laquelle ladite construction inactivant CHD est exprimée dans un tissu de tégument ou de nucelle.

33. Utilisation selon la revendication 16 ou 17 pour accroître l'efficacité de transformation, comprenant l'introduction d'une construction inactivant CHD et d'un gène d'intérêt dans une cellule hôte compatible.

34. Utilisation selon la revendication 16 ou 17 pour accroître la récupération de plantes régénérées, comprenant l'introduction d'une construction inactivant CHD telle que définie dans la revendication 16 ou 17 dans une cellule hôte compatible et la culture de ladite cellule pour produire une plante régénérée.

35. Utilisation selon la revendication 16 ou 17 pour réduire l'inactivation génique comprenant la transformation stable, avec une construction inactivant CHD telle que définie dans la revendication 16 ou 17 et un gène d'intérêt, d'une cellule hôte et la culture de la cellule hôte transformée.

36. Utilisation selon la revendication 16 ou 17 pour accroître la production d'huile dans une cellule hôte, comprenant la transformation stable d'une cellule hôte avec une construction inactivant CHD telle que définie dans la revendication 16 ou 17 et la culture de la cellule transformée pour produire des niveaux élevés d'huile en comparaison avec une cellule non transformée correspondante.

37. Utilisation selon l'une quelconque des revendications 17 à 36 dans laquelle ladite cellule de plante provient d'une monocotylédone ou d'une dicotylédone.

38. Utilisation selon la revendication 37 dans laquelle ladite monocotylédone ou dicotylédone est le maïs, soja, sorgho, blé, riz, luzerne, tournesol, colza, coton, ou graminée gazonnante.

39. Plante dans laquelle l'expression fonctionnelle d'une CHD (hélicase se liant à l'ADN modificatrice de l'organisation de la chromatine) est diminuée par une construction inactivant CHD, ou dans laquelle un anticorps dirigé contre un domaine fonctionnel critique de CHD réduit l'activité d'une protéine CHD endogène ; dans laquelle la CHD dont l'expression ou l'activité est réduite ou diminuée est une CHD telle que définie dans l'une quelconque des revendications 11 ou 12.

40. Plante selon la revendication 39 dans laquelle ladite diminution ou réduction est obtenue d'une manière définie dans la revendication 17.

41. Plante selon la revendication 39 ou 40 qui est une monocotylédone ou une dicotylédone.

42. Plante selon l'une quelconque des revendications 40 à 42 dans laquelle ladite monocotylédone ou dicotylédone est le maïs, soja, sorgho, blé, riz, luzerne, tournesol, colza, coton, ou graminée gazonnante.
